# EUROPEAN PATENT SPECIFICATION

(11) **EP 1 898 910 B1**
(45) Date of publication and mention of the grant of the patent: **28.09.2016**
(21) Application number: 06763891.6
(22) Date of filing: 26.06.2006
(51) Int. Cl.: A61K 31/4353, A61P 31/04, C07D 215/227, C07D 215/36, C07D 215/22

(54) **QUINOLINE DERIVATIVES AS ANTIBACTERIAL AGENTS**
CHINOLINDERIVATE ALS ANTIBAKTERIELLE WIRKSTOFFE
DÉRIVÉS DE LA QUINOLÉINE COMME AGENTS ANTIBACTÉRIENS

(30) Priority: 28.06.2005 EP 05105769
(43) Date of publication of application: 19.03.2008
(73) Proprietor: Janssen Pharmaceutica NV, 2340 Beerse (BE)
(72) Inventor: ANDRIES, Koenraad, Jozef, Lodewijk, Marcel, 2340 Beerse (BE); KOUL, Anil, 2340 Beerse (BE); GUILLEMONT, Jérôme Emile Georges, 27106 Val de Reuil Cedex (FR); MOTTE, Magali Madeleine Simone, 27106 Val de Reuil Cedex (FR)
(74) Representative: Vervoort, Liesbeth
(86) International application number: PCT/EP2006/063556
(87) International publication number: WO 2007/000436

(56) References cited:
- WO-A-2004/011436
- WO-A-2005/117875
- HELWIG, BURGHARD: "Moderne Arzneimittel" 1980, WISSENSCHAFTLICHE VERLAGSGESELLSCHAFT , STUTTGART , XP002359316 page 395
- ANDRIES, KOEN ET AL.: SCIENCE, vol. 307, 14 January 2005 (2005-01-14), pages 223-227, XP002358962 cited in the application
- DESAI P K ET AL: "QUINOLINE DERIVATIVES AS ANTITUBERCULAR/ANTIBACTERIAL AGENTS" INDIAN JOURNAL OF CHEMISTRY, JODHPUR, IN, vol. 35B, no. 8, August 1996 (1996-08), pages 871-873, XP000944820
- ANDRIES, K. ET AL.: 'Supporting Online Material', [Online] 2005, Supporting material to Andries: Science 307, 223 (2005) (XP2358962) Retrieved from the Internet: <URL:www.sciencemag.org/content/suppl/2005/ 01/18/1106753.DC1/Andries.SOM.pdf> [retrieved on 2013-01-16]

## Description

The present invention relates to the use of quinoline derivatives for the manufacture of a medicament for the treatment of a bacterial infection.

Resistance to first-line antibiotic agents is an emerging problem. Some important examples include penicillin-resistant *Streptococcus pneumoniae*, vancomycin-resistant enterococci, methicillin-resistant *Staphylococcus aureus,* multi-resistant salmonellae.

The consequences of resistance to antibiotic agents are severe. Infections caused by resistant microbes fail to respond to treatment, resulting in prolonged illness and greater risk of death. Treatment failures also lead to longer periods of infectivity, which increase the numbers of infected people moving in the community and thus exposing the general population to the risk of contracting a resistant strain infection.

Hospitals are a critical component of the antimicrobial resistance problem worldwide. The combination of highly susceptible patients, intensive and prolonged antimicrobial use, and cross-infection has resulted in infections with highly resistant bacterial pathogens.

Self-medication with antimicrobials is another major factor contributing to resistance. Self-medicated antimicrobials may be unnecessary, are often inadequately dosed, or may not contain adequate amounts of active drug.

Patient compliance with recommended treatment is another major problem. Patients forget to take medication, interrupt their treatment when they begin to feel better, or may be unable to afford a full course, thereby creating an ideal environment for microbes to adapt rather than be killed.

Because of the emerging resistance to multiple antibiotics, physicians are confronted with infections for which there is no effective therapy. The morbidity, mortality, and financial costs of such infections impose an increasing burden for health care systems worldwide.

Therefore, there is a high need for new compounds to treat bacterial infections, especially for the treatment of infections caused by resistant strains.

WO 2004/011436 discloses substituted quinoline derivatives having activity against Mycobacteria, in particular against *Mycobacterium tuberculosis.* One particular compound of these substituted quinoline derivatives is described in Science (2005), 307, 223-227.

It has now been found that quinoline derivatives described in WO 2004/011436 also show activity against other bacteria than Mycobacteria.

Therefore, the present invention relates to the use of a compound for the manufacture of a medicament for the treatment of a bacterial infection caused by Staphylococci, Enterococci or Streptococci, said compound being a compound of formula (Ia) or (Ib) 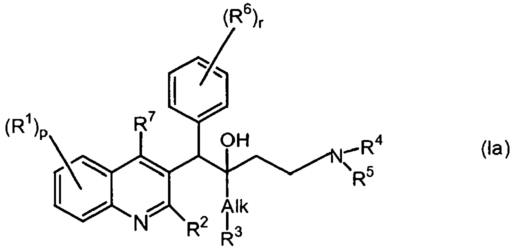 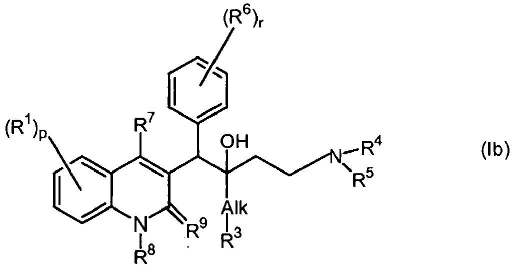 a pharmaceutically acceptable acid or base addition salt thereof, a stereochemically isomeric form thereof, a tautomeric form thereof or a *N*-oxide form thereof, wherein
- R¹: is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
- p: is an integer equal to 1, 2, 3 or 4 ;
- R²: is hydrogen, hydroxy, mercapto, alkyloxy, alkyloxyalkyloxy, alkylthio, mono or di(alkyl)amino or a radical of formula 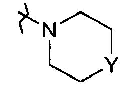 wherein Y is CH₂, O, S, NH or *N*-alkyl ;
- R³: is Ar or Het;
- R⁴ and R⁵: each independently are hydrogen, alkyl or benzyl; or

- R⁴ and R⁵: together and including the N to which they are attached may form a radical selected from the group of pyrrolidinyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, pyridazinyl, pyrimidinyl, pyrazinyl, triazinyl, morpholinyl and thiomorpholinyl, optionally substituted with alkyl, halo, haloalkyl, hydroxy, alkyloxy, amino, mono- or dialkylamino, alkylthio, alkyloxyalkyl, alkylthioalkyl or pyrimidinyl ;
- R⁶: is hydrogen, halo, haloalkyl, hydroxy, Ar, alkyl, alkyloxy, alkylthio, alkyloxyalkyl, alkylthioalkyl, Ar-alkyl or di(Ar)alkyl ; or
- two vicinal R⁶: radicals may be taken together to form a bivalent radical of formula -CH=CH-CH=CH- ;
- r: is an integer equal to 1, 2, 3, 4 or 5 ;
- R⁷: is hydrogen, alkyl, Ar or Het ;
- R⁸: is hydrogen or alkyl ;
- R⁹: is oxo ; or
- R⁸ and R⁹: together form the radical -CH=CH-N=;

- alkyl: is a straight or branched saturated hydrocarbon radical having from 1 to 6 carbon atoms ; or is a cyclic saturated hydrocarbon radical having from 3 to 6 carbon atoms ; or is a cyclic saturated hydrocarbon radical having from 3 to 6 carbon atoms attached to a straight or branched saturated hydrocarbon radical having from 1 to 6 carbon atoms; wherein each carbon atom can be optionally substituted with hydroxy, alkyloxy or oxo;
- Alk: is a straight or branched saturated hydrocarbon radical having from 1 to 6 carbon atoms;
- Ar: is a homocycle selected from the group of phenyl, naphthyl, acenaphthyl and tetrahydronaphthyl, each homocycle optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl ;
- Het: is a monocyclic heterocycle selected from the group of *N*-phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl and benzo[1,3]dioxolyl ; each monocyclic and bicyclic heterocycle may optionally be substituted with 1, 2 or 3 substituents, each substituent independently selected from the group of halo, hydroxy, alkyl, alkyloxy, and Ar-carbonyl;
- halo: is a substituent selected from the group of fluoro, chloro, bromo and iodo; and
- haloalkyl: is a straight or branched saturated hydrocarbon radical having from 1 to 6 carbon atoms or a cyclic saturated hydrocarbon radical having from 3 to 6 carbon atoms or a cyclic saturated hydrocarbon radical having from 3 to 6 carbon atoms attached to a straight or branched saturated hydrocarbon radical having from 1 to 6 carbon atoms; wherein one or more carbon atoms are substituted with one or more halo atoms.

The present invention also relates to a method of treating a bacterial infection in a mammal, in particular a warm-blooded mammal, more in particular a human, comprising administering an effective amount of a compound of the invention to the mammal.

The compounds according to Formula (Ia) and (Ib) are interrelated in that e.g. a compound according to Formula (Ib), with R⁹ equal to oxo is the tautomeric equivalent of a compound according to Formula (Ia) with R² equal to hydroxy (keto-enol tautomerism).

In the framework of this application, alkyl is a straight or branched saturated hydrocarbon radical having from 1 to 6 carbon atoms; or is a cyclic saturated hydrocarbon radical having from 3 to 6 carbon atoms; or is a cyclic saturated hydrocarbon radical having from 3 to 6 carbon atoms attached to a straight or branched saturated hydrocarbon radical having from 1 to 6 carbon atoms ; wherein each carbon atom can be optionally substituted with hydroxy, alkyloxy or oxo.
Preferably, alkyl is methyl, ethyl or cyclohexylmethyl, more preferably methyl or ethyl. An interesting embodiment of alkyl in all definitions used hereinbefore or hereinafter is C₁₋₆alkyl which represents a straight or branched saturated hydrocarbon radical having from 1 to 6 carbon atoms such as for example methyl, ethyl, propyl, 2-methyl-ethyl, pentyl, hexyl A preferred subgroup of C₁₋₆alkyl is C₁₋₄alkyl which represents a straight or branched saturated hydrocarbon radical having from 1 to 4 carbon atoms such as for example methyl, ethyl, propyl, 2-methyl-ethyl

In the framework of this application, Alk is a straight or branched saturated hydrocarbon radical having from 1 to 6 carbon atoms, in particular Alk is C₁₋₆alkanediyl which represents a bivalent straight and branched chain saturated hydrocarbon radical having from 1 to 6 carbon atoms such as, for example, methylene, 1,2-ethanediyl or ethylene, 1,3-propanediyl, 1,4-butanediyl, 1,5-pentanediyl, 1,6-hexanediyl.

In the framework of this application, Ar is a homocycle selected from the group of phenyl, naphthyl, acenaphthyl, tetrahydronaphthyl, each optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl. Preferably, Ar is naphthyl or phenyl, each optionally substituted with 1 or 2 substituents, each substituent independently selected from halo or alkyloxy.

In the framework of this application, Het is a monocyclic heterocycle selected from the group of *N*-phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl and benzo[1,3]dioxolyl ; each monocyclic and bicyclic heterocycle may optionally be substituted with 1, 2 or 3 substituents, each substituent independently selected from the group of halo, hydroxy, alkyl, alkyloxy and Ar-carbonyl. Preferably, Het is furanyl, piperidinyl, pyridinyl or benzo[1,3]dioxolyl or Het is thienyl, furanyl, pyridinyl or benzo[1,3]dioxolyl.

In the framework of this application, halo is a substituent selected from the group of fluoro, chloro, bromo and iodo and haloalkyl is a straight or branched saturated hydrocarbon radical having from 1 to 6 carbon atoms or a cyclic saturated hydrocarbon radical having from 3 to 6 carbon atoms or a cyclic saturated hydrocarbon radical having from 3 to 6 carbon atoms attached to a straight or branched saturated hydrocarbon radical having from 1 to 6 carbon atoms; wherein one or more carbon atoms are substituted with one or more halo atoms. Preferably, halo is bromo, fluoro or chloro and preferably, haloalkyl is polyhaloC₁₋₆alkyl which is defined as mono- or polyhalosubstituted C₁₋₆alkyl, for example, methyl with one or more fluoro atoms, for example, difluoromethyl or trifluoromethyl, 1,1 -difluoro-ethyl. In case more than one halo atom is attached to an alkyl group within the definition of haloalkyl or polyhaloC₁₋₆alkyl, they may be the same or different.

In the definition of Het, it is meant to include all the possible isomeric forms of the heterocycles, for instance, pyrrolyl comprises 1*H*-pyrrolyl and 2*H*-pymolyl.

The Ar or Het listed in the definitions of the substituents of the compounds of formula (Ia) or (Ib) (see for instance R³) as mentioned hereinbefore or hereinafter may be attached to the remainder of the molecule of formula (Ia) or (Ib) through any ring carbon or heteroatom as appropriate, if not otherwise specified. Thus, for example, when Het is imidazolyl, it may be 1-imidazolyl, 2-imidazolyl, 4-imidazolyl.

Lines drawn from substituents into ring systems indicate that the bond may be attached to any of the suitable ring atoms.

When two vicinal R⁶ radicals are taken together to form a bivalent radical of formula -CH=CH-CH=CH-, this means that the two vicinal R⁶ radicals form together with the phenyl ring to which they are attached a naphthyl.

For therapeutic use, salts of the compounds of formula (Ia) or (Ib) are those wherein the counterion is pharmaceutically acceptable. However, salts of acids and bases which are non-pharmaceutically acceptable may also find use, for example, in the preparation or purification of a pharmaceutically acceptable compound. All salts, whether pharmaceutically acceptable or not, are included within the ambit of the present invention.

The pharmaceutically acceptable addition salts as mentioned hereinabove or hereinafter are meant to comprise the therapeutically active non-toxic acid addition salt forms which the compounds of formula (Ia) or (Ib) are able to form. The latter can conveniently be obtained by treating the base form with such appropriate acids as inorganic acids, for example, hydrohalic acids, e.g. hydrochloric, hydrobromic sulfuric acid; nitric acid; phosphoric acid or organic acids, for example, acetic, propanoic, hydroxyacetic, 2-hydroxypropanoic, 2-oxopropanoic, oxalic, malonic, succinic, maleic, fumaric, malic, tartaric, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic acids. Conversely the salt form can be converted by treatment with alkali into the free base form.

The compounds of formula (Ia) or (Ib) containing acidic protons may be converted into their therapeutically active non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-*n*-butylamine, pyrrolidine, piperidine, morpholine, trimethylamine, triethylamine, tripropylamine, quinuclidine, pyridine, quinoline and isoquinoline, the benzathine, *N*-methyl-D-glucamine, 2-amino-2-(hydroxymethyl)-1,3-propanediol, hydrabamine salts, and salts with amino acids such as, for example, arginine, lysine. Conversely the salt form can be converted by treatment with acid into the free acid form.

The term addition salt also comprises the hydrates and solvent addition forms which the compounds of formula (Ia) or (Ib) are able to form. Examples of such forms are e.g. hydrates, alcoholates.

The *N*-oxide forms of the present compounds are meant to comprise the compounds of formula (Ia) or (Ib) wherein one or several tertiary nitrogen atoms are oxidized to the so-called *N*-oxide.

The compounds of formula (Ia) and (Ib) may be converted to the corresponding *N*-oxide forms following art-known procedures for converting a trivalent nitrogen into its *N*-oxide form. Said *N*-oxidation reaction may generally be carried out by reacting the starting material of formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. t.butyl hydro-peroxide. Suitable solvents are, for example, water, lower alcohols, e.g. ethanol, hydrocarbons, e.g. toluene, ketones, e.g. 2-butanone, halogenated hydrocarbons, e.g. dichloromethane, and mixtures of such solvents.

It will be appreciated that some of the compounds of formula (I) and their *N*-oxides or addition salts may contain one or more centres of chirality and exist as stereochemically isomeric forms.

Compounds of either formula (Ia) and (Ib) and some of the intermediate compounds invariably have at least two stereogenic centers in their structure which may lead to at least 4 stereochemically different structures.

The term "stereochemically isomeric forms" as used hereinbefore or hereinafter defines all the possible stereoisomeric forms which the compounds of formula (Ia) and (Ib), and their *N*-oxides, addition salts or physiologically functional derivatives may possess. Unless otherwise mentioned or indicated, the chemical designation of compounds denotes the mixture of all possible stereochemically isomeric forms, said mixtures containing all diastereomers and enantiomers of the basic molecular structure. In particular, stereogenic centers may have the R- or S-configuration; substituents on bivalent cyclic (partially) saturated radicals may have either the *cis*- or *trans-*configuration. Compounds encompassing double bonds can have an E (entgegen) or Z (zusammen) -stereochemistry at said double bond. The terms cis, trans, R, S, E and Z are well known to a person skilled in the art.
Stereochemically isomeric forms of the compounds of formula (Ia) and (Ib) are obviously intended to be embraced within the scope of this invention.

Following CAS-nomenclature conventions, when two stereogenic centers of known absolute configuration are present in a molecule, an R or S descriptor is assigned (based on Cahn-Ingold-Prelog sequence rule) to the lowest-numbered chiral center, the reference center. The configuration of the second stereogenic center is indicated using relative descriptors [*R*,R** ] or [*R*,S**], where *R** is always specified as the reference center and [*R**,*R**] indicates centers with the same chirality and [*R**,*S**] indicates centers of unlike chirality. For example, if the lowest-numbered chiral center in the molecule has an S configuration and the second center is *R*, the stereo descriptor would be specified as *S*-[*R**,*S**]. If "*α*" and "*β*" are used : the position of the highest priority substituent on the asymmetric carbon atom in the ring system having the lowest ring number, is arbitrarily always in the "*α*" position of the mean plane determined by the ring system. The position of the highest priority substituent on the other asymmetric carbon atom in the ring system relative to the position of the highest priority substituent on the reference atom is denominated "*α*", if it is on the same side of the mean plane determined by the ring system, or "*β*", if it is on the other side of the mean plane determined by the ring system.

When a specific stereoisomeric form is indicated, this means that said form is substantially free, *i.e.* associated with less than 50 %, preferably less than 20 %, more preferably less than 10 %, even more preferably less than 5 %, further preferably less than 2 % and most preferably less than 1 % of the other isomer(s). Thus, when a compound of formula (Ia) or (Ib) is for instance specified as (αS, βR), this means that the compound is substantially free of the (αR, βS) isomer.

The compounds of either formula (Ia) and (Ib) may be synthesized in the form of racemic mixtures of enantiomers which can be separated from one another following art-known resolution procedures. The racemic compounds of either formula (Ia) and (Ib) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of either formula (Ia) and (Ib) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The tautomeric forms of the compounds of either formula (Ia) and (Ib) are meant to comprise those compounds of either formula (Ia) and (Ib) wherein e.g. an enol group is converted into a keto group (keto-enol tautomerism).

Derivative compounds (usually called "pro-drugs") of pharmacologically-active compounds may be degraded *in vivo* to yield the compounds. Pro-drugs are usually (but not always) of lower potency at the target receptor than the compounds to which they are degraded. Pro-drugs are particularly useful when the desired compound has chemical or physical properties that make its administration difficult or inefficient. For example, the desired compound may be only poorly soluble, it may be poorly transported across the mucosal epithelium, or it may have an undesirably short plasma half-life. Further discussion on pro-drugs may be found in Stella, V. J. et al., "Prodrugs", Drug Delivery Systems, 1985, pp. 112-176, and Drugs, 1985, 29, pp. 455-473.

Pro-drugs forms of pharmacologically-active compounds will generally be the pharmaceutically acceptable acid or base addition salts the stereochemically isomeric forms the tautomeric forms and the *N*-oxide forms, having an acid group which is esterified or amidated. Included in such esterified acid groups are groups of the formula -COOR^{x}, where R^{x} is a C₁₋₆alkyl, phenyl, benzyl or one of the following groups :

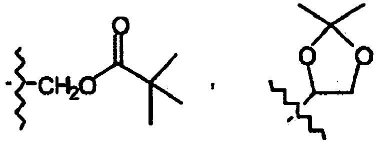

Amidated groups include groups of the formula - CONR^{y}R^{z}, wherein R^{y} is H, C₁₋₆alkyl, phenyl or benzyl and R² is -OH, H, C₁₋₆alkyl, phenyl or benzyl.

Compounds according to the invention having an amino group may be derivatised with a ketone or an aldehyde such as formaldehyde to form a Mannich base. This base will hydrolyze with first order kinetics in aqueous solution.

Whenever used herein, the term "compounds of formula (Ia) or (Ib)" is meant to also include their pharmaceutically acceptable acid or base addition salts, their *N*-oxide forms, their tautomeric forms or their stereochemically isomeric forms. Of special interest are those compounds of formula (Ia) or (Ib) which are stereochemically pure.

A first interesting embodiment of the present invention relates to a compound of formula (Ia) or (Ib) wherein Alk represent methylene or ethylene, in particular ethylene.

A second interesting embodiment relates to a compound of formula (Ia) or (Ib) or any subgroup thereof as mentioned hereinbefore as interesting embodiment wherein
- R¹: is hydrogen, halo, cyano, Ar, Het, alkyl, and alkyloxy ;
- p is: an integer equal to 1, 2, 3 or 4 ; in particular 1 or 2;
- R² is: hydrogen, hydroxy, alkyloxy, alkyloxyalkyloxy, alkylthio or a radical of formula 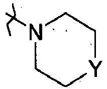 wherein Y is O ;
- R³: is Ar or Het ;
- R⁴ and R⁵: each independently are hydrogen, alkyl or benzyl;
- R⁶: is hydrogen, halo or alkyl ; or
- two vicinal R⁶: radicals may be taken together to form a bivalent radical of formula -CH=CH-CH=CH-;
- r: is an integer equal to 1 ;
- R⁷: is hydrogen;
- R⁸: is hydrogen or alkyl,
- R⁹: is oxo ; or
- R⁸ and R⁹: together form the radical -CH=CH-N=;
- alkyl: is a straight or branched saturated hydrocarbon radical having from 1 to 6 carbon atoms ; or is a cyclic saturated hydrocarbon radical having from 3 to 6 carbon atoms ; or is a a cyclic saturated hydrocarbon radical having from 3 to 6 carbon atoms attached to a straight or branched saturated hydrocarbon radical having from 1 to 6 carbon atoms ; wherein each carbon atom can be optionally substituted with hydroxy;
- Alk: is ethylene;
- Ar: is a homocycle selected from the group of phenyl, naphthyl, acenaphthyl and tetrahydronaphthyl, each homocycle optionally substituted with 1, 2 or 3 substituents, each substituent independently selected from the group of halo, haloalkyl, cyano, alkyloxy and morpholinyl;
- Het: is a monocyclic heterocycle selected from the group of *N*-phenoxypiperidinyl, piperidinyl, furanyl, thienyl, pyridinyl and pyrimidinyl ; or a bicyclic heterocycle selected from the group of benzothienyl, 2,3-dihydrobenzo[1,4]dioxinyl and benzo[1,3]dioxolyl; each monocyclic and bicyclic heterocycle may optionally be substituted with 1, 2 or 3 substituents, each substituent independently selected from alkyl or Ar-carbonyl; and
- halo: is a substituent selected from the group of fluoro, chloro and bromo.
- haloalkyl: is a straight or branched saturated hydrocarbon radical having from 1 to 6 carbon atoms or a cyclic saturated hydrocarbon radical having from 3 to 6 carbon atoms or a cyclic saturated hydrocarbon radical having from 3 to 6 carbon atoms attached to a straight or branched saturated hydrocarbon radical having from 1 to 6 carbon atoms; wherein one or more carbon atoms are substituted with one or more halo atoms.

A third interesting embodiment relates to a compound of formula (Ia) or (Ib) or any subgroup thereof as mentioned hereinbefore as interesting embodiment wherein R¹ is hydrogen, halo, alkyl, alkyloxy, Ar or Het; preferably, R¹ is hydrogen, halo, alkyl or alkyloxy; more preferably, R¹ is hydrogen or halo; most preferred R¹ is halo, e.g. bromo or chloro, in particular bromo.

A fourth interesting embodiment relates to a compound of formula (Ia) or (Ib) or any subgroup thereof as mentioned hereinbefore as interesting embodiment wherein p is equal to 1 or 2; preferably p is equal to 1; more preferably p is equal to 1 and R¹ is other than hydrogen.

A fifth interesting embodiment relates to a compound of formula (Ia) or (Ib) or any subgroup thereof as mentioned hereinbefore as interesting embodiment wherein p is equal to 1 and said R¹ substituent is placed in position 5, 6 or 7 of the quinoline ring; preferably in position 6.

A sixth interesting embodiment relates to a compound of formula (Ia) or (Ib) or any subgroup thereof as mentioned hereinbefore as interesting embodiment wherein R² is hydrogen, alkyloxy, alkylthio, alkyloxyalkyloxy or mono or di(alkyl)amino; preferably, R² is hydrogen, alkyloxy or alkylthio; more preferably, R² is alkyloxy or alkylthio; most preferably, R² is alkyloxy, in particular C₁₋₄alkyloxy; more in particular methyloxy.

A seventh interesting embodiment relates to a compound of formula (Ia) or (Ib) or any subgroup thereof as mentioned hereinbefore as interesting embodiment wherein R³ is Ar, optionally substituted with 1 or 2 substituents, said substituent preferably being a halo, haloalkyl, alkyloxy, haloalkyloxy or alkyl; more preferably said substituent being a halo, haloalkyl or alkyloxy; even more preferably said substituent being a halo or alkyloxy; most preferably said substituent being a halo; preferably, Ar in the definition of R³ is naphthyl or phenyl, optionally substituted with 1 or 2 halo atoms, in particular 4-halophenyl; more preferably, R³ is naphthyl or phenyl; most preferred R³ is 1-naphthyl or phenyl.

Another interesting embodiment relates to a compound of formula (Ia) or (Ib) or any subgroup thereof as mentioned hereinbefore as interesting embodiment wherein R³ is Het, in particular benzo[1,3]dioxolyl.

An eighth interesting embodiment relates to a compound of formula (Ia) or (Ib) or any subgroup thereof as mentioned hereinbefore as interesting embodiment wherein R⁴ and R⁵ each independently are hydrogen, alkyl or benzyl; preferably hydrogen or alkyl, in particular hydrogen or C₁₋₄alkyl; more preferably C₁₋₄alkyl; most preferably methyl.

A ninth interesting embodiment relates to a compound of formula (Ia) or (Ib) or any subgroup thereof as mentioned hereinbefore as interesting embodiment wherein R⁴ and R⁵ together and including the N to which they are attached form a radical selected from the group of pyrrolidinyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, pyridazinyl, pyrimidinyl, pyrazinyl, triazinyl, morpholinyl and thiomorpholinyl, optionally substituted with alkyl, halo, haloalkyl, hydroxy, alkyloxy, amino, mono- or dialkylamino, alkylthio, alkyloxyalkyl, alkylthioalkyl or pyrimidinyl; preferably R⁴ and R⁵ together and including the N to which they are attached form a radical selected from the group of pyrrolidinyl, imidazolidinyl, pyrazolidinyl, piperidinyl, piperazinyl, morpholinyl and thiomorpholinyl, optionally substituted with alkyl, halo, haloalkyl, hydroxy, alkyloxy, amino, mono- or dialkylamino, alkylthio, alkyloxyalkyl, alkylthioalkyl or pyrimidinyl; more preferably R⁴ and R⁵ together and including the N to which they are attached form a radical selected from the group of piperidinyl or morpholinyl, in particular a piperidinyl.

A tenth interesting embodiment relates to a compound of formula (Ia) or (Ib) or any subgroup thereof as mentioned hereinbefore as interesting embodiment wherein R⁶ is hydrogen, alkyl, alkyloxy or halo; preferably, R⁶ is hydrogen.

An eleventh interesting embodiment relates to a compound of formula (Ia) or (Ib) or any subgroup thereof as mentioned hereinbefore as interesting embodiment wherein r is 1 or 2; preferably r is 1.

A twelfth interesting embodiment relates to a compound of formula (Ia) or (Ib) or any subgroup thereof as mentioned hereinbefore as interesting embodiment wherein R⁷ is hydrogen or methyl; preferably R⁷ is hydrogen.

A thirteenth interesting embodiment relates to a compound of formula (Ia) or (Ib) or any subgroup thereof as mentioned hereinbefore as interesting embodiment wherein, for compounds according to Formula (Ib) only, R⁸ is hydrogen or alkyl; and R⁹ is oxo; preferably R⁸ is alkyl, preferably methyl, and R⁹ is oxo.

A fourteenth interesting embodiment relates to a compound of formula (Ia) or (Ib) or any subgroup thereof as mentioned hereinbefore as interesting embodiment wherein the compound is a compound according to formula (Ia).

A fifteenth interesting embodiment relates to a compound of formula (Ia) or any subgroup thereof as mentioned hereinbefore as interesting embodiment wherein one or more, preferably all, of the following definitions apply :
R¹ is hydrogen, halo, alkyl, alkyloxy, Ar or Het; in particular hydrogen, halo, C₁₋₄alkyl, C₁₋₄alkyloxy, Ar or Het; more in particular hydrogen, bromo, methyl, methyloxy, hydroxymethyl, phenyl, pyridinyl, thienyl or furanyl;
p = 1 or 2; in particular 1;
R² is alkyloxy, alkylthio, mono-or di(alkyl)amino, alkyloxyalkyloxy; in particular C₁₋₄alkyloxy, C₁₋₄alkylthio, mono-or di(C₁₋₄alkyl)amino,
C₁₋₄alkyloxyC₁₋₄alkyloxyC₁₋₄alkyloxy; more in particular C₁₋₄alkyloxy, such as methyloxy;
R³ is naphthyl, phenyl, each of said ring systems being optionally substituted; in particular phenyl, optionally substituted with 1 or 2 substituents selected from halo, haloalkyl, alkyloxy, haloalkyloxy or alkyl; or naphthyl; more in particular phenyl, optionally substituted with 1 or 2 substituents selected from halo, haloC₁₋₄alkyl, C₁₋₄alkyloxy, haloC₁₋₄alkyloxy or C₁₋₄alkyl; or naphthyl;
R⁴ and R⁵ each independently are hydrogen or alkyl; in particular hydrogen or C₁₋₄alkyl; more in particular hydrogen or methyl; or R⁴ and R⁵ together and including the N to which they are attached form a piperidinyl;
R6 is hydrogen, halo, alkyl or alkyloxy; in particular hydrogen, halo, C₁₋₄alkyl or C₁₋₄alkyloxy;
r is equal to 1;
R⁷ is hydrogen;
Alk is methylene or ethylene.

A sixteenth interesting embodiment is the use of a compound of formula (Ia) or (Ib) or any subgroup thereof as mentioned hereinbefore as interesting embodiment for the manufacture of a medicament for the treatment of an infection with a gram-positive and/or a gram-negative bacterium.

A seventeenth interesting embodiment is the use of a compound of formula (Ia) or (Ib) or any subgroup thereof as mentioned hereinbefore as interesting embodiment for the manufacture of a medicament for the treatment of an infection with a gram-positive bacterium.

A eighteenth interesting embodiment is the use of the compounds of formula (Ia) or (Ib) or any subgroup thereof as mentioned hereinbefore as interesting embodiment for the manufacture of a medicament for the treatment of an infection with a gram-negative bacterium.

A nineteenth interesting embodiment is the use of a compound of formula (Ia) or (Ib) or any subgroup thereof as mentioned hereinbefore as interesting embodiment for the manufacture of a medicament for the treatment of a bacterial infection wherein the compound of formula (Ia) or (Ib) has a IC₉₀ < 15 µl/ml against at least one bacterium, in particular a gram-positive bacterium; preferably a IC₉₀ < 10 µl/ml; more preferably a IC₉₀ < 5 µl/ml; the IC₉₀ value being determined as described hereinafter.

Preferably, in the compounds of formula (Ia) and (Ib) or any subgroup thereof as mentioned hereinbefore as interesting embodiment, the term "alkyl" represents C₁₋₆alkyl, more preferably C₁₋₄alkyl.

Preferred compounds are selected from the following compounds :

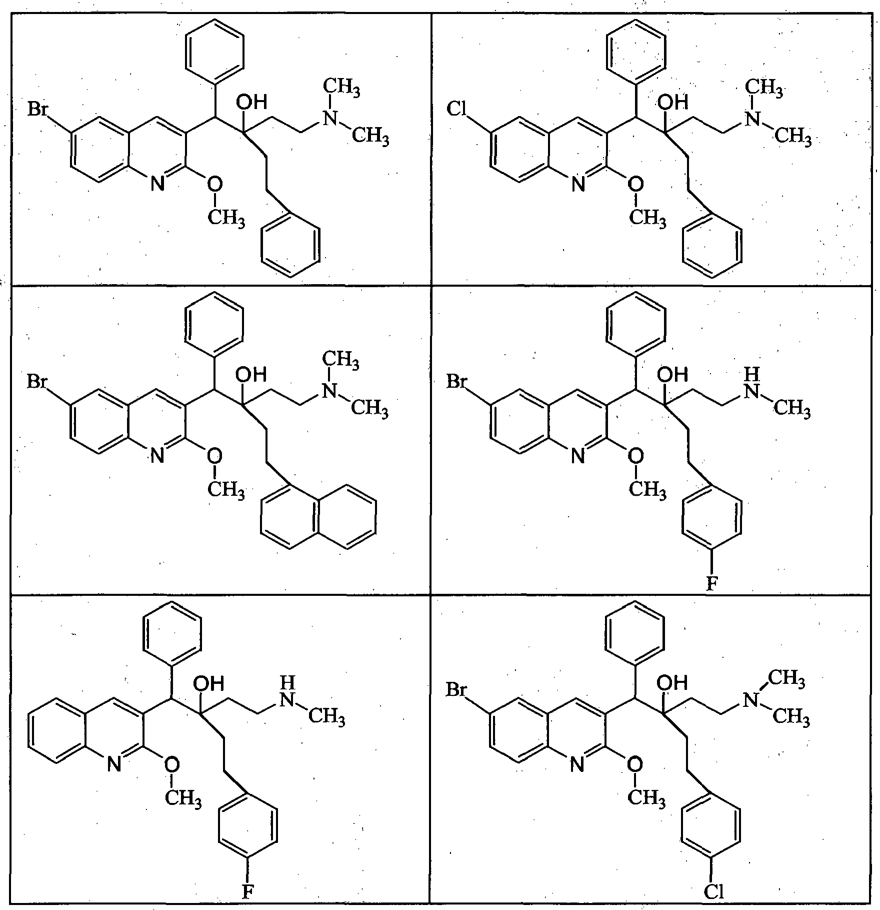

a pharmaceutically acceptable acid or base addition salt thereof, a stereochemically isomeric form thereof, a tautomeric form thereof or a *N*-oxide form thereof.

Especially preferred compounds are selected from compound 14, 15, 7, 8, 9, 20, 39, 37, 38, 55 and 40 (see Tables hereinbelow), a *N*-oxide thereof, a tautomeric form thereof or a stereochemically isomeric form thereof; in particular preferred compounds are compounds 39, 37, 38, 55 and 40, a *N*-oxide thereof, a tautomeric form thereof or a stereochemically isomeric form thereof.

The present invention also relates to any one compound out of Tables 1 to 5 hereinbelow.

In particular, the present invention relates to a compound selected from

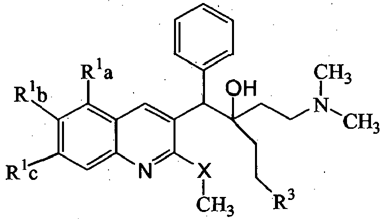

| R¹ₐ | R¹_{b} | R¹_{c} | R³ | X |
|---|---|---|---|---|
| H | H | H | phenyl | O |
| H | CH₃ | H | phenyl | O |
| H | OCH₃ | H | phenyl | O |
| H | Br | H | phenyl | S |
| H | Br | H | 1-naphthyl | O |
| H | Br | CH₃ | phenyl | O |
| H | Cl | H | phenyl | O |
| Br | H | H | phenyl | O |

a pharmaceutically acceptable acid or base addition salt thereof, a stereochemically isomeric form thereof, a tautomeric form thereof or a *N*-oxide form thereof.

The present invention also relates to a compound selected from

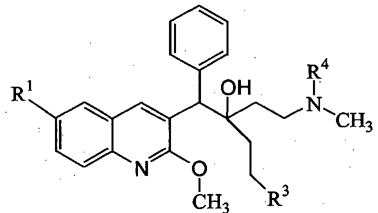

| R¹ | R³ | R⁴ |
|---|---|---|
| Br | 4-fluorophenyl | H |
| H | 4-fluorophenyl | H |
| Br | 4-chlorophenyl | CH₃ |

a pharmaceutically acceptable acid or base addition salt thereof, a stereochemically isomeric form thereof, a tautomeric form thereof or a *N*-oxide form thereof.

The present invention also relates to a compound selected from

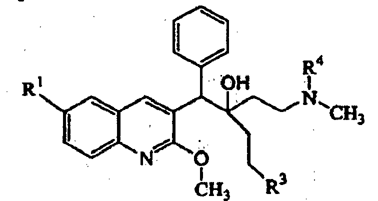

| R¹ | R³ | R⁴ | stereochemistry |
|---|---|---|---|
| Br | 4-fluorophenyl | H | (A) |
| Br | 4-fluorophenyl | H | (B) |
| H | 4-fluorophenyl | H | (A) |
| H | 4-fluorophenyl | H | (B) |
| Br | 4-chlorophenyl | CH₃ | (B) |

a pharmaceutically acceptable acid or base addition salt thereof, a stereochemically isomeric form thereof, a tautomeric form thereof or a *N*-oxide form thereof.

Preferably, the compound of formula (Ia) or (Ib) is a particular diastereoisomer (substantially free of the other diastereoisomer(s)). In case the compound of formula (Ia) or (Ib) has two chiral centers this means that the compound is a racemic mixture of the (R,S) and (S,R) enantiomers or a racemic mixture of the (R,R) and (S,S) enantiomer. Hereinafter, the racemic mixtures of 2 enantiomers are indicated as diastereoisomer A or B. Whether the racemic mixture is indicated as A or B depends on whether it is first isolated in the synthesis protocol (i.e. A) or second (i.e. B). More preferably, the compound of formula (Ia) or (Ib) is a particular enantiomer (substantially free of the other enantiomers). In case the compound of formula (Ia) or (Ib) has two chiral centers this means that the compound is the (R,S), (S,R), (R,R) or (S,S) enantiomer. Hereinafter, said particular enantiomers are indicated as A1, A2, B1 or B2. Whether the enantiomer is indicated as A1, A2, B1 or B2 depends on whether it is isolated first or second in the synthesis protocol.

The compounds of formula (Ia) or (Ib) can be prepared according to the methods described in WO 2004/011436.

In general, the compounds according to the invention can be prepared by a succession of steps, each of which is known to the skilled person.

In particular, the compounds according to formula (Ia) can be prepared by reacting an intermediate compound of formula (II) with an intermediate compound of formula (III) according to the following reaction scheme (1) : 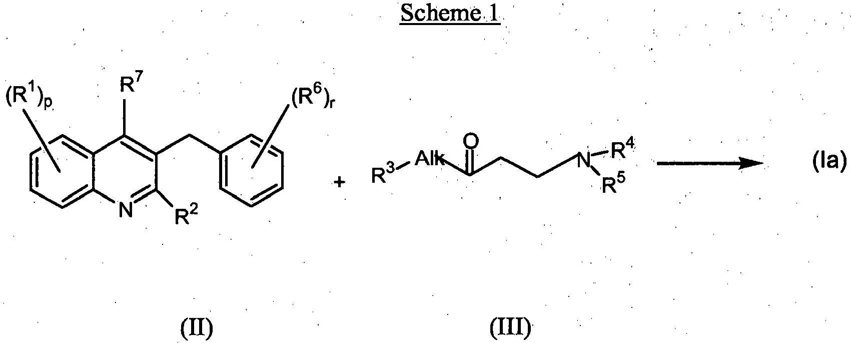 using nBuLi in a mixture of diisopropyl amine and tetrahydrofuran, wherein all variables are defined as in formula (Ia). Stirring may enhance the rate of the reaction. The reaction may conveniently be carried out at a temperature ranging between -20 and -70 °C.

The same reaction procedure can be used to synthesize compounds of formula (Ib).

The starting materials and the intermediate compounds of formula (II) and (III) are compounds that are either commercially available or may be prepared according to conventional reaction procedures generally known in the art. For example, intermediate compounds of formula (II-a) or (II-b) may be prepared according to the following reaction scheme (2): 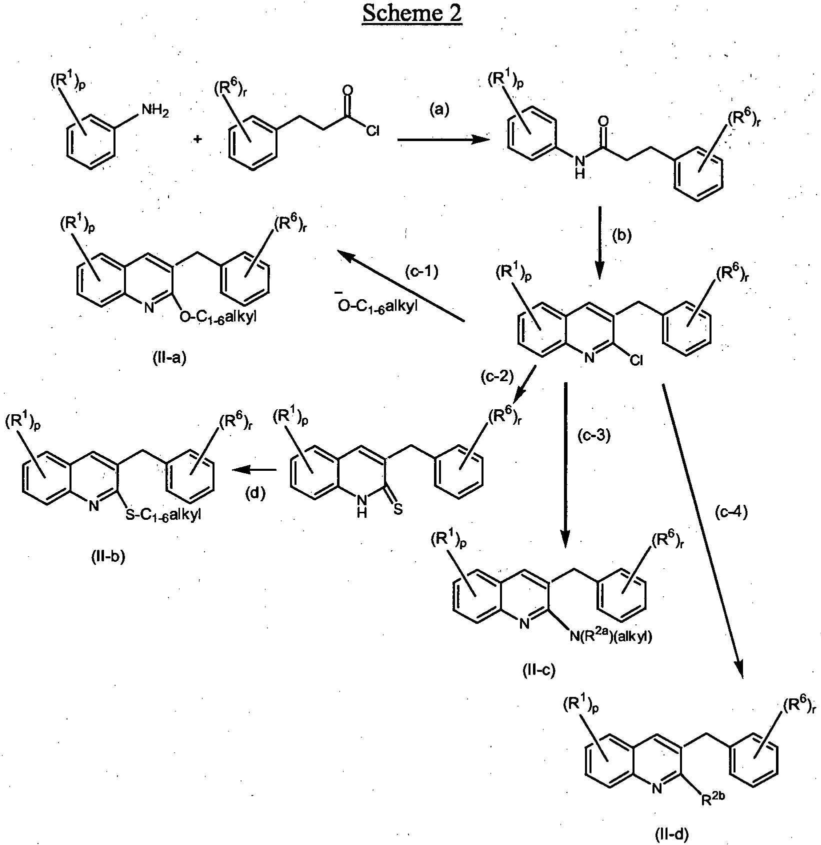 wherein all variables are defined as in formula (Ia). Reaction scheme (2) comprises step (a) in which an appropriately substituted aniline is reacted with an appropriate acylchloride such as 3-phenylpropionyl chloride, 3-fluorobenzenepropionyl chloride or *p*-chlorobenzenepropionyl chloride, in the presence of a suitable base, such as triethylamine and a suitable reaction-inert solvent, such as methylene chloride or ethylene dichloride. The reaction may conveniently be carried out at a temperature ranging between room temperature and reflux temperature. In a next step (b) the adduct obtained in step (a) is reacted with phosphoryl chloride (POCl₃) in the presence of *N,N*-dimethylformamide (Vilsmeier-Haack formylation followed by cyclization). The reaction may conveniently be carried out at a temperature ranging between room temperature and reflux temperature. In a next step (c-1), a specific R²-group, wherein R² is for example a C₁₋₆alkyloxy radical is introduced by reacting the intermediate compound obtained in step (b) with -O-C₁₋₆alkyl in the presence of a suitable solvent, such as for example HO-C₁₋₆alkyl. The intermediate compound obtained in step (b) can also be converted into an intermediate compound wherein R² is for example a C₁₋₆alkylthio radical by reaction with S=C(NH₂)₂ in the presence of a suitable solvent, such as for example an alcohol, e.g. ethanol (step (c-2)) followed by reaction with C₁₋₆alkyl-1 in the presence of a suitable base, such as for example K₂CO₃ and a suitable solvent, such as for example 2-propanone. The intermediate compound obtained in step (b) can also be converted into an intermediate compound wherein R² is N(R^{2a})(alkyl) wherein R^{2a} is hydrogen or alkyl, by reaction with a suitable salt of NH(R^{2a})(alkyl) in the presence of a suitable base, such as for example potassium carbonate, and a suitable solvent, such as for example acetonitrile (step (c-3)). The intermediate compound obtained in step (b) can also be converted into an intermediate compound wherein R² is alkyloxyalkyloxy optionally substituted with alkyloxy, said R² being represented by R^{2b}, by reaction with alkyloxyalkylOH optionally substituted with alkyloxy in the presence of NaH and a suitable solvent, such as for example tetrahydrofuran (step (C-4)).

Intermediate compounds according to formula (II-e) may be prepared according to the following reaction scheme (3), wherein in a first step (a) a substituted indole-2,3-dione is reacted with an optionally substituted 3-phenylpropionaldehyde in the presence of a suitable base such as sodium hydroxide (Pfitzinger reaction), after which the carboxylic acid compound is decarboxylated in a next step (b) at high temperature in the presence of a suitable reaction-inert solvent such as diphenylether.

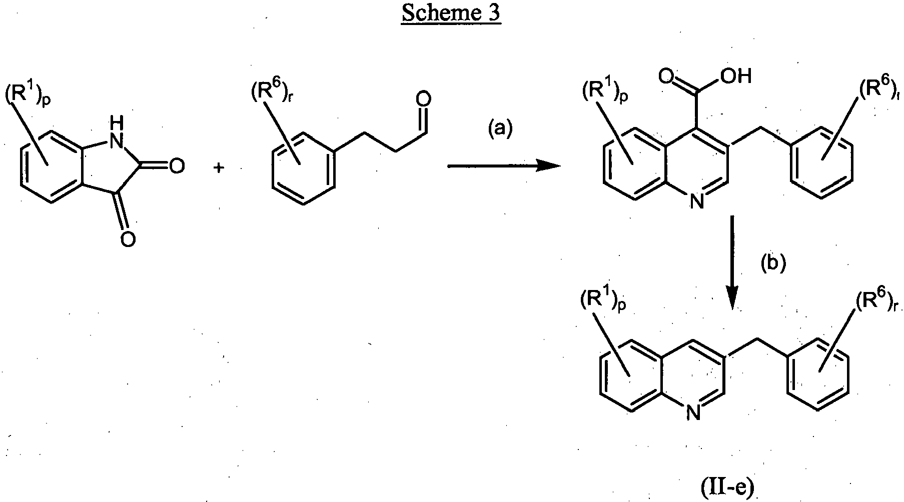

It is evident that in the foregoing and in the following reactions, the reaction products may be isolated from the reaction medium and, if necessary, further purified according to methodologies generally known in the art, such as extraction, crystallization and chromatography. It is further evident that reaction products that exist in more than one enantiomeric form, may be isolated from their mixture by known techniques, in particular preparative chromatography, such as preparative HPLC, chiral chromatography. Individual diastereoisomers or individual enantiomers can also be obtained by Supercritical Fluid Chromatography (SCF).

The intermediate compounds of formula (III) are compounds that are either commercially available or may be prepared according to conventional reaction procedures generally known in the art. For example, intermediate compounds of formula (III) may be prepared according to the following reaction scheme (4):

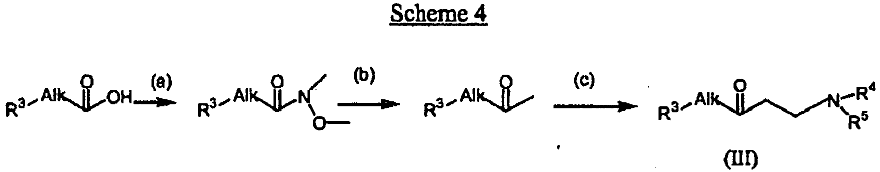

Reaction scheme (4) comprises step (a) in which for instance a suitable acid is reacted with NH(CH₃)(OCH₃) in the presence of 1,1'-carbonyldiimidazole and a suitable solvent, such as for example CH₂Cl₂. In a next step (b), the product obtained in step (a) is reacted with Grignard reagens (CH₃MgCl) in the presence of a suitable solvent, such as for example tetrahydrofuran. In a next step (c), an amino group (-NR⁴R⁵) is introduced by reacting the intermediate compound obtained in step (b) with a primary or secondary amine HNR⁴R⁵ in the presence of CH₂(=O), a suitable acid, such as for example hydrochloric acid and a suitable solvent, such as for example an alcohol, e.g. ethanol.

Intermediate compounds of formula (III) wherein Alk represents ethylene, said intermediates being represented by formula (III-a), can alternatively be prepared according to the following reaction scheme (5):

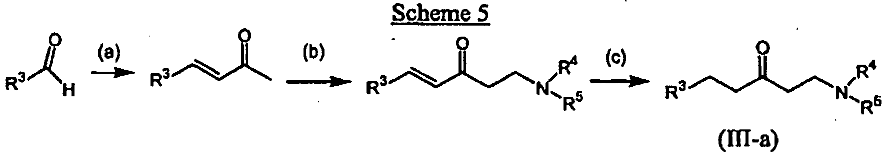

Reaction scheme 5 comprises step (a) wherein a suitable aldehyde is reacted with acetone in the presence of a suitable base, such as for example sodium hydroxide. In a next step (b), the product obtained in step (a) is reacted with a primary or secondary amine HNR⁴R⁵ in the presence of CH₂(=O), a suitable acid, such as for example hydrochloric acid, and a suitable solvent, such as for example an alcohol, e.g. ethanol. In a next step (c), the product obtained in step (b) is hydrogenated (H₂) in the presence of a suitable catalyst, such as for example palladium on charcoal, and a suitable solvent, such as for example water and an alcohol, e.g. ethanol.

The compounds of formula (Ia) or (Ib) can also be converted into each other following art-known functional group transformation reactions, comprising the one described hereinafter.

For instance, compounds of formula (Ia) or (Ib) wherein R¹ is halo, in particular bromo, can be converted into a compound of formula (Ia) or (Ib) wherein R¹ is hydrogen, by reaction with HCOONH₄ in the presence of a suitable catalyst such as for example palladium on charcoal, and in the presence of a suitable solvent, such as for example an alcohol, e.g. methanol. The same reaction conditions can be used to convert a compound of formula (Ia) or (Ib) wherein R⁴ is benzyl into a compound of formula (Ia) or (Ib) wherein R⁴ is hydrogen.

Compounds of formula (Ia) or (Ib) wherein R¹ is halo, in particular bromo, can also be converted into a compound of formula (Ia) or (Ib) wherein R¹ is Het, in particular pyridine, by reaction with a suitable boronic acid derivative of Het, e.g. pyridine-3-boronic acid, in the presence of a suitable catalyst, such as for example (triphenylphosphine)palladium(0), in the presence of a suitable solvent, such as for example ethyleneglycol dimethylether, and a suitable base, such as for example sodium carbonate.

Compounds of formula (Ia) or (Ib) wherein R¹ is halo, in particular bromo, can also be converted into an intermediate wherein R¹ is formyl by reaction with *N,N*-dimethylformamide in the presence of *n*BuLi and a suitable solvent, such as for example tetrahydrofuran. These intermediates can then be converted into a compound of formula (Ia) or (Ib) wherein R¹ is -CH₂-OH by reaction with a suitable reducing agent, such as for example NaBH₄ and in the presence of a suitable solvent, such as for example an alcohol, e.g. methanol, and tetrahydrofuran.

As indicated above, the compounds of formula (Ia) and (Ib) can be used as antibacterials.

In general, bacterial pathogens may be classified as either gram-positive or gram-negative pathogens. Antibiotic compounds with activity against both gram-positive and gram-negative pathogens are generally regarded as having a broad spectrum of activity. The compounds of the present invention are regarded as active against gram-positive and/or gram-negative bacterial pathogens. In particular, the present compounds are active against at least one gram-positive bacterium, preferably against several gram-positive bacteria, more preferably against one or more gram-positive bacteria and/or one or more gram-negative bacteria.

The present compounds have bactericidal or bacteriostatic activity.

Examples of gram-positive and gram-negative aerobic and anaerobic bacteria, include Staphylococci, for example *S. aureus;* Enterococci, for example *E. faecalis;* Streptococci, for example *S. pneumoniae, S. mutans, S. pyogens;* Bacilli, for example *Bacillus subtilis;* Listeria, for example *Listeria monocytogenes;* Haemophilus, for example *H. influenza;* Moraxella, for example *M. catarrhalis;* Pseudomonas, for example *Pseudomonas aeruginosa;* and Escherichia, for example *E. coli.* Gram-positive pathogens, for example Staphylococci, Enterococci and Streptococci are particularly important because of the development of resistant strains which are both difficult to treat and difficult to eradicate from for example a hospital environment once established. Examples of such strains are methicillin resistant *Staphylococcus aureus* (MRSA), methicillin resistant coagulase negative staphylococci (MRCNS), penicillin resistant *Streptococcus pneumoniae* and multiple resistant *Enterococcus faecium.*

The compounds of the present invention also show activity against resistant bacterial strains.

The compounds of the present invention are especially active against *Streptococcus pneumoniae* and/or *Staphylococcus aureus,* including resistant *Staphylococcus aureus* such as for example methicillin resistant *Staphylococcus aureus* (MRSA), especially against *Staphylococcus aureus,* including resistant *Staphylococcus aureus.* The present compounds have especially a good activity against SPN 6305 *(Streptococcus pneumoniae* (ATCC6305)) and/or STA 29213 *(Staphylococcus aureus* (ATCC29213)).

In particular, the compounds of the present invention are active on those bacteria of which the viability depends on proper functioning of F1F0 ATP synthase. Without being bound to any theory, it is taught that the activity of the present compounds lies in inhibition of the F1F0 ATP synthase, in particular the inhibition of the F0 complex of the F1F0 ATP synthase, more in particular the inhibition of subunit c of the F0 complex of the F1F0 ATP synthase, leading to killing of the bacteria by depletion of the cellular ATP levels of the bacteria.

Whenever used hereinbefore or hereinafter, that the compounds can treat a bacterial infection it is meant that the compounds can treat an infection with one or more bacterial strains.
Whenever used hereinbefore or hereinafter, that the bacterial infection is other than a Mycobacterial infection it is meant that the bacterial infection is other than an infection with one or more Mycobacteria strains.

The compounds of the present invention have an acceptable t_{1/2}, i.e. The half-life (t_{1/2}) of a compound refers to the time course necessary for the quantity of the compound in the body (or plasma concentration) to be reduced to half of its original level through various elimination processes.

The exact dosage and frequency of administration of the present compounds depends on the particular compound of formula (Ia) or (Ib) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention.

The compound of the present invention may be administered in a pharmaceutically acceptable form optionally in a pharmaceutically acceptable carrier. The compounds and compositions comprising the compounds can be administered by routes such as topically, locally or systemically. Systemic application includes any method of introducing the compound into the tissues of the body, e.g., intrathecal, epidural, intramuscular, transdermal, intravenous, intraperitoneal, subcutaneous, sublingual, rectal, and oral administration. The specific dosage of antibacterial to be administered, as well as the duration of treatment, may be adjusted as needed.

Bacterial infections which may be treated by the present compounds include, for example, central nervous system infections, external ear infections, infections of the middle ear, such as acute otitis media, infections of the cranial sinuses, eye infections, infections of the oral cavity, such as infections of the teeth, gums and mucosa, upper respiratory tract infections, lower respiratory tract infections, genitourinary infections, gastrointestinal infections, gynecological infections, septicemia, bone and joint infections, skin and skin structure infections, bacterial endocarditis, burns, antibacterial prophylaxis of surgery, and antibacterial prophylaxis in immunosuppressed patients, such as patients receiving cancer chemotherapy, or organ transplant patients.

Given the fact that the compounds of formula (Ia) or (Ib) are active against bacterial infections, the present compounds may be combined with other antibacterial agents in order to effectively combat bacterial infections.

Therefore, the present invention also relates to a combination of (a) a compound of formula (Ia) or (Ib), and (b) one or more other antibacterial agents provided that the one or more other antibacterial agents are other than antimycobacterial agents.

The present invention also relates to a combination of (a) a compound of formula (Ia) or (Ib), and (b) one or more other antibacterial agents provided that the one or more other antibacterial agents are other than antimycobacterial agents, for use as a medicine.

A pharmaceutical composition comprising a pharmaceutically acceptable carrier and, as active ingredient, a therapeutically effective amount of (a) a compound of formula (Ia) or (Ib), and (b) one or more other antibacterial agents provided that the one or more other antibacterial agents are other than antimycobacterial agents, is also comprised by the present invention.

The present invention also relates to the use of a combination or pharmaceutical composition as defined above for the treatment of a bacterial infection.

The present pharmaceutical composition may have various pharmaceutical forms for administration purposes. As appropriate compositions there may be cited all compositions usually employed for systemically administering drugs. To prepare the pharmaceutical compositions of this invention, an effective amount of the particular compounds, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally or by parenteral injection. For example, in preparing the compositions in oral dosage form, any of the usual pharmaceutical media may be employed such as, for example, water, glycols, oils, alcohols in the case of oral liquid preparations such as suspensions, syrups, elixirs, emulsions and solutions; or solid carriers such as starches, sugars, kaolin, diluents, lubricants, binders, disintegrating agents in the case of powders, pills, capsules and tablets. Because of their ease in administration, tablets and capsules represent the most advantageous oral unit dosage forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents may be employed. Also included are solid form preparations which are intended to be converted, shortly before use, to liquid form preparations.

Depending on the mode of administration, the pharmaceutical composition will preferably comprise from 0.05 to 99 % by weight, more preferably from 0.1 to 70 % by weight of the active ingredients, and, from 1 to 99.95 % by weight, more preferably from 30 to 99.9 weight % of a pharmaceutically acceptable carrier, all percentages being based on the total composition.

The weight to weight ratio's of the compound of formula (Ia) or (Ib) and (b) the other antibacterial agent(s) when given as a combination may be determined by the person skilled in the art. Said ratio and the exact dosage and frequency of administration depends on the particular compound of formula (Ia) or (Ib) and the other antibacterial agent(s) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention.

The compounds of formula (Ia) or (Ib) and the one or more other antibacterial agents may be combined in a single preparation or they may be formulated in separate preparations so that they can be administered simultaneously, separately or sequentially. Thus, the present invention also relates to a product or kit containing (a) a compound of formula (Ia) or (Ib), and (b) one or more other antibacterial agents provided that the one or more other antibacterial agents are other than antimycobacterial agents, as a combined preparation for simultaneous, separate or sequential use in the treatment of a bacterial infection.

The pharmaceutical composition may additionally contain various other ingredients known in the art, for example, a lubricant, stabilising agent, buffering agent, emulsifying agent, viscosity-regulating agent, surfactant, preservative, flavouring or colorant.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and segregated multiples thereof.
The daily dosage of the compound according to the invention will, of course, vary with the compound employed, the mode of administration, the treatment desired and the bacterial disease indicated.

The other antibacterial agents which may be combined with the compounds of formula (Ia) or (Ib) are antibacterial agents known in the art. The other antibacterial agents comprise antibiotics of the β-lactam group such as natural penicillins, semisynthetic penicillins, natural cephalosporins, semisynthetic cephalosporins, cephamycins, 1-oxacephems, clavulanic acids, penems, carbapenems, nocardicins, monobactams; tetracyclines, anhydrotetracyclines, anthracyclines; aminoglycosides; nucleosides such as *N*-nucleosides, C-nucleosides, carbocyclic nucleosides, blasticidin S; macrolides such as 12-membered ring macrolides, 14-membered ring macrolides, 16-membered ring macrolides; ansamycins; peptides such as bleomycins, gramicidins, polymyxins, bacitracins, large ring peptide antibiotics containing lactone linkages, actinomycins, amphomycin, capreomycin, distamycin, enduracidins, mikamycin, neocarzinostatin, stendomycin, viomycin, virginiamycin; cycloheximide; cycloserine; variotin; sarkomycin A; novobiocin; griseofulvin; chloramphenicol; mitomycins; fumagillin; monensins; pyrrolnitrin; fosfomycin; fusidic acid; D-(*p*-hydroxyphenyl)glycine; D-phenylglycine; enediynes.
Specific antibiotics which may be combined with the present compounds of formula (Ia) or (Ib) are for example benzylpenicillin (potassium, procaine, benzathine), phenoxymethylpenicillin (potassium), phenethicillin potassium, propicillin, carbenicillin (disodium, phenyl sodium, indanyl sodium), sulbenicillin, ticarcillin disodium, methicillin sodium, oxacillin sodium, cloxacillin sodium, dicloxacillin, flucloxacillin, ampicillin, mezlocillin, piperacillin sodium, amoxicillin, ciclacillin, hectacillin, sulbactam sodium, talampicillin hydrochloride, bacampicillin hydrochloride, pivmecillinam, cephalexin, cefaclor, cephaloglycin, cefadroxil, cephradine, cefroxadine, cephapirin sodium, cephalothin sodium, cephacetrile sodium, cefsulodin sodium, cephaloridine, cefatrizine, cefoperazone sodium, cefamandole, vefotiam hydrochloride, cefazolin sodium, ceftizoxime sodium, cefotaxime sodium, cefmenoxime hydrochloride, cefuroxime, ceftriaxone sodium, ceftazidime, cefoxitin, cefmetazole, cefotetan, latamoxef, clavulanic acid, imipenem, aztreonam, tetracycline, chlortetracycline hydrochloride, demethylchlortetracycline, oxytetracycline, methacycline, doxycycline, rolitetracycline, minocycline, daunorubicin hydrochloride, doxorubicin, aclarubicin, kanamycin sulfate, bekanamycin, tobramycin, gentamycin sulfate, dibekacin, amikacin, micronomicin, ribostamycin, neomycin sulfate, paromomycin sulfate, streptomycin sulfate, dihydrostreptomycin, destomycin A, hygromycin B, apramycin, sisomicin, netilmicin sulfate, spectinomycin hydrochloride, astromicin sulfate, validamycin, kasugamycin, polyoxin, blasticidin S, erythromycin, erythromycin estolate, oleandomycin phosphate, tracetyloleandomycin, kitasamycin, josamycin, spiramycin, tylosin, ivermectin, midecamycin, bleomycin sulfate, peplomycin sulfate, gramicidin S, polymyxin B, bacitracin, colistin sulfate, colistinmethanesulfonate sodium, enramycin, mikamycin, virginiamycin, capreomycin sulfate, viomycin, enviomycin, vancomycin, actinomycin D, neocarzinostatin, bestatin, pepstatin, monensin, lasalocid, salinomycin, amphotericin B, nystatin, natamycin, trichomycin, mithramycin, lincomycin, clindamycin, clindamycin palpitate hydrochloride, flavophospholipol, cycloserine, pecilocin, griseofulvin, chloramphenicol, chloramphenicol palmitate, mitomycin C, pyrrolnitrin, fosfomycin, fusidic acid, bicozamycin, tiamulin, siccanin.

### EXPERIMENTAL PART

Of some compounds the absolute stereochemical configuration of the stereogenic carbon atom(s) therein was not experimentally determined. In those cases the stereochemically isomeric form which was first isolated is designated as "A" and the second as "B", without further reference to the actual stereochemical configuration. However, said "A" and "B" isomeric forms can be unambiguously characterized by a person skilled in the art, using art-known methods such as, for example, X-ray diffraction.

In case "A" and "B" are stereoisomeric mixtures, in particular mixtures of diastereoisomers, they can be further separated whereby the respective first fractions isolated are designated "A1" respectively "B1" and the second as "A2" respectively "B2", without further reference to the actual stereochemical configuration. However, said "A1", "A2" and "B1", "B2" isomeric forms, in particular said "A1", "A2" and "B1", "B2"enantiomeric forms, can be unambiguously characterized by a person skilled in the art, using art-known methods such as, for example, X-ray diffraction.

Hereinafter, "THF" is defined as tetrahydrofuran, "DMF" is defined as *N,N-*dimethylformamide, "DIPE" is defined as diisopropyl ether and "CDI" is defined as 1,1'-carbonyldiimidazole.

### A. Preparation of the intermediate compounds

### Example A1

### a) Preparation of intermediate 1

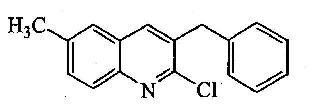

POCl₃ (327 ml) was added slowly at 5°C to DMF (120 ml). After complete addition, *N*-(4-methylphenyl)benzenepropanamide (0.501 mol) was added. The mixture was stirred at 80°C overnight, then brought to room temperature and poured out on ice. EtOAc was added. The mixture was stirred for 1 hour, while ice was added and then extracted with EtOAc. The organic layer was separated, washed with H₂O, dried (MgSO₄), filtered and the solvent was evaporated. Yield: 182.2 g of intermediate 1.

### b) Preparation of intermediate 2

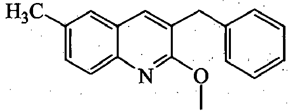

A mixture of intermediate 1 (0.5 mol) in CH₃ONa (30 %) (300 ml) and CH₃OH (300 ml) was stirred at 70°C for 48 hours. The mixture was brought to room temperature, poured out on ice and extracted with CH₂Cl₂. The organic layer was separated, washed with H₂O, dried (MgSO₄), filtered and the solvent was evaporated. The residue (120 g) was purified by column chromatography over silica gel (eluent: CH₂Cl₂/cyclohexane 30/70; 20-45 µm). The pure fractions were collected and the solvent was evaporated. Yield: 64 g of intermediate 2.

### Example A2

### a) Preparation of intermediate 3

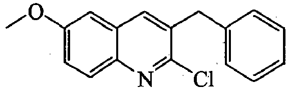

POCl₃ (2.74 mol) was added dropwise at 5°C/10°C to DMF (94 ml). *N*-(4-methoxyphenyl)benzenepropanamide (0.38 mol) was added. The mixture was stirred at 80°C overnight, then brought to room temperature and poured out on ice. The precipitate was filtered off, washed with H₂O and dried in vacuo. Yield: 41.5 g of intermediate 3 (37 %).

### b) Preparation of intermediate 4

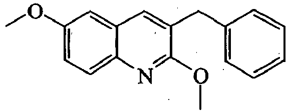

A mixture of intermediate 3 (0.14 mol) in CH₃ONa 30 % (90 ml) and CH₃OH (400 ml) was stirred and refluxed overnight. The mixture was brought to room temperature, poured out on ice and extracted with EtOAc. The organic layer was separated, dried (MgSO₄), filtered and the solvent was evaporated. The residue (38 g) was purified by column chromatography over silica gel (eluent: CH₂Cl₂/cyclohexane 65/35; 35-70 µm). The pure fractions were collected and the solvent was evaporated. Yield: 30 g of intermediate 4 (73 %).

### Example A3

### a) Preparation of intermediate 5

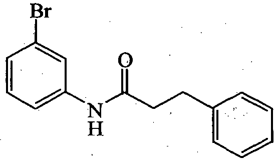

Benzenepropanoyl chloride (0.67 mol) was added dropwise at 5°C to a mixture of 3-bromobenzenamine (0.58 mol) and Et₃N (0.72 mol) in CH₂Cl₂ (1000 ml). The mixture was stirred at room temperature for 4 hours, poured out into ice water and NHₐOH. The organic layer was washed with HCl 1N, then with K₂CO₃ 10 %, dried (MgSO₄), filtered, and the solvent was evaporated till dryness. Yield: 190 g of intermediate 5.

### b) Preparation of intermediate 6 and 7

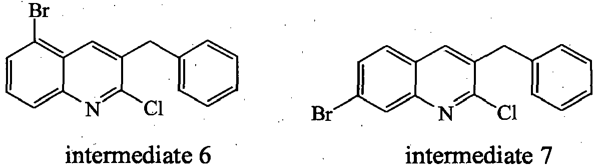

POCl₃ (2.3 mol) was added dropwise at 5°C to DMF (0.98 mol). The mixture was brought to room temperature. Intermediate 5 (0.33 mol) was added. The mixture was stirred at 85°C for 6 hours, then cooled to room temperature, poured out into ice water. CH₂Cl₂ was added. Both layers were stirred for 2 hours. The mixture was extracted with CH₂Cl₂. The organic layer was washed with K₂CO₃ 10 %, dried (MgSO₄), filtered and the solvent was evaporated. The residue (84g) was purified by column chromatography over silica gel (eluent: CH₂Cl₂/cyclohexane 30/70; 20-45 µm). The desired fractions were collected and the solvent was evaporated. Yield: 34.1g (31 %) of intermediate 6 and 9g (8 %) of intermediate 7.

### c. Preparation of intermediate 8

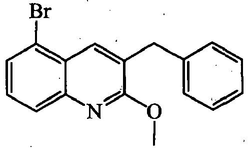

A mixture of intermediate 6 (0.1 mol) and NaOCH₃ (0.53 mol) in methanol (340 ml) was stirred and refluxed for 20 hours, then cooled to room temperature, poured out into ice water and extracted with CH₂Cl₂. The organic layer was separated, dried (MgSO₄), filtered, and the solvent was evaporated. Yield: 79 % of intermediate 8 (melting point: 100°C).

### Example A4

### a. Preparation of intermediate 9

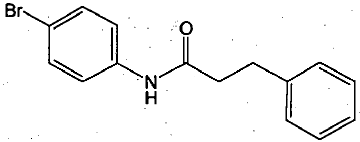

Benzenepropanoylchloride (0.488 mol) was added dropwise at room temperature to a solution of 4-bromobenzenamine (0.407 mol) in Et₃N (70 ml) and CH₂Cl₂ (700 ml) and the mixture was stirred at room temperature overnight. The mixture was poured out into water and concentrated NH₄OH, and extracted with CH₂Cl₂. The organic layer was dried (MgSO₄), filtered, and the solvent was evaporated. The residue was crystallized from diethyl ether. The residue (119.67 g) was taken up in CH₂Cl₂ and washed with HCl 1N. The organic layer was dried (MgSO₄), filtered, and the solvent was evaporated. Yield: 107.67 g of intermediate 9.

### b. Preparation of intermediate 10

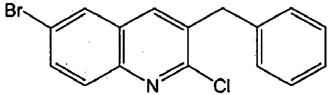

The reaction was carried out twice. POCl₃ (1.225 mol) was added dropwise at 10°C to DMF (0.525 mol). Then intermediate 9 (0.175 mol) was added at room temperature. The mixture was stirred overnight at 80°C, poured out on ice and extracted with CH₂Cl₂. The organic layer was dried (MgSO₄), filtered, and the solvent was evaporated. Yield: 77.62 g of intermediate 10 (67 %).

### c. Preparation of intermediate 11

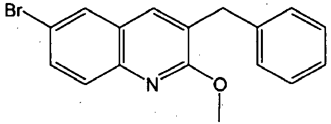

A mixture of intermediate 10 (0.233 mol) in CH₃ONa
(30 %) in methanol (222.32 ml) and methanol (776 ml) was stirred and refluxed overnight, then poured out on ice and extracted with CH₂Cl₂. The organic layer was separated, dried (MgSO₄), filtered and the solvent was evaporated . The residue was purified by column chromatography over silica gel (eluent: CH₂Cl₂/cyclohexane 20/80 and then 100/0; 20-45 µm). The pure fractions were collected and the solvent was evaporated. Yield: 25 g of intermediate 11 (33 %) (melting point: 84°C).

### Example A5

### a. Preparation of intermediate 12

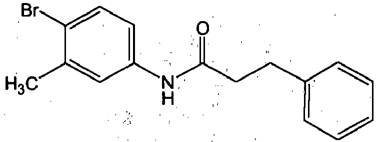

Benzenepropanoyl chloride (0.17 mol) was added dropwise at 5 °C to a mixture of 4-bromo-3-methylbenzenamine (0.13 mol) and Et₃N (0.18 mol) in CH₂Cl₂ (250 ml). The mixture was brought to room temperature, stirred for 16 hours, poured out into ice water and NH₄OH 30 % and extracted with CH₂Cl₂. The organic layer was washed with HCl 1N, H₂O and K₂CO₃ 10 %, dried (MgSO₄), filtered, and the solvent was evaporated. The residue was taken up in diethyl ether. The precipitate was filtered off and dried. Yield: 39 g of intermediate 12 (91 %).

### b. Preparation of intermediate 13

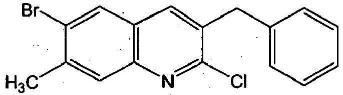

POCl₃ (0.8 mol) was added dropwise at 5°C to DMF (0.34 mol). The mixture was brought to room temperature. Intermediate 12 (0.11 mol) was added. The mixture was stirred at 85°C for 7 hours, then cooled to room temperature, poured out into ice water and extracted with CH₂Cl₂. The organic layer was separated, dried (MgSO₄), filtered, and the solvent was evaporated till dryness. The residue was crystallized from *i*PrOH. The precipitate was filtered, washed with iPrOH and dried. Yield: 13.9 g of intermediate 13 (35 %).

### c. Preparation of intermediate 14

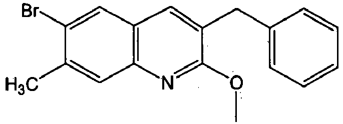

A mixture of intermediate 13 (0.04 ml) and CH₃ONa (0.2 mol) in CH₃OH (140 ml) was stirred and refluxed for 16 hours, then cooled to room temperautre, poured out into ice water and extracted with CH₂Cl₂. The organic layer was separated, dried (MgSO₄), filtered, and the solvent was evaporated. Yield: 13.5 g of intermediate 14 (98 %).

### Example A6A

### a. Preparation of intermediate 15

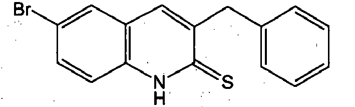

A mixture of intermediate 10 (prepared according to A4.b) (0.045 ml) and thiourea (0.05 mol) in ethanol (150 ml) was stirred and refluxed for 8 hours and then brought to room temperature. A solution of KOH (0.068 mol) in H₂O (15 ml) was added. The mixture was stirred and refluxed for 1 hour and poured out on ice. The precipitate was filtered off, washed with H₂O and dried. Yield: 11 g of intermediate 15 (74 %).

### b. Preparation of intermediate 16

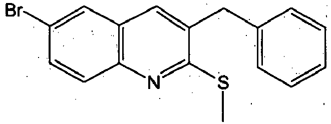

CH₃I (0.037 mol) was added slowly at room temperature to a mixture of intermediate 15 (0.033 mol) and K₂CO₃ (0.037 mol) in 2-propanone (150 ml). The mixture was stirred at room temperature for 8 hours, poured out into H₂O and extracted with CH₂Cl₂. The organic layer was separated, dried (MgSO₄), filtered and the solvent was evaporated. Yield: 11.2 g (97 %). Part of this fraction (2 g) was crystallized from diethyl ether. The precipitate was filtered off and dried. Yield: 1.45 g of intermediate 16(70%).

### Example A6B

### a. Preparation of intermediate 17

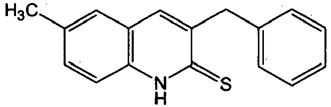

A solution of intermediate 1 (8 g, 0.03 mol) and thiourea (2.5 g, 0.033 mol) in ethanol (100 ml) was stirred at 80°C for 4 hours and was then cooled to room temperature. A solution of potassium hydroxide (2.5 g, 0.045 mol) in water (10 ml) was added and the mixture was heated for 1 hour at 80°C and was then cooled to room temperature and poured out into water. The precipitate was filtered off, washed with water and dried. Yield: 7.6 g of intermediate 17 (95 %).

### b. Preparation of intermediate 18

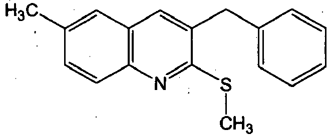

A solution of intermediate 17 (7.6 g, 0.029 mol), methyliodide (1.9 ml, 0.031 mol), and potassium carbonate (4.3 g, 0.031 mol) in acetone (170 ml) was stirred for 4 hours at room temperature, poured into water and extracted with CH₂Cl₂. The organic layer was washed with water, dried over MgSO₄, filtered and the solvent was evaporated. The residue was crystallized from ethylic ether. The precipitate was filtered off and dried. Yield: 5.83 g of intermediate 18 (73%) (melting point: 82°C). Intermediate 19 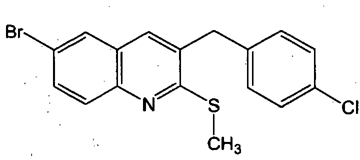 was prepared from intermediate 20 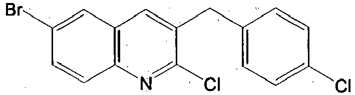 (prepared according to Example A2 in WO2004/011436 starting from 3-(4-chlorophenyl) propionic acid;yield: 88 g of intermediate 20 (70.7 %)) following the same procedure as outlined above in Example A6A and A6B. Yield: 94% of intermediate 19.

### Example A7

### a. Preparation of intermediate 21

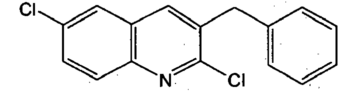

POCl₃ (3.234 mol) was added slowly at 5°C to DMF (111 ml). After complete addition, *N*-(4-chlorophenyl)benzenepropanamide (0.462 mol) was added. The mixture was stirred at 80°C overnight, then brought to room temperature and poured out on ice. EtOAc was added. The mixture was stirred for 1 hour while ice was added and then extracted with EtOAc. The organic layer was separated, washed with H₂O, dried (MgSO₄), filtered and the solvent was evaporated. Yield: 129 g of intermediate 21 (97 %).

### b Preparation of intermediate 22

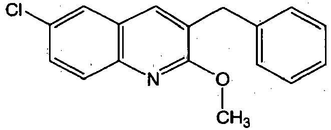

A mixture of intermediate 21 (0.447 mol) in CH₃ONa 30 % (300 ml) and CH₃OH (300 ml) was stirred at 80°C overnight. The mixture was brought to room temperature, poured out on ice and extracted with CH₂Cl₂. The organic layer was separated, washed with H₂O, dried (MgSO₄), filtered and the solvent was evaporated. The residue (82 g) was purified by column chromatography over silica gel (eluent: cyclohexane/CH₂Cl₂ 70/30; 20-45 µm). The pure fractions were collected and the solvent was evaporated. Yield: 45 g of intermediate 22 (35 %).

### Example A8

### a. Preparation of intermediate 23

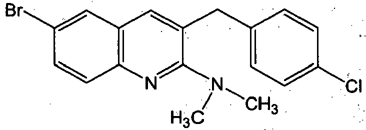

A solution of intermediate 20 (1.5 g, 0.00409 mol), dimethylamine hydrochloride (1.33 g, 0.001636 mol), potassium carbonate (2.83 g, 0.002045 mol) in acetonitrile (15 ml) was stirred for 20 hours at 80°C, poured out into water and extracted with diethylether. The organic layer was dried over MgSO₄, filtered and the solvent was evaporated. The residue (1.5 g) was purified by column chromatography over silica gel (eluent: cyclohexane/AcOEt: 97/3). The pure fractions were collected and the solvent was evaporated. Yield: 0.7 g of intermediate 23 (47 %).

### Example A9

### a. Preparation of intermediate 24

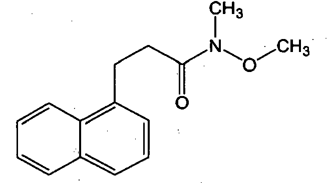

CDI (0.038 mol) was added at 5°C to a solution of 3-(1-naphthyl)-propionic acid (0.025 mol) in CH₂Cl₂ (60 ml). The mixture was stirred at 5°C for 1 hour. *N*-methoxymethanamine HCl (0.038 mol) was added. The mixture was stirred at room temperature overnight. HCl 1N was added. The mixture was extracted with CH₂Cl₂. The organic layer was washed with K₂CO₃ 10 %, dried (MgSO₄), filtered, and the solvent was evaporated. The residue was purified by column chromatography over silica gel (eluent: CH₂Cl₂ 100). The pure fractions were collected and the solvent was evaporated. Yield: 5.4 g of intermediate 24 (94 %).

### b. Preparation of intermediate 25

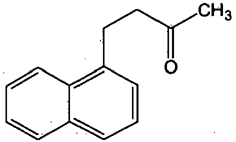

CH₃MgCl (0.025 mol) was added dropwise at 5°C to a solution of intermediate 24 (0.021 mol) in THF (51 ml). The mixture was stirred at 5°C for 2 hours, then brought to room temperature. A solution of NH₄Cl was added. The mixture was extracted with CH₂Cl₂. The organic layer was separated, dried (MgSO₄), filtered, and the solvent was evaporated. Yield: 3.7 g of intermediate 25 (89 %).

### c. Preparation of intermediate 26

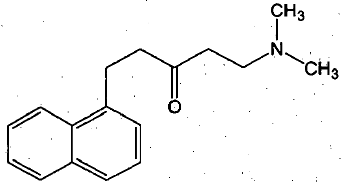

A mixture of intermediate 25 (0.019 mol), formaldehyde (0.076 ml) and *N*-methylmethanamine (0.076 mol) in concentrated HCl (0.8 ml) and EtOH (23 ml) was stirred and refluxed for 24 hours, then cooled to room temperature. EtOH was evaporated. The residue was taken up in EtOAc. The mixture was basified with NaHCO₃ and extracted with CH₂Cl₂. The organic layer was separated, dried (MgSO₄), filtered and the solvent was evaporated. The residue was purified by column flash chromatography over silica gel (eluent: CH₂Cl₂/CH₃OH 97/3; 15-40 µm). Two fractions were collected and the solvent was evaporated. The desired fraction yielded 1.17 g of intermediate 26. Intermediate 27 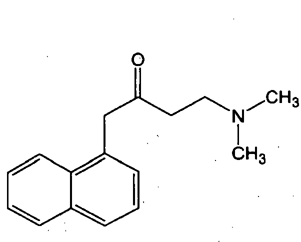 was prepared in the same way as intermediate 26. Yield: 18 % of intermediate 27 (oil).

### Example A 10

### a. Preparation of intermediate 28

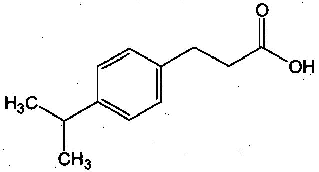

Formic acid (31.5 ml, 0.834 mol) was added dropwise to DMF (100 ml) under stirring and cooling with ice-cold water. Triethylamine (50.8 ml, 0.361 mol) was added in the same way followed by Meldrum's acid (40 g, 0.278 mol). After dissolution cuminaldehyde (0.278 mol) was added. The mixture was heated up to 80°C for 14 hours, then cooled down and poured out into 1 liter of ice-cold water under vigorous stirring. Concentrated HCl was added till pH 1-2. The precipitate was filtered off, washed with water and air-dried. Yield: 99% of intermediate 28.

### b. Preparation of intermediate 29

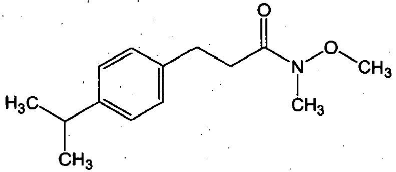

1,1'-carbonyldiimidazole (6.6 g, 0.041 mol) was added portionwise to a mixture of intermediate 28 (0.027 mol) in CH₂Cl₂ (50 ml) cooled in a ice bath at 5°C. The mixture was stirred 1 hour at 5°C and N-methoxymethanamine hydrochloride (4 g, 0.041 mol) was added and the suspension was stirred at room temperature for 20 hours. The mixture was poured out into HCl 1N and extracted with CH₂Cl₂. The organic layer was washed with K₂CO₃ 10%, dried over magnesium sulfate, filtered, and the solvent was evaporated. The residue was purified by column chromatography over silica gel (eluent: CH₂Cl₂: 100). The pure fractions were collected and the solvent was evaporated. Yield: 93% of intermediate 29 (93 %).

### c. Preparation of intermediate 30

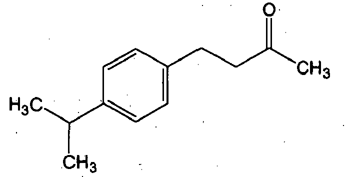

Methyl magnesium chloride (22 % in THF, 8.1 ml, 0.023 mol) was added slowly at 0°C under N₂ flow to a solution of intermediate 29 (0.019 mol) in THF (45 ml). The mixture was stirred at 0°C for 2 hours and hydrolyzed at 0°C with NH₄Cl 10%, and extracted with EtOAc. The organic layer was dried over MgSO₄, filtered, and the solvent was evaporated. The residue was used without further purification. Yield: 83% of intermediate 30 (83 %).

### d. Preparation of intermediate 31

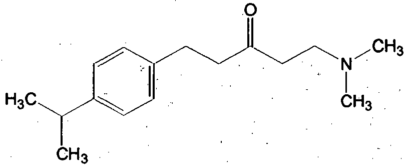

A mixture of intermediate 30 (0.019 mol), paraformaldehyde (2.3 g, 0.076 mol), dimethylamine hydrochloride (6.2 g, 0.076 mol) and hydrochloric acid concentrated (0.8 ml) in EtOH (23 ml) was stirred at reflux for 24 hours, then cooled down, and the solvent was evaporated. The residue was poured out into CH₂Cl₂, basified with NaHCO₃, and extracted with CH₂Cl₂. The organic layer was dried over MgSO₄, filtered, and the solvent was evaporated. The residue was purified by column chromatography over silica gel (eluent: CH₂Cl₂/MeOH : 97/3).The pure fractions of the two isomers were collected and the solvent was evaporated. Yield : 10 % of intermediate 31 (10%).

### Example A11

### a. Preparation of intermediate 32

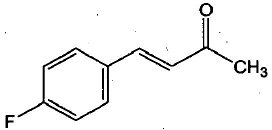

A solution of NaOH 1% (50 ml) was added portionwise to a mixture of 4-fluorobenzaldehyde (21.6 ml, 0.2 mol) and acetone (40 ml, 0.55 mol) in water (40 ml). The mixture was stirred for 2 hours at 65°C, then the mixture was poured out into ice water and extracted with ethyl acetate. The organic layer was washed with brine, dried over MgSO₄, filtered, and the solvent was evaporated. The residue was used without further purification in the next step as an oil. Yield: 34 g of intermediate 32 (100 %).

### b. Preparation of intermediate 33

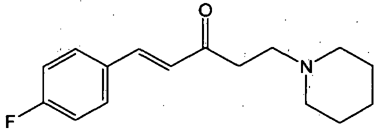

A mixture of intermediate 32 (4 g, 0.0244 mol), paraformaldehyde (1.1 g, 0.0365 mol), piperidine hydrochloride (0.0244 mol) and hydrochloric acid concentrated (0.8 ml) in EtOH (6 ml) was stirred at reflux for 24 hours, cooled, and the solvent was evaporated. The precipitate was filtered off, washed with EtOH and dried under vacuum at 60°C to afford intermediate 33 (63 %).

### c. Preparation of intermediate 34

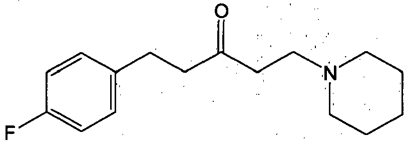

A mixture of intermediate 33 (7.34 mmol), palladium on activated carbon 10% (0.22 g) in EtOH/H₂O (22 ml, 50/50) was stirred under hydrogen atmosphere at room temperature for 2 hours. The mixture was filtered over celite, washed with EtOH, and the solvent was evaporated. The residue was treated by a solution of NaOH 1N in Et₂O. The organic layer was separated, and washed with brine, dried (MgSO₄), filtered, and the solvent was evaporated. The residue was used without further purification in the next step as an oil. Yield: 76% of intermediate 34.

### Example A12

### a. Preparation of intermediate 35

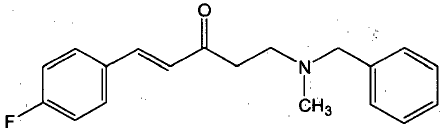

A mixture of intermediate 32 (4.8 g, 0.0292 mol), paraformaldehyde (1.32 g, 0.0439 mol), *N*-benzylmethylamine hydrochloride (4.6 g, 0.0292 mol) and hydrochloric acid concentrated (0.8 ml) in EtOH (100 ml) was stirred at reflux for 18 hours, cooled, and the solvent was evaporated. The precipitate was filtered off, washed with acetone and dried under vacuum at 60°C. Yield: 3.8 g of intermediate 35 (39 %).

### b. Preparation of intermediate 36

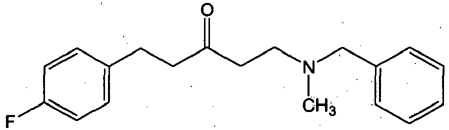

A mixture of intermediate 35 (3.8 g, 0.0114 mol), palladium on activated carbon 10% (0.38 g) in EtOH/H₂O (38 ml, 50/50) was stirred under hydrogen atmosphere at room temperature for 2 hours. The mixture was filtered over celite, washed with EtOH, and the solvent was evaporated. The residue was treated by a solution of NaOH 1N in Et₂O. The organic layer was separated, and washed with brine, dried (MgSO₄), filtered, and the solvent was evaporated. The residue (2.5 g) was purified by column chromatography over silica gel (eluent: CH₂Cl₂/CH₃OH; 99/1; 15-40 µm). The pure fraction was collected and the solvent was evaporated. Yield: 0.75 g of intermediate 36 (22%).

### Example A 13

### a. Preparation of intermediate 37

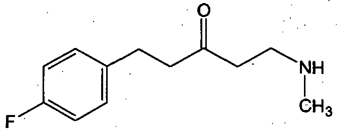

A mixture of intermediate 35 (2.3 g, 0.00689 mol), palladium on activated carbon 10 % (0.23 g) in EtOH/H₂O (24 ml, 50/50) was stirred under hydrogen atmosphere at room temperature for 3 hours. The mixture was filtered over celite, washed with EtOH, and the solvent was evaporated. The residue was treated by a solution of NaOH 1N in Et₂O. The organic layer was separated, and washed with brine, dried (MgSO₄), filtered, and the solvent was evaporated. The residue was used without further purification in the next step as oil. Yield: 1.3 g of intermediate 37 (90 %).

### Example A14

### a. Preparation of intermediate 38

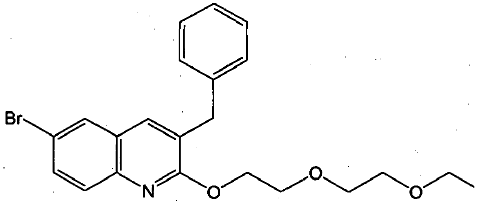

NaH (60 % in oil; 0.0072 mol) was added portionwise at 0 °C to a solution of 2-(2-ethoxyethoxy)-ethanol (0.0072 mol) in THF (12.5 ml) under N₂ flow. The mixture was stirred at 0°C for 1 hour. A solution of intermediate 10 (0.006 mol) in THF (12.5 ml) was added dropwise. The mixture was stirred and refluxed for 18 hours and was then cooled to room temperature. EtOAc and H₂O were added. The organic layer was washed with H₂O and then with saturated NaCl. The separated organic layer was dried (MgSO₄), filtered and the solvent was evaporated. Yield: 2.5 g intermediate 38 (97 %).

### Example A15

### Preparation of intermediate 39

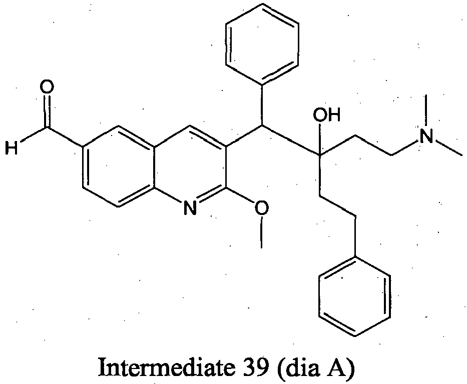

nBuLi 1.6M in hexane (0.0018 mol) was added dropwise at -70°C to a solution of compound 14 (0.0007 mol) in THF (4ml) under N₂- flow. The mixture was stirred for 2 hours. *N,N*-dimethylformamide (0.0037 mol) was added slowly. The mixture was stirred at -70°C for 2 hours, poured out into H₂O and extracted with EtOAc. The organic layer was washed with saturated NaCl, dried (MgSO₄), filtered and the solvent was evaporated. Yield: 0.38 g of intermediate 39 (100%).

### B. Preparation of the final compounds

### Example B1

### a. Preparation of compounds 1 and 2

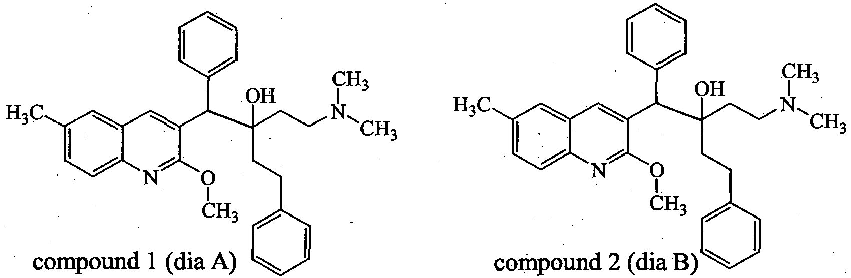

nBuLi 1.6M (0.0084 mol) was added dropwise at -20°C to a solution of *N*-(1-methylethyl)-2-propanamine (0.0084 mol) in THF (24 ml). The mixture was stirred at -20°C for 20 minutes, then cooled to -70°C. A solution of intermediate 2 (prepared according to A1.b) (0.0076 mol) in THF (20 ml) was added. The mixture was stirred at -70°C for 1 hour and 30 minutes. A solution of 1-(dimethylamino)-5-phenyl-3-pentanone (0.0107 mol) in THF (22 ml) was added. The mixture was stirred at -70°C for 3 hours, poured out into -30°C and extracted with CH₂Cl₂. The organic layer was separated, dried (MgSO₄), filtered and the solvent was evaporated. The residue (4.3 g) was purified by column chromatography over silica gel (eluent: CH₂Cl₂/CH₃OH/NH₄OH 97/3/0.2; 15-40 µm). The pure fractions were collected and the solvent was evaporated. The residue was purified twice by column chromatography over kromasil (eluent: CH₃CN/NH₄HCO₃ 0.5 % 85/15; 10 µm). Three fractions were collected and the solvent was evaporated. Yield: 0.155 g of fraction 1; 0.08 g of fraction 2 and 0.1 g of fraction 3. Fraction 1 and fraction 3 were crystallized from DIPE. The precipitate was filtered off and dried. Yield: 0.14 g of final compound 1 (8 %) (diastereoisomer A; melting point: 142°C) and 0.102 g of final compound 2 (6 %) (diastereoisomer B; melting point: 159°C).

### b-1. Preparation of compounds 3 and 4

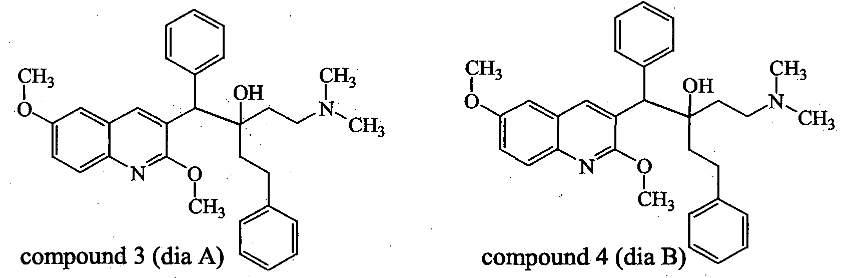

*n*BuLi 1.6M (0.0095 mol) was added dropwise at -20°C to a solution of *N*-(1-methylethyl)-2-propanamine (0.0095 mol) in THF (26 ml). The mixture was stirred at -20°C for 20 minutes, then cooled to -70°C. A solution of intermediate 4 (prepared according to A2.b) (0.0086 mol) in THF (24 ml) was added. The mixture was stirred at -70°C for 1 hour and 30 minutes. A solution of 1-(dimethylamino)-5-phenyl-3-pentanone (0.012 mol) in THF (25 ml) was added. The mixture was stirred at -70°C for 3 hours, poured out on ice at -30°C and extracted with CH₂Cl₂. The organic layer was separated, dried (MgSO₄), filtered and the solvent was evaporated. The residue (5.2 g) was purified by column chromatography over silica gel (eluent: CH₂Cl₂/CH₃OH/NH₄OH 97/3/0.1; 15-40µm). The pure fractions were collected and the solvent was evaporated. The residue (0.2 g) was purified by column chromatography over kromasil (eluent: cyclohexane/*i*PrOH/NH₄OH 95/5/0.3; 10 µm). The desired fractions were collected and the solvent was evaporated. Yield: 0.035 g of final compound 3 (3 %) (diastereoisomer A) and 0.03 g of final compound 4 (2.8 %) (diastereoisomer B).

### b-2. Preparation of compounds 3 and 4

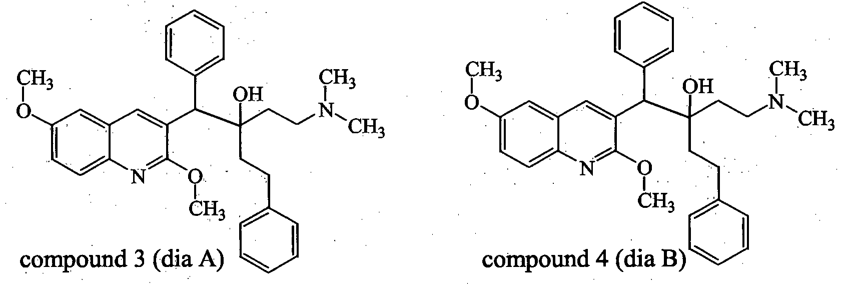

nBuLi 1.6M (0.0118 mol) was added dropwise at -20°C to a solution of *N*-(1-methylethyl)-2-propanamine. (0.0118 mol) in THF (30 ml). The mixture was stirred at -20°C for 20 minutes, then cooled to -70°C. A solution of intermediate 4 (prepared according to A2.b) (0.0107 mol) in The (35 ml) was added. The mixture was stirred at -70°C for 1 hour and 30 minutes. A solution of 1-(dimethylamino)-5-phenyl-3-pentanone (0.015 mol) in THF (30 ml) was added. The mixture was stirred at -70°C for hours, poured out on ice at -30°C and extracted with EtOAc. The organic layer was separated, dried (MgSO₄), filtered, and the solvent was evaporated. The residue was purified by column chromatography over silica gel (eluent: CH₂Cl₂/CH₃OH/NH₄OH 97/3/0.1 then CH₂Cl₂/*i*PrOH/NH₄OH 95/5/0.4; 15-40 µm). Two fractions were collected and the solvent was evaporated. Yield: 0.13 g of fraction 1 and 0.12 g of fraction 2. Fraction 1 was crystallized from DIPE. The precipitate was filtered off and dried. Yield: 0.063 g of final compound 3 (diastereoisomer A). Fraction 2 was crystallized from DIPE. The precipitate was filtered off and dried. Yield: 0.066 g of final compound 4 (diastereoisomer B).

### c. Preparation of compounds 5 and 6

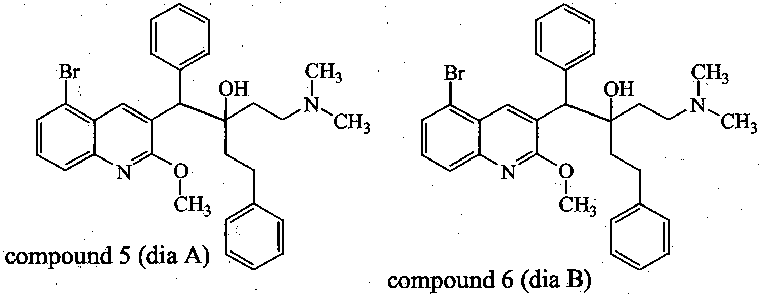

nBuLi 1.6M (0.0084 mol) was added dropwise at -20°C to a solution of *N*-(1-methylethyl)-2-propanamine (0.0084 mol) in THF (24 ml). The mixture was stirred at -20°C for 20 minutes, then cooled to -70°C. A solution of intermediate 8 (prepared according to A3.c) (0.0076 mol) in THF (25 ml) was added. The mixture was stirred at -70°C for 1 hour and 30 minutes. A solution of 1-(dimethylamino)-5-phenyl-3-pentanone (0.0107 mol) in THF (22 ml) was added. The mixture was stirred at -70°C for 3 hours, poured out on ice at -30°C and extracted with CH₂Cl₂. The organic layer was separated, dried (MgSO₄), filtered and the solvent was evaporated. The residue (5.1 g) was purified by column chromatography over silica gel (eluent: CH₂Cl₂/CH₃OH/NH₄OH 97/3/0.1 then toluene/*i*PrOH/NH₄OH 95/5/0.1; 15-40µm). Three fractions were collected and the solvent was evaporated. Yield: 0.87 g of fraction 1; 0.7 g of fraction 2 and 0.4 g of fraction 3. Fraction 3 was purified by column chromatography over kromasil (eluent: toluene/*i*PrOH/NH₄OH 99/1/0.05; 10µm). Two fractions were collected and the solvent was evaporated. Yield: 0.15 g of fraction A and 0.139 g of fraction B. Fraction B was crystallized from DIPE. The precipitate was filtered off and dried. Yield: 0.585 g of final compound 5 (30 %) (diastereoisomer A; melting point: 156°C). Fraction A was crystallized from DIPE. The precipitate was filtered off and dried. Yield: 0.15 g of final compound 6 (8 %) (diastereoisomer B; melting point: 126°C).

### d. Preparation of compounds 7 and 8

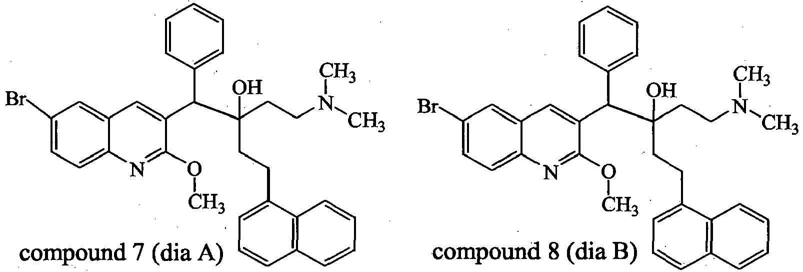

A solution of intermediate 11 (prepared according to A4.c) (0.0035 mol) in THF (12 ml) was added dropwise at -70°C to a solution of *N*-(1-methylethyl)-2-propanamine lithium salt (0.0038 mol) in THF (19 ml). The mixture was stirred at -70°C for 1 hour and 30 minutes. A solution of intermediate 26 (prepared according to A9.c) (0.0046 mol) in THF (12 ml) was added. The mixture was stirred at -70°C for 3 hours, poured out into -30°C and extracted with CH₂Cl₂. The organic layer was separated, dried (MgSO₄), filtered, and the solvent was evaporated. The residue (2.2 g) was purified twice by column chromatography over silica gel (eluent: CH₂Cl₂/CH₃OH/NH₄OH 98/2/0.1; 15-40 µm). Three fractions were collected and the solvent was evaporated. Yield: 0.3 g of fraction 1 (dia A), 0.027 g of fraction 2 and 0.242 g of fraction 3 (dia B). Fraction 1 was crystallized from DIPE. The precipitate was filtered off and dried. Yield: 0.26 g of final compound 7 (25 %) (diastereoisomer A; melting point: 206°C). Fraction 3 was crystallized from DIPE. The precipitate was filtered off and dried. Yield: 0.128 g of final compound 8 (12 %) (diastereoisomer B; melting point: 160°C).

### e. Preparation of compound 9

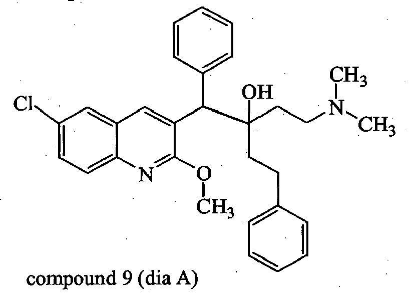

nBuLi (0.0084 mol) was added at -20°C to a solution of *N*-(1-methylethyl)-2-propanamine (0.0084 mol) in THF (25 ml). The mixture was stirred at -20°C for 20 minutes, then cooled to -70°C. A solution of intermediate 22 (prepared according to A7b) (0.0076 mol) in THF (26 ml) was added. A solution of 1-(dimethylamino)-5-phenyl-3-pentanone (0.0107 mol) in THF (24 ml) was added. The mixture was stirred at -70°C for 3 hours, poured out on ice at -30°C and extracted with CH₂Cl₂. The organic layer was separated, dried (MgSO₄), filtered, and the solvent was evaporated. The residue was purified by column chromatography over silica gel (eluent: CH₂Cl₂/CH₃OH/NH₄OH 98/2/0.1; 15-40 µm) then purified by column chromatography over kromasil (eluent: CH₂Cl₂/CH₃OH/NH₄OH 99/1/0.05). Three fractions were collected and the solvent was evaporated. Yield: 0.44 g of fraction 1 (dia A), 0.257 g of fraction 2 and 0.02 g of fraction 3. Fraction 1 was crystallized from DIPE. The precipitate was filtered off and dried. Yield: 0.14 g of final compound 9 (melting point: 172°C).

### f. Preparation of compounds 10 and 11

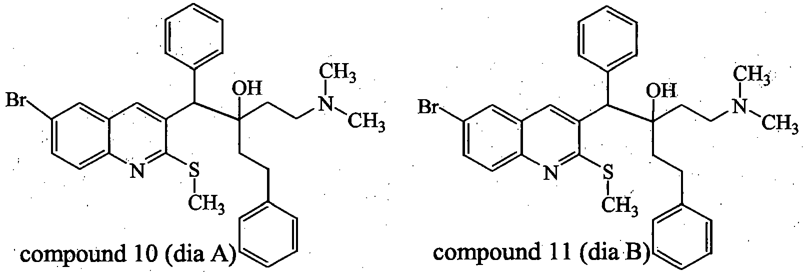

*n*BuLi 1.6M(0.0084 mol) was added dropwise at -20°C to a solution of *N*-(1-methylethyl)-2-propanamine (0.0084 mol) in THF (24 ml). The mixture was stirred at -20°C for 20 minutes, then cooled to -70°C. A solution of intermediate 16 (prepared according to A6A.b) (0.0076 mol) in THF (26 ml) was added. The mixture was stirred at -70°C for 1 hour and 30 minutes. A solution of 1-(dimethylamino)-5-phenyl-3-pentanone (0.0107 mol) in THF (22 ml) was added. The mixture was stirred at -70°C for 3 hours, then poured out on ice at 30°C and extracted with CH₂Cl₂. The organic layer was separated, dried (MgSO₄), filtered and the solvent was evaporated. The residue (4.8 g) was purified by column chromatography over silica gel (eluent: CH₂Cl₂/CH₃OH/NH₄OH 98/2/0.1; 15-40 µm). Two fractions were collected and the solvent was evaporated. Yield: 0.52 g of fraction 1 and 0.42 g of fraction 2. Both fractions were crystallized from DIPE. The precipitate was filtered off and dried. Yield: 0.47 g of final compound 10 (23 %) (diastereoisomer A; melting point: 191 °C) and 0.27 g of final compound 11 (7 %) (diastereoisomer B; melting point: 179°C).

### g. Preparation of compounds 17, 18, 19 and 20

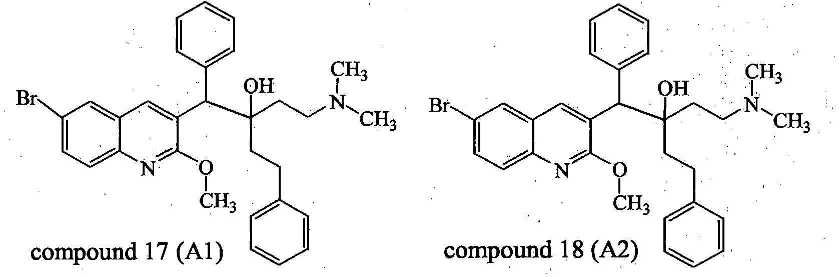

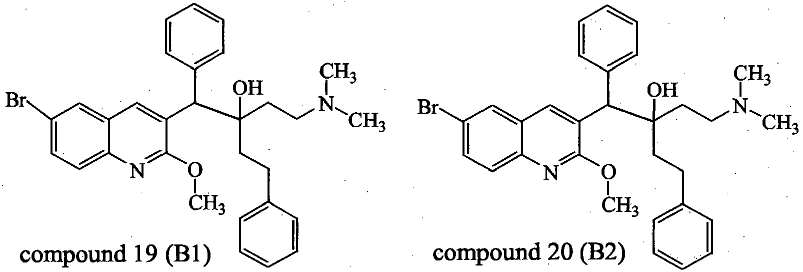

*n*BuLi 1.6 M (0.0114 mol) was added dropwise at -20°C to a solution of *N*-(1-methylethyl)-2-propanamine (0.0114 mol) in THF (32 ml). The mixture was stirred at -20°C for 20 minutes, then cooled to -70°C. A solution of intermediate 11 (prepared according to A4.c) (0.0104 mol) in THF (34 ml) was added. The mixture was stirred for 1 hour and 30 minutes. A solution of 1-(dimethylamino)-5-phenyl-3-pentanone (0.0146 mol) in THF (30 ml) was added. The mixture was stirred at -70°C for 3 hours, then poured out into -30°C and extracted with CH₂Cl₂. The organic layer was separated, dried (MgSO₄), filtered, and the solvent was evaporated. The residue (5.3 g) was purified twice by column chromatography over silica gel (eluent: CH₂Cl₂/CH₃OH/NH₄OH 98/2/0.1; 15-40 µm). Two fractions were collected and the solvent was evaporated. Yield : 0.45g F1 and 0.22g F2. Both fractions were crystallized from DIPE. The precipitate was filtered off and dried. Yield: 0.154g F3 (dia A) and 0.11g F4 (dia B). F3 was divided into two enantiomers by Chiral PAK AD (eluent: EtOH 100; 20 µm). Two fractions were collected and the solvent was evaporated. Each fraction was crystallized separately from DIPE/diethyl ether. The precipitate was filtered off and dried. Yield: 0.19 g of compound 17 (A1) and 0.175 g of compound 18 (A2). F4 was divided into two enantiomers by Chiral PAK AD (eluent: EtOH/iPrOH 90/10; 20 µm). Two fractions were collected and the solvent was evaporated. Each fraction was crystallized separately from DIPE/diethyl ether. The precipitate was filtered off and dried. Yield: 0.1 g of compound 19 (B1) and 0.1 g of compound 20 (B2).

### h. Preparation of compounds 21 and 22

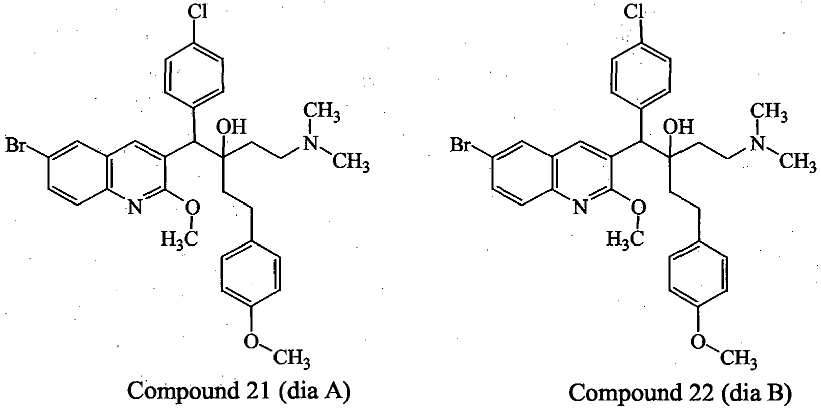

nBuLi 1.6 M in hexane (3.4 ml, 0.0055 mol) was added slowly at -20°C under N₂ flow to a solution of diisopropylamine (0.78 ml, 0.0055 mol) in THF (8.5 ml). The mixture was stirred at -20°C for 20 minutes, then cooled at -70°C. A solution of 3-(4-chloro-benzyl)-6-bromo-2-methoxy-quinoline (1.67 g, 0.0046 mol) in THF (34 ml) was added slowly. The mixture was stirred at -70°C for 1 hour and 30 minutes. A solution of 1-(dimethylamino)-5-(4-methoxy-phenyl)-pentan-3-one (1.13 g, 0.0055 mol) in THF (30 ml) was added slowly. The mixture was stirred at -70°C for 2 hours, hydrolyzed at -30°C with ice water, and extracted with EtOAc. The organic layer was separated, dried over MgSO₄, filtered, and the solvent was evaporated. The residue was purified by column chromatography over silica gel (eluent: CH₂Cl₂/CH₃OH/NH₄OH; 97/3/0.1; 15-40 µm). One fraction was collected and the solvent was evaporated. This fraction was purified by Super Critical Fluid Chromatography (SCF) (CO₂/MeOH/2-propanol: 95/5/0.5, column cyano). Two fractions were collected and the solvent was evaporated. Fractions were separately crystallized from diisopropylether. Yield: 0.220 g of final compound 21 (8 %) (diastereoisomer A; melting point: 142°C) as a white solid and 0.09 g of final compound 22 (3.3 %) (diastereoisomer B; melting point: 160°C) as a white solid.

### i. Preparation of compounds 23 and 24

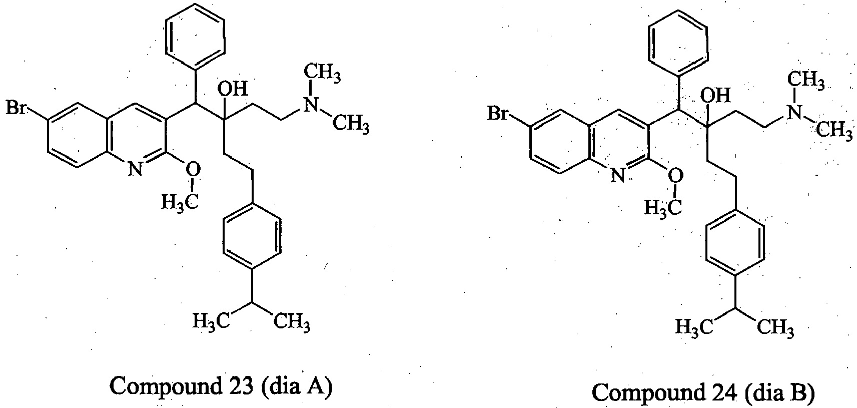

nBuLi 1.6 M in hexane (3.4 ml, 0.0055 mol) was added slowly at -20°C under N₂ flow to a solution of diisopropylamine (0.78 ml, 0.0055 mol) in THF (8.5 ml). The mixture was stirred at -20°C for 20 minutes, then cooled at -70°C. A solution of intermediate 31 (0.0046 mol) in THF (34 ml) was added slowly. The mixture was stirred at -70°C for 1 hour and 30 minutes. A solution of intermediate 24 (0.0055 mol) in THF (30 ml) was added slowly. The mixture was stirred at -70°C for 2 hours, hydrolyzed at -30°C with ice water, and extracted with EtOAc. The organic layer was separated, dried over MgSO₄, filtered, and the solvent was evaporated. The residue was purified by column chromatography over silica gel (eluent: CH₂Cl₂/CH₃OH/NH₄OH; 97/3/0.1; 15-40 µm). One fraction was collected and the solvent was evaporated. This fraction was purified by SFC (CO₂/MeOH/2-propanol: 95/5/0.5, column cyano). Two fractions were collected and the solvent was evaporated. Fractions were separately crystallized from diisopropylether. Yield: Final compound 23 (5 %) (diastereoisomer A) as a white foam and final compound 24 (2.3 %) (diastereoisomer B) as a white foam.

### j. Preparation of compounds 29 and 30

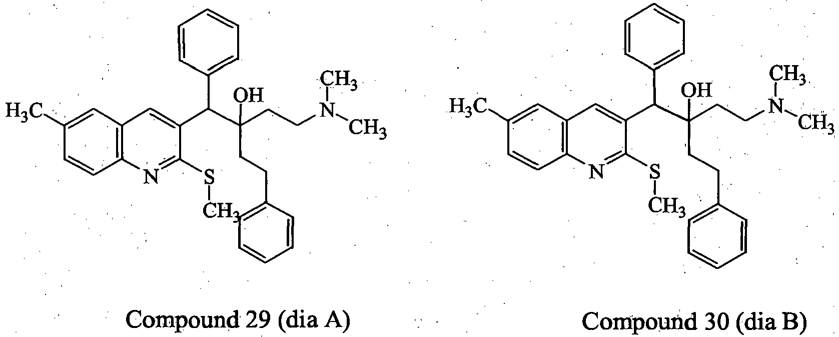

Compounds 29 and 30 were prepared according to the procedure for compounds 14 and 15, but starting from intermediate 18 and 1-(dimethylamino)-5-phenyl-3-pentanone (prepared in the same way as described in J.Am.Chem.Soc., 1950, 72, 718-721). Yield: Final compound 29 (4 %) (diastereoisomer A, melting point: 180°C) and final compound 30 (5 %) (diastereoisomer B, melting point: 120°C).

### k. Preparation of compounds 31 and 32

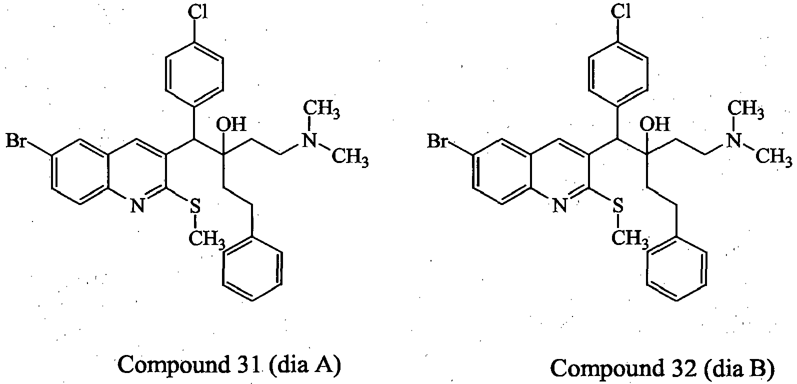

Compounds 31 and 32 were prepared in the same way as compounds 21 and 22, but starting from intermediate 19 and 1-(dimethylamino)-5-phenyl-3-pentanone (prepared in the same way as described in J.Am.Chem.Soc., 1950, 72, 718-721).
Yield: Final compound 31 (9 %) (diastereoisomer A) and final compound 32 (diastereoisomer B, melting point: 222°C).

### l. Preparation of compounds 34 and 35

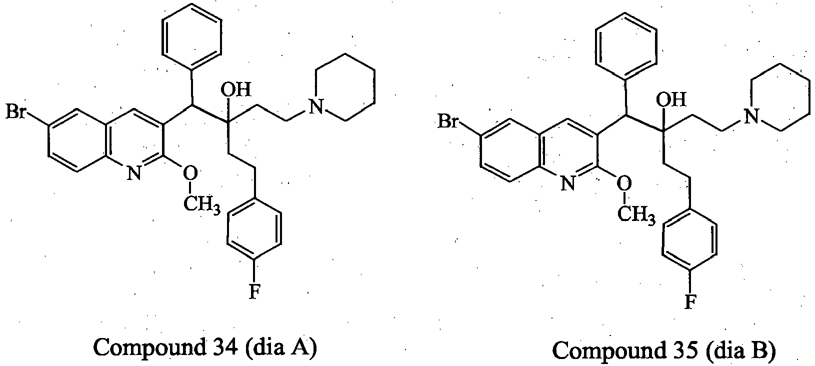

nBuLi 1.6M in hexane (2.3 ml, 3.66 mmol) was added slowly at -20°C under N₂ flow to a solution of diisopropylamine (0.513 ml, 3.66 mmol) in THF (8 ml). The mixture was stirred at -20°C for 20 minutes, and then cooled at -70°C. A solution of intermediate 11 (1.0 g, 3.05 mmol) in THF (10 ml) was added slowly. The mixture was stirred at -70°C for 1 hour. A solution of intermediate 34 (0.96 g, 3.66 mmol) in THF (10 ml) was added slowly. The mixture was stirred at -70°C for 1 hour, hydrolyzed at -30°C with ice water, and extracted with EtOAc. The organic layer was separated, dried over MgSO₄, filtered, and the solvent was evaporated. The residue was purified by column chromatography over silica gel (eluent: CH₂Cl₂/CH₃OH/NH₄OH; 99/1/0.05; 15-40 µm). Two fractions were collected and the solvent was evaporated. The fractions were separately crystallized from methanol. Yield: 0.15 g of final compound 34 (8 %) (diastereoisomer A, melting point: 194°C) as a white solid and 0.13 g of final compound 35 (7 %) (diastereoisomer B, melting point: 170°C) as a white solid.

### m. Preparation of compounds 39 and 40

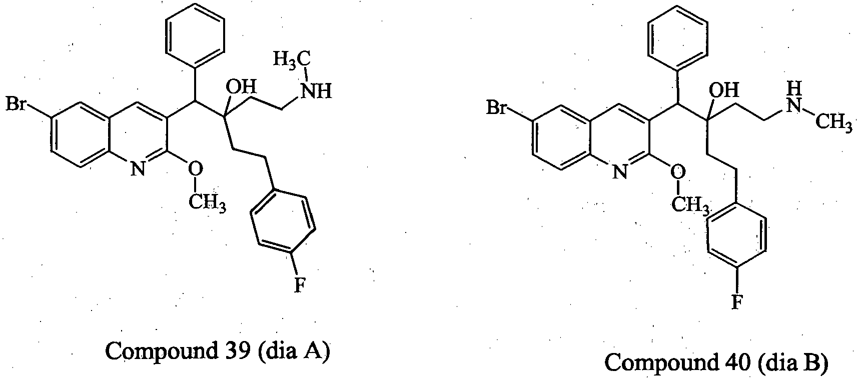

nBuLi 1.6M in hexane (8.1 ml, 0.013 mol) was added slowly at -20°C under N₂ flow to a solution of diisopropylamine (1.83 ml, 0.013 mol) in THF (30 ml). The mixture was stirred at -20°C for 20 minutes, and then cooled at -70°C. A solution of intermediate 11 (4.1 g, 0.0124 mol) in THF (40 ml) was added slowly. The mixture was stirred at -70°C for 1 hour and 30 minutes. A solution of intermediate 37 (1.3 g, 0.00662 mol) in THF (13 ml) was added slowly. The mixture was stirred at -70°C for 1 hour, hydrolyzed at -30°C with ice water, and extracted with EtOAc. The organic layer was separated, dried over MgSO₄, filtered, and the solvent was evaporated. The residue (5.7 g) was purified by column chromatography over silica gel (eluent: CH₂Cl₂/CH₃OH/NH₄OH; 94/6/0.1; 15-40 µm). Two fractions were collected and the solvent was evaporated. The fractions were separately crystallized from DIPE. Yield: 0.106 g of final compound 39 (2 %) (diastereoisomer A, melting point: 140°C) as a white solid and 0.068 g of final compound 40 (1 %) (diastereoisomer B, melting point: 250°C) as a white solid.

### n. Preparation of compounds 41 and 42

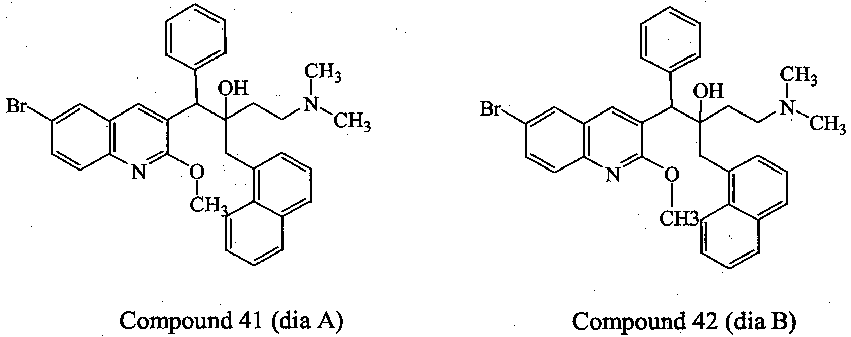

nBuLi 1.6M in hexane (3 ml, 0.0048 mol) was added slowly at -20°C under N₂ flow to a solution of diisopropylamine (0.67 ml, 0.0048 mol) in THF (14 ml). The mixture was stirred at -20°C for 20 minutes, then cooled at -70°C. A solution of intermediate 11 (1.44 g, 0.0044 mol) in THF (15 ml) was added slowly. The mixture was stirred at -70°C for 1 hour and 30 minutes. A solution of intermediate 27 (1.5 g, 0.0062 mol) in THF (15 ml) was added slowly. The mixture was stirred at -70°C for 3 hours, hydrolyzed at -30°C with ice water, and extracted with EtOAc. The organic layer was separated, dried over MgSO₄, filtered, and the solvent was evaporated. The residue (3.2 g) was purified by column chromatography over C18 (eluent: CH₃OH/NH₄HCO₃: 95/5; Kromasil C18, 10 µm). Two fractions were collected and the solvent was evaporated. The fractions were crystallized separately from diisopropyether and diethylether. Yield: 0.045g of final compound 41 (3 %) (diastereoisomer A, melting point: 112°C) as a white solid and 0.2 g of final compound 42 (12 %) (diastereoisomer B, melting point: 124°C) as a white solid.

### o. Preparation of compounds 43 and 44

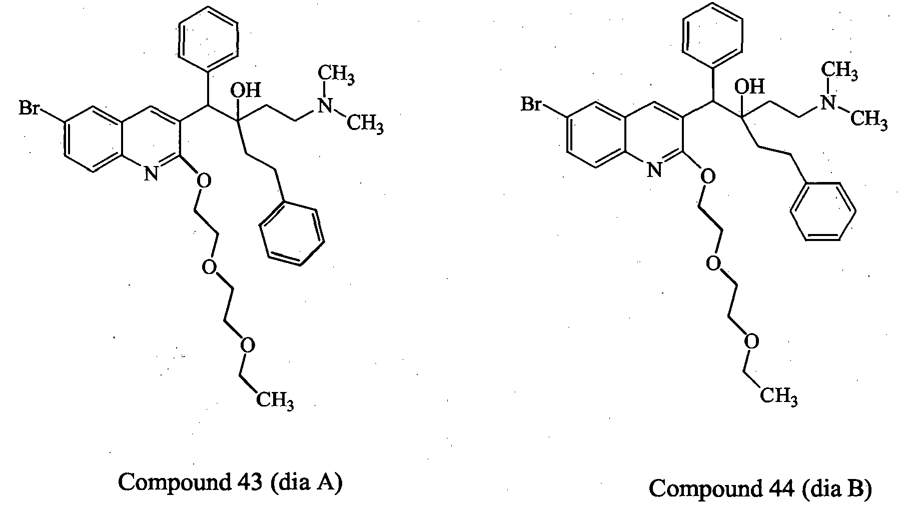

nBuLi 1.6M in hexane (4.1 ml, 0.0066 mol) was added dropwise at -20°C under N₂ flow to a solution of diisopropylamine (0.93 ml, 0.0066 mol) in THF (12 ml). The mixture was stirred at -20°C for 20 minutes, then cooled at -70°C. A solution of intermediate 38 (2.6 g, 0.0060 mol) in THF (27 ml) was added. The mixture was stirred at -70°C for 1 hour and 30 minutes. A solution of 1-(dimethylamino)-5-phenyl-3-pentanone (prepared in the same way as described in J.Am.Chem.Soc., 1950, 72, 718-721) (1.7 g, 0.0084 mol) in THF (20 ml) was added. The mixture was stirred at -70°C for 3 hours, hydrolyzed at -30°C with ice water, and extracted with EtOAc. The organic layer was separated, dried over MgSO₄, filtered, and the solvent was evaporated. The residue (2.5 g) was purified by column chromatography over silica gel (eluent: CH₂Cl₂/CH₃OH/NH₄OH: 97/3/0.1; 15-40 µm). Two fractions were collected and the solvent was evaporated, Yield: 0:15 g fraction 1 and 0.22 g fraction 2. Fraction 1 was crystallized from DIPE/diethyl ether. The precipitate was filtered off and dried. Yield: 0.129 of final compound 43 (3.4 %) (diastereoisomer A, melting point: 94°C) Fraction 2 was repurified by column chromatography over silica gel (eluent: CH₂Cl₂/CH₃OH/NH₄OH: 97/3/0.1; 15-40 µm) and crystallized from DIPE/diethyl ether. The precipitate was filtered off and dried. 0.059 g of final compound 44 (2 %) (diastereoisomer B, melting point: 103°C).

### Example B2

### a. Preparation of compound 12

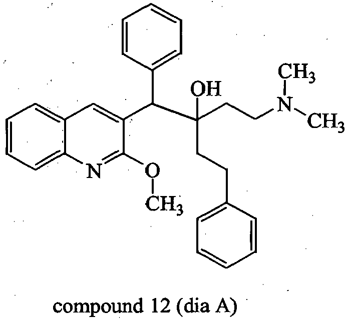

A mixture of final compound 5 (prepared according to Bl.c) (0.282 mol) and HCOONH₄ (1.41 mol) in Pd/C (0.15 ml) and CH₃OH (3 ml) was stirred and refluxed for 30 minutes, then cooled to room temperature, filtered over celite and washed with CH₂Cl₂. The filtrate was washed with H₂O, then with saturated NaCl. The organic layer was separated, dried (MgSO₄), filtered and the solvent was evaporated. The residue was crystallized from DIPE. The precipitate was filtered off and dried. Yield: 0.11 g of final compound 12 (86 %) (melting point: 122°C).

### b. Preparation of compound 36

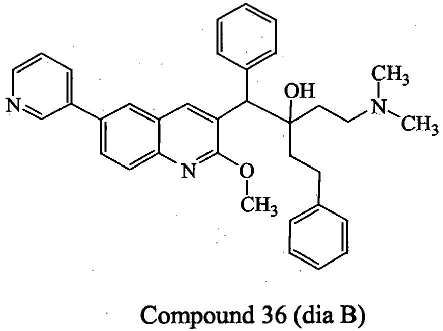

A solution of final compound compound 15 (0.25 g, 0.00047 mol), pyridine-3-boronic acid (0.116 g, 0.00094 mol) and tetrakis (triphenylphosphine) palladium(0) (0.054 g, 0.000047 mol) in ethyleneglycol dimethyl ether (13 ml) and a solution of sodium carbonate 2M (0.94 ml) was stirred overnight at 80°C. Then the solution was cooled to room temperature, poured out into water and extracted with CH₂Cl₂. The organic layer was separated, dried over MgSO₄, filtered, and the solvent was evaporated. The residue (0.3 g) was purified by column chromatography over silica gel (eluent: CH₂Cl₂/CH₃OH/NH₄OH; from 99/1/0.1 to 94/6/0.6; 15-30 µm). The pure fraction was collected and the solvent was evaporated. Yield: 0.024 g of final compound 36 (9.6 %).

### Example B3

### a. Preparation of compounds 25, 26, 27 and 28

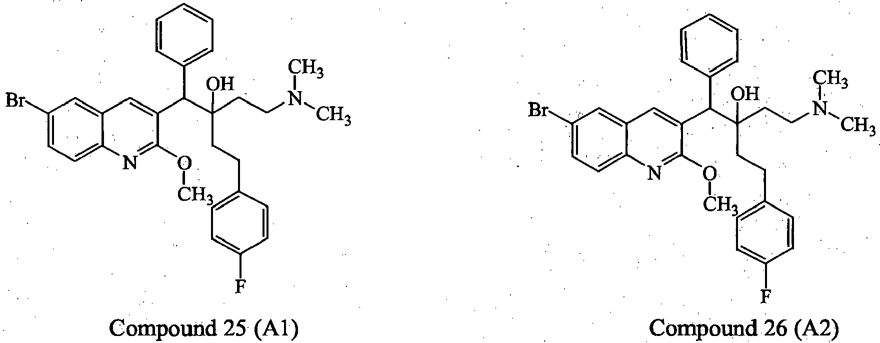

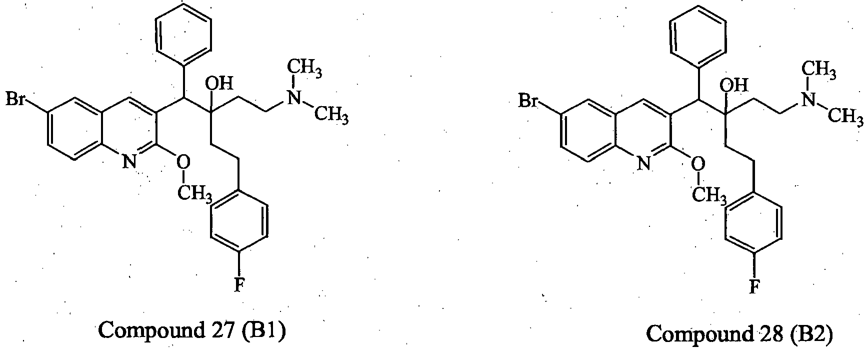

To obtain the corresponding enantiomers, 0.416 g of final compound 49 (diastereoisomer A) was purified by SFC chiral chromatography (ChiralPakADH 250x21mm, eluent: CO₂/EtOH/2-propanol 85/15/0.3). Two fractions were collected and the solvent was evaporated, to yield 0.13 g of final compound 25 (enantiomer A1) as a white solid and 0.13 g of final compound 26 (enantiomer A2).

To obtain the corresponding enantiomers, 0.655 g of final compound 50 (diastereoisomer B) was purified by SFC chiral chromatography (ChiralPakADH 250x21mm, eluent: CO₂/EtOH/2-propanol:85/15/0.3). Two fractions were collected and the solvent was evaporated, to yield 0.105 g of final compound 27 (enantiomer B1) as a white solid and 0.1 g of final compound 28 (enantiomer B2).

### Example B4

### a. Preparation of compound 33

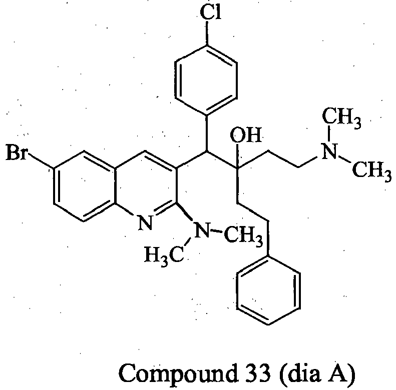

Final compound 33 was prepared in the same way as compound 21 starting from intermediate 23 and 1-(dimethylamino)-5-phenyl-3-pentanone (prepared in the same way as described in J.Am.Chem.Soc., 1950, 72, 718-721). Yield: 5% of final compound 33 (diastereoisomer A).

### Example B5

### a. Preparation of compound 92

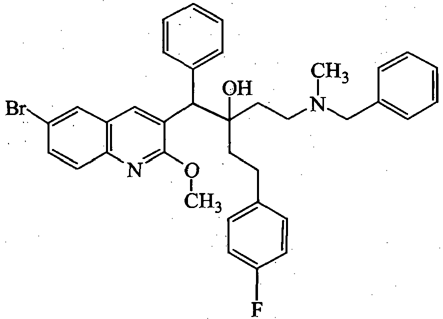

nBuLi 1.6M in hexane (2.5 ml, 0.004 mol) was added slowly at -20°C under N₂ flow to a solution of diisopropylamine (0.562 ml, 0.004 mol) in THF (9 ml). The mixture was stirred at -20°C for 20 minutes, then cooled at -70°C. A solution of intermediate 11 (1.1 g, 0.00334 mol) in THF (11 ml) was added slowly. The mixture was stirred at -70°C for 1 hour. A solution of intermediate 36 (1.0 g, 0.00334 mol) in THF (10 ml) was added slowly. The mixture was stirred at -70°C for 1 hour, hydrolyzed at -30°C with ice water, and extracted with EtOAc. The organic layer was separated, dried over MgSO₄, filtered, and the solvent was evaporated. The residue was purified by column chromatography over silica gel (eluent: cyclohexane/EtOAc; 83/17; 15-40 µm). The pure fraction was collected and the solvent was evaporated. Yield: 0.75 g of intermediate 36 (mixture of diastereoisomers) (36 %).

### b. Preparation of compounds 37 and

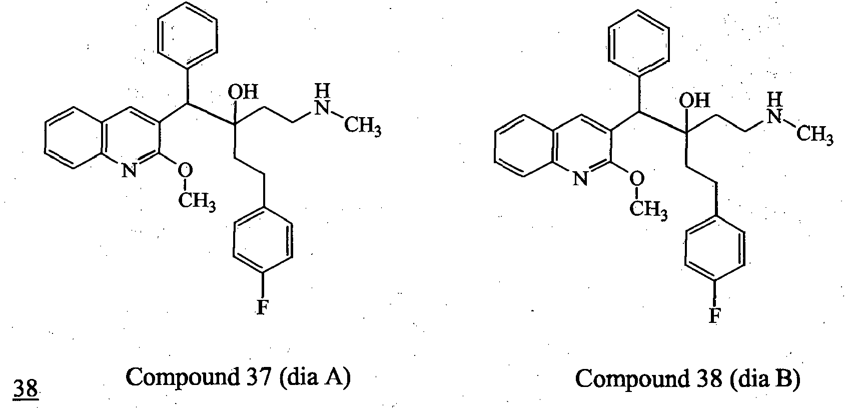

A mixture of final compound 92 (0.45 g, 0.72 mmol) in CH₂Cl₂ (2 ml), ammonium formate (0.23 g, 0.0036 mol), palladium on activated carbon 10 % (0.45 g) in methanol (9 ml) was stirred for 30 minutes at 80°C. Then the mixture was poured out into ice water and extracted with ethyl acetate. The organic layer was washed with brine, dried over MgSO₄, filtered, and the solvent was evaporated. The residue (0.45 g) was purified by column chromatography over silica gel (eluent: toluene/2-propanol/ NH₄OH; 90/10/0.5; 15-40 µm). Two fractions were collected and the solvent was evaporated. The fractions were separately crystallized from DIPE. Yield: 0.102 g of final compound 37 (30 %) (diastereoisomer A, melting point: 134°C) as a white solid and 0.064 g of final compound 38 (20 %) (diastereoisomer B, melting point: 138°C) as a white solid.

### Example B6

### Preparation of compound 58

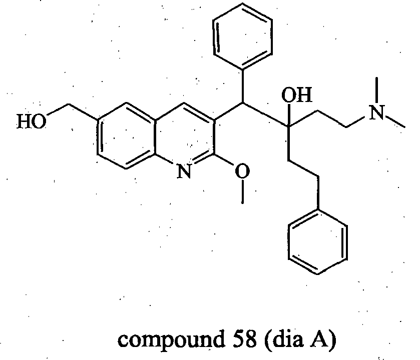

NaBH₄ (0.0007 mol) was added at 0°C to a solution of intermediate 39 (0.0007 mol) (prepared according to Example A15) in MeOH (6 ml) and THF (6ml). The mixture was stirred at 0°C for 2 hours, poured out into H₂O and extracted with EtOAc. The organic layer was washed with saturated NaCl, dried (MgSO₄), filtered and the solvent was evaporated. The residue (0.7 g) was purified by column chromatography over kromasil (eluent: CH₂Cl₂/CH₃OH/NH₄OH 98/2/0.2; 3.5 µm). The pure fractions were collected and the solvent was evaporated. This fraction was crystallized from DIPE/diethyl ether. The precipitate was filtered off and dried Yield: 0.05 g of compound 58 (dia A).

Tables 1 to 5 below list compounds which were prepared according to one of the above Examples (Ex. No.)

**Table 1:**

| | | | | | | | |
|---|---|---|---|---|---|---|---|
| 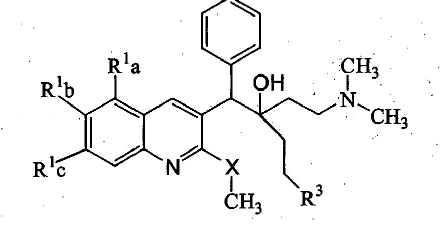 | | | | | | | |

| Comp .nr. | Ex. nr. | R¹ₐ | R¹_{b} | R¹_{c} | R³ | | melting points and stereochemistry |
|---|---|---|---|---|---|---|---|
| 12 | B2.a | H | H | H | phenyl | O | (A); 122°C |
| 13 | B2.a | H | H | H | phenyl | O | (B); 162°C |
| 1 | B1.a | H | CH₃ | H | phenyl | O | (A); 142°C |
| 2 | B1.a | H | CH₃ | H | phenyl | O | (B); 159°C |
| 3 | B1.b | H | OCH₃ | H | phenyl | O | (A) |
| 4 | B1.b | H | OCH₃ | H | phenyl | O | (B) |
| 14 | * | H | Br | H | phenyl | O | (A); 178°C |
| 15 | * | H | Br | H | phenyl | O | (B); 146°C |
| 17 | B1g | H | Br | H | phenyl | O | (A1) |
| 18 | B1g | H | Br | H | phenyl | O | (A2) |
| 19 | B1g | H | Br | H | phenyl | O | (B1) |
| 20 | B1g | H | Br | H | phenyl | O | (B2) |
| 10 | B1.f | H | Br | H | phenyl | S | (A); 191°C |
| 11 | B1.f | H | Br | H | phenyl | S | (B); 179°C |
| 7 | B1.d | H | Br | H | 1-naphthyl | O | (A); 206°C |
| 8 | B1.d | H | Br | H | 1-naphthyl | O | (B); 160°C |
| 16 | B1.f | H | Br | CH₃ | phenyl | O | (A); 168°C |
| 9 | B1.e | H | Cl | H | phenyl | O | (A); 172°C |
| 5 | B1.c | Br | H | H | phenyl | O | (A); 156°C |
| 6 | B1.c | Br | H | H | phenyl | O | (B); 126°C |
| 24 | B1.i | H | Br | H | 4-isopropylphenyl | O | (B) |
| 23 | B1.i | H | Br | H | 4-isopropylphenyl | O | (A) |
| 45 | B1.i | H | Br | H | 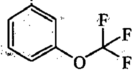 | O | (B) |
| 46 | B1.i | H | Br | H | 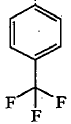 | O | (B); 154°C |
| 47 | B1.i | H | Br | H | 3-fluorophenyl | O | (A); 165°C |
| 48 | B1.i | H | Br | H | 3-fluorophenyl | O | (B); 167°C |
| 49 | B1.i | H | Br | H | 4-fluorophenyl | O | (A); 148°C |
| 50 | B1.i | H | Br | H | 4-fluorophenyl | O | (B); 156°C |
| 27 | B3.a | H | Br | H | 4-fluorophenyl | O | (B1) |
| 28 | B3.a | H | Br | H | 4-fluorophenyl | O | (B2) |
| 25 | B3.a | H | Br | H | 4-fluorophenyl | O | (A1) |
| 26 | B3.a | H | Br | H | 4-fluorophenyl | O | (A2) |
| 51 | B1.i | H | Br | H | 3,4-difluorophenyl | O | (B); 140°C |
| 52 | B1.i | H | Br | H | 4-methylphenyl | O | (B); 154°C |
| 53 | B1.i | H | Br | H | 4-methylphenyl | O | (A); 138°C |
| 54 | B1.i | H | Br | H | 2-chlorophenyl | O | (B); 146°C |
| 55 | B1.i | H | Br | H | 4-chlorophenyl | O | (B); 148°C |
| 56 | B1.i | H | Br | H | 4-chlorophenyl | O | (A); 167°C |
| 57 | B1.i | H | Br | H | 3,4-dichlorophenyl | O | (B); 164°C |
| 58 | B6 | H | HOCH₂-- | H | phenyl | O | (A) |
| 29 | B1.j | H | CH₃ | H | phenyl | S | (A); 180°C |
| 30 | B1.j | H | CH₃ | H | phenyl | S | (B); 120°C |
| 59 | B2.b | H | phenyl | H | phenyl | O | (B) |
| 60 | B2.b | H | phenyl | H | phenyl | O | (A) |
| 61 | B2.b | H | 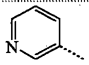 | H | phenyl | O | (A) |
| 36 | B2.b | H | 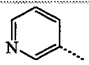 | H | phenyl | O | (B) |
| 62 | B2.b | H | 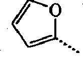 | H | phenyl | O | (B); 128°C |
| 63 | B2.b | H | 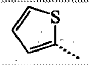 | H | phenyl | O | (A) |
| 64 | B2.b | H | 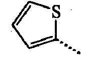 | H | phenyl | O | (B) |

| | | | | | | | |
|---|---|---|---|---|---|---|---|
| * prepared as described in WO 2004/01146 | | | | | | | |

**Table 2:**

| | | | | |
|---|---|---|---|---|
| 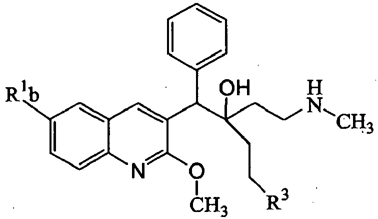 | | | | |

| Comp .nr. | Ex. nr. | R¹_{b} | R³ | melting points and stereochemistry |
|---|---|---|---|---|
| 37 | B5.a | H | 4-fluorophenyl | (A); 134°C |
| 38 | B5.a | H | 4-fluorophenyl | (B); 138°C |
| 39 | B1.m | Br | 4-fluorophenyl | (A); 250°C |
| 40 | B1.m | Br | 4-fluorophenyl | (B); 140°C |

**Table 3:**

| | | | | | | |
|---|---|---|---|---|---|---|
| 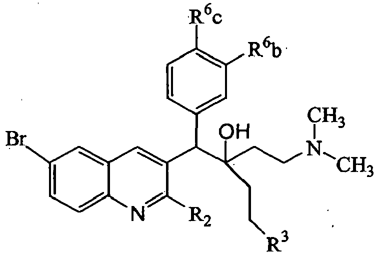 | | | | | | |

| Comp. nr. | Ex. nr. | R² | R³ | R⁶_{b} | R⁶_{c} | melting points and stereochemistry |
|---|---|---|---|---|---|---|
| 65 | B1.h | OCH₃ | phenyl | H | CH₃ | (A) |
| 66 | B1.h | OCH₃ | phenyl | H | CH₃ | (B); 149°C |
| 67 | B1.h | OCH₃ | phenyl | Cl | H | (A); 166°C |
| 68 | B1.h | OCH₃ | phenyl | Cl | H | (B); 154°C |
| 69 | B1.h | OCH₃ | phenyl | H | Cl | (A) |
| 70 | B1.h | OCH₃ | phenyl | H | Cl | (B); 162°C |
| 71 | B1.h | OCH₃ | phenyl | CH₃ | H | (A); 158°C |
| 72 | B1.h | OCH₃ | phenyl | CH₃ | H | (B) |
| 73 | B1-h | OCH3 | phenyl | H | OCH3 | (A) |
| 74 | B1.h | OCH₃ | phenyl | H | OCH₃ | (B); 151°C |
| 75 | B1.h | OCH₃ | 4-chlorophenyl | H | Cl | (A); 190°C |
| 76 | B1.h | OCH₃ | 4-chlorophenyl | H | Cl | (B); 174°C |
| 77 | B1.h | OCH₃ | 3-fluorophenyl | H | Cl | (A); 174°C |
| 78 | B1.h | OCH₃ | 3-fluorophenyl | H | Cl | (B); 172°C |
| 79 | B1.h | OCH₃ | 4-fluorophenyl | H | Cl | (A) |
| 80 | B1.h | OCH₃ | 4-fluorophenyl | Cl | H | (A); 169°C |
| 81 | B1.h | OCH₃ | 4-fluorophenyl | Cl | H | (B); 158°C |
| 82 | B1.h | OCH₃ | 4-fluorophenyl | CH₃ | H | (A); 138°C |
| 83 | B1.h | OCH₃ | 4-fluorophenyl | CH₃ | H | (B); 144°C |
| 84 | B1.h | OCH₃ | 4-fluorophenyl | H | OCH₃ | (A); 156°C |
| 85 | B1.h | OCH₃ | 4-fluorophenyl | H | OCH₃ | (B); 172°C |
| 86 | B1.h | OCH3 | 4-methylphenyl | H | Cl | (A) |
| 87 | B1.h | OCH₃ | 4-methylphenyl | H | Cl | (B); 180°C |
| 88 | B1.h | OCH₃ | 2-methoxyphenyl | H | Cl | (B) |
| 22 | B1.h | OCH₃ | 4-methoxyphenyl | H | Cl | (B); 160°C |
| 21 | B1.h | OCH₃ | 4-methoxyphenyl | H | Cl | (A); 142°C |
| 89 | B1.h | OCH₃ | 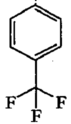 | H | Cl | (B); 140°C |
| 90 | B1.h | OCH₃ | 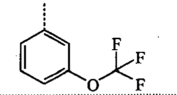 | H | Cl | (A); 161°C |
| 91 | B1.h | OCH₃ | 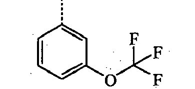 | H | Cl | (B) |
| 43 | B1.o | 2-(2-ethoxy-ethoxy)ethoxy | phenyl | H | H | (A); 94°C |
| 44 | B1.o | 2-(2-ethoxy-ethoxy)ethoxy | phenyl | H | H | (B); 103°C |
| 31 | B1.k | SCH₃ | phenyl | H | Cl | |
| 32 | B1.k | SCH₃ | phenyl | H | Cl | (B); 222°C |
| 33 | B4.a | N(CH₃)₂ | phenyl | H | Cl | (A) |

**Table 4:**

| | | | | |
|---|---|---|---|---|
| 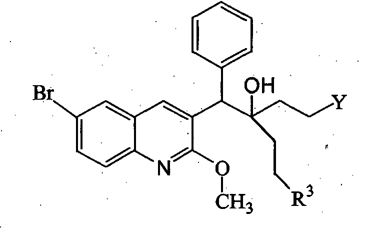 | | | | |

| Comp .nr. | Ex. nr. | R³ | Y | melting points and stereochemistry |
|---|---|---|---|---|
| 34 | B1.1 | 4-fluorophenyl | 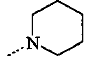 | (A); 140°C |
| 35 | B1.1 | 4-fluorophenyl | 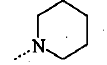 | (B); 179°C |
| 92 | B5.a | 4-fluorophenyl | -N(CH₃)(CH₂-C₆H₅) | |

**Table 5:**

| | | |
|---|---|---|
| 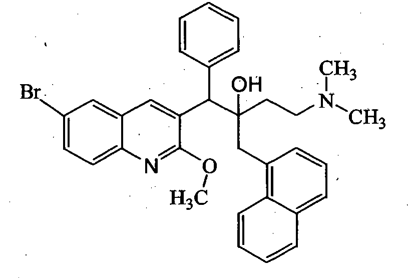 | | |

| Comp .nr. | Ex. nr. | melting points and stereochemistry |
|---|---|---|
| 41 | B1.n | (A); 112°C |
| 42 | B1.n | (B); 124°C |

### ANALYTICAL PART

### LCMS results

### General procedure

The HPLC gradient was supplied by an Alliance HT 2795 (Waters) system consisting of a quaternary pump with degasser, an autosampler, and DAD detector. Flow from the column was split to the MS detector. MS detectors were configured with an electrospray ionization source. The capillary needle voltage was 3 kV and the source temperature was maintained at 100 °C on the LCT (Time of Flight-Z-spray mass spectrometer from Waters) and 3.15 kV and 110 °C on the ZQ (simple quadripole- Z-spray mass spectrometer from Waters). Nitrogen was used as the nebulizer gas. Data acquisition was performed with a Waters-Micromass MassLynx-Openlynx data system.

### Method 1

In addition to the general procedure: Reversed phase HPLC was carried out on an Kromasil C18 column (5 µm, 4.6 x 150 mm) with a flow rate of 1.0 ml/min. Three mobile phases (mobile phase A: 100 % 7 mM ammonium acetate; mobile phase B: 100 % acetonitrile; mobile phase C: 0.2 % formic acid + 99.8 % ultra-pure Water) were employed to run a gradient condition from 30 % A , 40% B and 30% C (hold for 1 minute) to 100 % B in 4 minutes, 100% B for 5 minutes and reequilibrated with initial conditions for 3 minutes. An injection volume of 5 µl was used.

Cone voltage was 20 V for positive ionization mode. Mass spectra were acquired by scanning from 100 to 900 in 0.8 seconds using an interscan delay of 0.08 seconds.

### Method 2

In addition to the general procedure: Reversed phase HPLC was carried out on a Sunfire C18 column (3.5 µm, 4.6 x 100 mm) with an intial flow rate of 0.8 ml/min. Two mobile phases (mobile phase A: 25% 6.5mM ammonium acetate + 50% acetonitrile +25% formic acid (2ml/l); mobile phase B: 100% acetonitrile) were employed to run a gradient condition from 100 % A (hold for 1 minute) to 100% B in 4 minutes, hold at 100% B at a flow rate of 1.2 ml/min for 4 minutes and reequilibrated with initial conditions for 3 minutes). An injection volume of 10 µl was used. Cone voltage was 20 V for positive and negative ionization mode. Mass spectra were acquired by scanning from 100 to 1000 in 0.4 seconds using an interscan delay of 0.3 seconds.

### Method 3

In addition to the general procedure: Reversed phase HPLC was carried out on an Kromasil C18 column (5 µm, 4.6 x 150 mm) with a flow rate of 1.0 ml/min. Three mobile phases (mobile phase A: 100 % 7 mM ammonium acetate; mobile phase B: 100 % acetonitrile; mobile phase C: 0.2 % formic acid + 99.8 % ultra-pure Water) were employed to run a gradient condition from 30 % A, 40% B and 30% C (hold for 1 minute) to 100 % B in 4 minutes, 100% B for 5 minutes and reequilibrated with initial conditions for 3 minutes. An injection volume of 5 µl was used. Cone voltage was 20 V for positive and negative ionization mode. Mass spectra were acquired by scanning from 100 to 900 in 0.8 seconds using an interscan delay of 0.08 seconds.

### Method 4

In addition to the general procedure: Reversed phase HPLC was carried out on a Sunfire C18 column (3.5 µm, 4.6 x 100 mm) with an initial flow rate of 0.8 ml/min. Two mobile phases (mobile phase A: 35% 6.5mM ammonium acetate + 30% acetonitrile + 35% formic acid (2ml/l); mobile phase B: 100% acetonitrile) were employed to run a gradient condition from 100 % A (hold for 1 minute) to 100% B in 4 minutes, hold at 100% B at a flow rate of 1.2 ml/min for 4 minutes and reequilibrated with initial conditions for 3 minutes. An injection volume of 10 µl was used. Cone voltage was 20 V for positive and negative ionization mode. Mass spectra were acquired by scanning from 100 to 1000 in 0.4 seconds using an interscan delay of 0.3 seconds.

**Table 6 : LCMS results (retention time Rt (minutes) and molecular weight as the MH⁺**

| **Comp. No.** | **Rt** | **MH+** | **Method LCMS** |
|---|---|---|---|
| 3 | 5.34 | 485 | 1 |
| 4 | 5.43 | 485 | 1 |
| 17 | 6 | 533 | 3 |
| 18 | 6.04 | 533 | 3 |
| 19 | 6.07 | 533 | 3 |
| 20 | 6.06 | 533 | 3 |
| 24 | 5.5. | 575 | 4 |
| 23 | 5.43 | 575 | 4 |
| 45 | 5.23 | 617 | 4 |
| 27 | 4.98 | 551 | 4 |
| 28 | 4.98 | 551 | 4 |
| 25 | 4.97 | 551 | 4 |
| 26 | 4.97 | 551 | 4 |
| 58 | 4.57 | 485 | 1 |
| 59 | 6.5 | 531 | 1 |
| 60 | 6.56 | 531 | 1 |
| 61 | 5.13 | 532 | 1 |
| 36 | 4.37 | 532 | 1 |
| 63 | 6.38 | 537 | 1 |
| 64 | 6.37 | 537 | 1 |
| 65 | 3.55 | 547 | 2 |
| 69 | 4.05 | 567 | 2 |
| 72 | 5.15 | 547 | 4 |
| 73 | 5.93 | 563 | 1 |
| 79 | 5.23 | 585 | 4 |
| 86 | 5.33 | 581 | 4 |
| 88 | 5.23 | 597 | 4 |
| 91 | 5.6 | 651 | 4 |
| 31 | 5.42 | 583 | 4 |
| 33 | 5.13 | 580 | 4 |

### Optical rotation

The optical rotation was measured using a polarimeter. [α]D²⁰ indicates the optical rotation measured with light at the wavelength of the D-line of sodium (589 nm) at a temperature of 20°C. Table 7 lists the obtained optical rotation values, concentration and solvent used to measure the optical rotation.

**Table 7**

| **Comp. No.** | **[α]_{D}²⁰** | **concentration** | **solvent** |
|---|---|---|---|
| 17 | +141.82° | 0.483 w/v % | DMF |
| 18 | -140.28° | 0.494 w/v % | DMF |
| 19 | +154.08° | 0.392 w/v % | DMF |
| 20 | -139.21° | 0.4195 w/v % | DMF |
| 27 | +135.71° | 0.532 w/v% | DMF |
| 26 | -143.38° | 0.521 w/v % | DMF |
| 25 | +142.9° | 0.536 w/v % | DMF |
| 28 | -141.23° | 0.519 w/v % | DMF |

### Pharmacological examples

### Preparation of bacterial suspensions for susceptibility testing:

The bacteria used in this study were grown overnight in flasks containing 100 ml Mueller-Hinton Broth (Becton Dickinson - cat. no. 275730) in sterile de-ionized water, with shaking, at 37 °C. Stocks (0.5 ml/tube) were stored at -70 °C until use. Bacteria titrations were performed in microtiter plates and colony forming units (CFUs) were determined. In general, an inoculum level of approximately 100 CFUs was used for susceptibility testing.

### Anti bacterial Susceptibility testing: IC₉₀ determination

### Microtitre plate assay

Flat-bottom, sterile 96-well plastic microtiter plates were filled with 180 µl of sterile deionized water, supplemented with 0.25 % BSA. Subsequently, stock solutions (7.8 x final test concentration) of compounds were added in 45 µl volumes in column 2. Serial five-fold dilutions (45 µl in 180 µl) were made directly in the microtiter plates from column 2 to reach column 11. Untreated control samples with (column 1) and without (column 12) inoculum were included in each microtiter plate. Depending on the bacteria type, approximately 10 to 60 CFU per well of bacteria inoculum (100 TCID50), in a volume of 100 µl in 2.8x Mueller-Hinton broth medium, was added to the rows A to H, except column 12. The same volume of broth medium without inoculum was added to column 12 in row A to H. The cultures were incubated at 37°C for 24 hours under a normal atmosphere (incubator with open air valve and continuous ventilation). At the end of incubation, one day after inoculation, the bacterial growth was quantitated fluorometrically. Therefore resazurin (0.6 mg/ml) was added in a volume of 20 µl to all wells 3 hours after inoculation, and the plates were re-incubated overnight. A change in colour from blue to pink indicated the growth of bacteria.

The fluorescence was read in a computer-controlled fluorometer (Cytofluor Biosearch) at an excitation wavelength of 530 nm and an emission wavelength of 590 nm. The % growth inhibition achieved by the compounds was calculated according to standard methods. The IC₉₀ (expressed in µg/ml) was defined as the 90 % inhibitory concentration for bacterial growth. The results are shown in Table 8 below.

### Agar dilution method.

MIC₉₉ values (the minimal concentration for obtaining 99 % inhibition of bacterial growth) can be determined by performing the standard Agar dilution method according to NCCLS standards* wherein the media used includes Mueller-Hinton agar.
* Clinical laboratory standard institute. 2005. Methods for dilution Antimicrobial susceptibility tests for bacteria that grows Aerobically: approved standard -sixth edition

### Time kill assays

Bactericidal or bacteriostatic activity of the compounds may be determined in a time kill assay using the broth microdilution method*. In a time kill assay on *Staphylococcus aureus* and methicillin resistant *S. aureus* (MRSA), the starting inoculum of *S. aurues* and MRSA is 10⁶ CFU / ml in Muller Hinton broth. The antibacterial compounds are used at the concentration of 0.1 to 10 times the MIC (i.e. IC₉₀ as determined in microtitre plate assay). Wells receiving no antibacterial agent constitute the culture growth control. The plates containing the microorganism and the test compounds are incubated at 37 °C. After 0, 4, 24, and 48 hrs of incubation samples are removed for determination of viable counts by serial dilution (10-¹ to 10⁻⁶) in sterile PBS and plating (200 µl) on Mueller Hinton agar. The plates are incubated at 37 °C for 24 hours and the number of colonies are determined. Killing curves can be constructed by plotting the log₁₀CFU per ml versus time. A bactericidal effect is commonly defined as 3-log₁₀ decrease in number of CFU per ml as compared to untreated inoculum. The potential carryover effect of the drugs is removed by serial dilutions and counting the colonies at highest dilution used for plating. No carryover effect is observed at the dilution of 10⁻² used for plating. This results in limit of detection 5 X 10² CFU / ml or <2.7 log CFU/ml.
* Zurenko,G.E. et al. In vitro activities of U-100592 and U-100766, novel oxazolidinone antibacterial agents. Antimicrob. Agents Chemother. 40, 839-845 (1996).

### Determination of cellular ATP levels

In order to analyse the change in the total cellular ATP concentration (using ATP bioluminescence Kit, Roche), assays are carried out by growing a culture of *S. aureus* (ATCC29213) stock in 100 ml Mueller Hinton flasks and incubate in a shaker-incubator for 24 hrs at 37 °C (300 rpm). Measure OD₄₀₅ nm and calculate the CFU/ml. Dilute the cultures to 1 x 10⁶ CFU/ml (final concentration for ATP measurement: 1 x 10⁵ CFU/100 µl per well) and add test compound at 0.1 to 10 times the MIC (i.e. IC₉₀ as determined in microtitre plate assay). Incubate these tubes for 0, 30 and 60 minutes at 300 rpm and 37 °C. Use 0.6 ml bacterial suspension from the snap-cap tubes and add to a new 2 ml eppendorf tubes. Add 0.6 ml cell lysis reagent (Roche kit), vortex at max speed and incubate for 5 minutes at room temperature. Cool on ice. Let the luminometer warm up to 30°C (Luminoskan Ascent Labsystems with injector). Fill one column (= 6 wells) with 100 µl of the same sample. Add 100 µl Luciferase reagent to each well by using the injector system. Measure the luminescence for 1 sec.

**Table 8 : IC₉₀ values (µg/ml) determined according to the Microtitre plate assay.**

| | IC₉₀ µg/ml | | | | | | | | | | |
|---|---|---|---|---|---|---|---|---|---|---|---|
| Comp. No. | BSU | EFA | EFA | LMO | PAE | SMU | SPN | SPY | STA | STA | STA |
| | 43639 | 14506 | 29212 | 49594 | 27853 | 33402 | 6305 | 8668 | 25923 | 29213 | RMETH |
| 17 | | | | | | | 4.8 | | | 8.5 | |
| 18 | | | 10.6 | | 10.6 | | 2.1 | 8.5 | | 8.5 | |
| 19 | | | 1.7 | | 1.7 | | 2.1 | 1.7 | | 8.5 | |
| 20 | | | 1.7 | | 1.7 | | 1.1 | 1.7 | | 8.5 | |
| 8 | | | 1.9 | 1.9 | 2.3 | | 11.6 | 1.9 | | 1.9 | 1.9 |
| 15 | 10.6 | | 4.8 | 2.1 | 4.8 | | 7.5 | 4.2 | 4.8 | 4.8 | 10.6 |
| 12 | | | | | | | 9.1 | | | 10.2 | |
| 1 | | | 37.2 | | 18.7 | | 1.9 | 7.4 | | 9.4 | |
| 2 | | | 37.2 | | 37.2 | | 1.9 | 37.2 | | 7.4 | |
| 3 | | | 9.7 | | 9.7 | | 3.4 | 9.7 | | 12.2 | |
| 4 | | | | | | | 9.7 | | | 10.9 | |
| 14 | 13.4 | | 9.5 | 10.6 | 9.5 | | 42.4 | 10.6 | 11.9 | 13.4 | 13.4 |
| 6 | | | | | | | 1.7 | | | 8.5 | |
| 16 | | | 10.9 | | 2.2 | | 2.2 | 2.2 | | 19.4 | |
| 10 | | | | | | | 9.8 | | | 13.8 | |
| 11 | | | 43.7 | | 43.7 | | 3.9 | 8.7 | | 11.0 | |
| 13 | | | | | | | 4.1 | | | 36.1 | |
| 5 | | | 53.4 | | 53.4 | | 4.8 | 42.4 | | 23.8 | |
| 7 | | | | | | | 46.4 | | | 52.0 | |
| 9 | | | | | | | 1.7 | | | 24.5 | |
| 39 | | | | | | | 0.3 | | | 1.7 | |
| 37 | | | | | | | 0.3 | | | 2.9 | |
| 38 | | | | | | | 0.3 | | | 1.5 | |
| 55 | | | | | | | 0.3 | | | 1.8 | |
| 40 | | | | | | | 0.3 | | | 1.5 | |
| 24 | | | | | | | 0.3 | | | 2.6 | |
| 23 | | | | | | | 0.3 | | | 16.2 | |
| 91 | | | | | | | 0.3 | | | 32.7 | |
| 22 | | | 9.5 | | 1.9 | | 0.4 | 1.9 | | 1.9 | |
| 45 | | | 2.2 | | 0.7 | | 0.4 | 2.0 | | 2.5 | |
| 76 | | | | | | | 0.4 | | | **2**.**1** | |
| 31 | | | 58.4 | | 9.3 | | 0.4 | 1.9 | | 58.4 | |
| 50 | 9.8 | | 1.7 | 8.7 | 1.7 | | 0.4 | 1.7 | | 1.7 | 8.7 |
| 69 | | | 1.8 | | 1.8 | | 0.5 | 4.0 | | 1.8 | 12.7 |
| 83 | | | 9.0 | | 4.0 | | 0.5 | 1.8 | | 1.8 | |
| 88 | | | 37.7 | | 2.1 | | 0.5 | 1.9 | | 9.5 | |
| 46 | | | 1.9 | | 1.9 | | 0.5 | 1.9 | | 1.9 | |
| 81 | | | 9.3 | | 1.9 | | 0.6 | 1.9 | | 1.9 | |
| 68 | | | 56.8 | | 11.3 | | 0.7 | 56.8 | | 2.3 | |
| 65 | | | 1.7 | | 1.7 | | 0.8 | 1.7 | | 3.9 | 10.9 |
| 66 | | | 9.7 | | 43.5 | | 0.8 | 1.7 | | 1.7 | 12.3 |
| 33 | | | 1.8 | | 1.8 | | 0.8 | 1.5 | | 1.8 | |
| 77 | | | 3.7 | | 1.9 | | 0.8 | 1.9 | | 1.9 | |
| 52 | | | 1.7 | | 1.7 | | 1.4 | 1.7 | | 6.9 | |
| 28 | | | 3.9 | | 3.9 | | 0.9 | 3.9 | | 1.7 | |
| 78 | | | 9.3 | | 1.9 | | 1.5 | 1.9 | | 7.4 | |
| 71 | | | 54.8 | | 54.8 | | 1.7 | 43.5 | | 2.2 | |
| 25 | | | | | | | 1.7 | | | 7.8 | |
| 73 | | | 8.9 | | 8.0 | | 1.8 | 4.0 | | 7.1 | |
| 57 | | | | | | | 0.5 | | | 6.0 | |
| 74 | | | 1.8 | | 1.8 | | 1.8 | 1.8 | | 1.8 | 12.6 |
| 56 | | | | | | | 5.7 | | | 9.0 | |
| 70 | | | 56.8 | | 45.1 | | 1.8 | 45.1 | | 1.8 | 12.7 |
| 80 | | | 8.3 | | 1.9 | | 1.9 | 3.3 | | 9.3 | |
| 61 | | | | | | | 7.5 | | | | |
| 36 | | | 42.2 | | 42.2 | | 1.7 | 16.8 | | 1.7 | |
| 53 | | | 6.9 | | 3.9 | | 1.7 | 1.7 | | 1.7 | 13.8 |
| 26 | | | 1.7 | | 1.7 | | 2.0 | 1.7 | | 4.4 | |
| 27 | | | | | | | 2.0 | | | 1.7 | |
| 84 | | | 58.2 | | 46.2 | | 2.1 | 46.2 | | 4.6 | |
| 85 | | | | | | | 2.1 | | | 4.1 | |
| 86 | | | | | | | 2.1 | | | 41.2 | |
| 32 | | | | | | | 2.1 | | | 9.3 | |
| 21 | | | 1.9 | | 1.9 | | 2.1 | 1.9 | | 1.9 | |
| 63 | | | 19.0 | | 8.5 | | 2.1 | 21.4 | | 10.7 | |
| 72 | | | 43.5 | | 43.5 | | 2.2 | 43.5 | | 1.7 | |
| 48 | | | 43.8 | | 43.8 | | 2.2 | 43.8 | 43.8 | 1.7 | |
| 82 | | | 1.8 | | 1.8 | | 2.3 | 1.8 | | 1.8 | |
| 43 | | | 10.1 | | 10.1 | | 2.3 | 10.1 | | 10.1 | |
| 67 | | | 56.8 | | 56.8 | | 2.3 | 56.8 | | 2.3 | |
| 89 | | | | | | | 2.3 | | | 22.6 | |
| 87 | | | 11.6 | | 9.2 | | 2.3 | 5.2 | | 9.2 | |
| 79 | | | 58.6 | | 46.5 | | 2.3 | 46.5 | | 9.3 | |
| 90 | | | | | | | 2.6 | | | 16.4 | |
| 75 | | | | | | | 3.4 | | | 3.0 | |
| 35 | | | 59.2 | | 59.2 | | 3.7 | 9.4 | | 59.2 | |
| 44 | | | 10.1. | | 4.5 | | 4.5 | 8.0 | | 22.6 | |
| 58 | | | | | | | 8.6 | | | 8.6 | |
| 47 | | | | | | | 8.7 | | | 43.8 | |
| 34 | | | | | | | 9.4 | | | 59.2 | |
| 62 | | | | | | | 10.4 | | | 8.3 | |
| 60 | | | | | | | 10.6 | | | 53.1 | |
| 59 | | | | | | | 10.6 | | | 42.2 | |
| 64 | | | | | | | 10.7 | | | 42.6 | |
| 29 | | | | | | | 7.7 | | | 48.5 | |
| 30 | | | | | | | 1.9 | | | 7.7 | |
| 54 | | | 4.5 | | 2.0 | | 2.3 | 2.0 | | 10.1 | |
| 41 | | | | | | | 2.3 | | | 57.0 | |
| 42 | | | 22.7 | | 10.1 | | 2.3 | 4.5 | | 9.0 | |
| 51 | | | 10.1 | | 9.0 | | 0.4 | 5.7 | | 1.8 | 11.4 |
| 49 | | | 43.8 | | 43.8 | | 2.2 | 43.8 | | 11.0 | |

| | | | | | | | | | | | |
|---|---|---|---|---|---|---|---|---|---|---|---|
| BSU 43639 means *Bacillus subtilis* (ATCC43639); EFA 14506 means *Enterococcus faecalis* (ATCC14506); EFA 29212 means *Enterococcus faecalis* (ATCC29212); LMO 49594 means *Listeria monocytogenes* (ATCC49594); PAE 27853 means *Pseudomonas aeruginosa* (ATCC27853); SMU 33402 means *Streptococcus mutans* (ATCC33402); SPN 6305 means *Streptococcus pneumoniae* (ATCC6305); SPY 8668 means *Streptococcus pyogens* (ATCC8668); STA 25923 means *Staphylococcus aureus* (ATCC25923); STA 29213 means *Staphylococcus aureus* (ATCC29213); STA RMETH means methicilline resistant *Staphylococcus aureus* (MRSA) (a clinical isolate from the University of Antwerp). ATCC means American type tissue culture. | | | | | | | | | | | |

## Claims

1. Use of a compound for the manufacture of a medicament for the treatment of a bacterial infection caused by *Staphylococci, Enterococci* or *Streptococci,* said compound being a compound of formula (Ia) or (Ib) 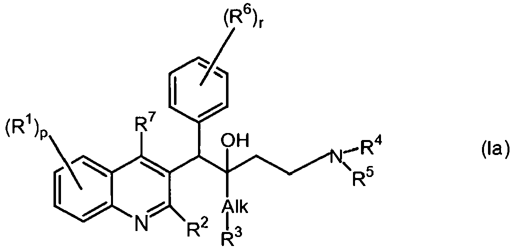 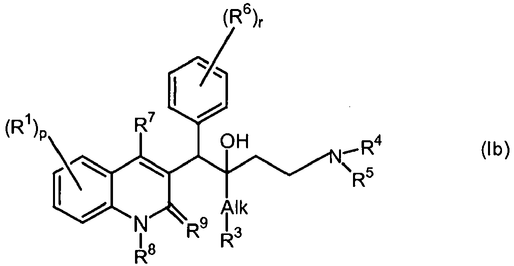 a pharmaceutically acceptable acid or base addition salt thereof, a stereochemically isomeric form thereof, a tautomeric form thereof or a *N*-oxide form thereof, wherein
R¹ is hydrogen, halo, haloalkyl, cyano, hydroxy, Ar, Het, alkyl, alkyloxy, alkylthio, alkyloxyalkyl, alkylthioalkyl, Ar-alkyl or di(Ar)alkyl ;
p is an integer equal to 1, 2, 3 or 4 ;
R² is hydrogen, hydroxy, mercapto, alkyloxy, alkyloxyalkyloxy, alkylthio, mono or di(alkyl)amino or a radical of formula 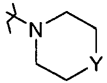 wherein Y is CH₂, O, S, NH or *N*-alkyl;
R³ is Ar or Het;
R⁴ and R⁵ each independently are hydrogen, alkyl or benzyl; or
R⁴ and R⁵ together and including the N to which they are attached may form a radical selected from the group of pyrrolidinyl, 2-pyrrolinyl, 3-pyrrolinyl, pyrrolyl, imidazolidinyl, pyrazolidinyl, 2-imidazolinyl, 2-pyrazolinyl, imidazolyl, pyrazolyl, triazolyl, piperidinyl, pyridinyl, piperazinyl, pyridazinyl, pyrimidinyl, pyrazinyl, triazinyl, morpholinyl and thiomorpholinyl, optionally substituted with alkyl, halo, haloalkyl, hydroxy, alkyloxy, amino, mono- or dialkylamino, alkylthio, alkyloxyalkyl, alkylthioalkyl or pyrimidinyl ;
R⁶ is hydrogen, halo, haloalkyl, hydroxy, Ar, alkyl, alkyloxy, alkylthio, alkyloxyalkyl, alkylthioalkyl, Ar-alkyl or di(Ar)alkyl ; or
two vicinal R⁶ radicals may be taken together to form a bivalent radical of formula -CH=CH-CH=CH- ;
r is an integer equal to 1, 2, 3, 4 or 5 ;
R⁷ is hydrogen, alkyl, Ar or Het ;
R⁸ is hydrogen or alkyl;
R⁹ is oxo ; or
R⁸ and R⁹ together form the radical -CH=CH-N=;
alkyl is a straight or branched saturated hydrocarbon radical having from 1 to 6 carbon atoms ; or is a cyclic saturated hydrocarbon radical having from 3 to 6 carbon atoms ; or is a cyclic saturated hydrocarbon radical having from 3 to 6 carbon atoms attached to a straight or branched saturated hydrocarbon radical having from 1 to 6 carbon atoms; wherein each carbon atom can be optionally substituted with hydroxy, alkyloxy or oxo;
Alk is a straight or branched saturated hydrocarbon radical having from 1 to 6 carbon atoms ;
Ar is a homocycle selected from the group of phenyl, naphthyl, acenaphthyl and tetrahydronaphthyl, each homocycle optionally substituted with 1, 2 or 3 substituents, each substituent independently selected from the group of hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl and mono- or dialkylaminocarbonyl ;
Het is a monocyclic heterocycle selected from the group of *N*-phenoxypiperidinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl and pyridazinyl; or a bicyclic heterocycle selected from the group of quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl and benzo[1,3]dioxolyl ; each monocyclic and bicyclic heterocycle may optionally be substituted with 1, 2 or 3 substituents, each substituent independently selected from the group of halo, hydroxy, alkyl, alkyloxy, and Ar-carbonyl;
halo is a substituent selected from the group of fluoro, chloro, bromo and iodo; and
haloalkyl is a straight or branched saturated hydrocarbon radical having from 1 to 6 carbon atoms or a cyclic saturated hydrocarbon radical having from 3 to 6 carbon atoms or a cyclic saturated hydrocarbon radical having from 3 to 6 carbon atoms attached to a straight or branched saturated hydrocarbon radical having from 1 to 6 carbon atoms; wherein one or more carbon atoms are substituted with one or more halo atoms.

2. Use according to claim 1 wherein R¹ is hydrogen, halo, alkyl, alkyloxy, Ar or Het.

3. Use according to claim 2 wherein R¹ is hydrogen, halo, alkyl or alkyloxy.

4. Use according to claim 3 wherein R¹ is hydrogen or halo.

5. Use according to claim 4 wherein R¹ is halo.

6. Use according to any one of the preceding claims wherein p is equal to 1.

7. Use according to claim 6 wherein the R¹ substituent is placed in position 6 of the quinoline ring.

8. Use according to any one of the preceding claims wherein R² is alkyloxy, alkylthio, mono-or di(alkyl)amino or alkyloxyalkyloxy.

9. Use according to any one of claims 1 to 7 wherein R² is hydrogen, alkyloxy or alkylthio.

10. Use according to claim 8 or 9 wherein R² is C₁₋₄alkyloxy.

11. Use according to any one of the preceding claims wherein R³ is Ar.

12. Use according to claim 11 wherein R³ is naphthyl or phenyl, optionally substituted with 1 or 2 halo.

13. Use according to any one of the preceding claims wherein R⁴ and R⁵ each independently are hydrogen or C₁₋₄alkyl.

14. Use according to any one of the preceding claims wherein R⁶ is hydrogen.

15. Use according to any one of the preceding claims wherein R⁷ is hydrogen.

16. Use according to any one of the preceding claims wherein Alk is methylene or ethylene.

17. Use according to claim 16 wherein Alk is ethylene.

18. Use according to any one of the preceding claims wherein the compound is a compound according to formula (Ia).

19. Use according to claim 1 wherein the compound is a compound of formula (Ia)
wherein R¹ is hydrogen, halo, C₁₋₄alkyl, C₁₋₄alkyloxy, Ar or Het; p = 1 or 2; R² is C₁₋₄alkyloxy, C₁₋₄alkylthio, mono-or di(C₁₋₄alkyl)amino, C₁₋₄alkyloxyC₁₋₄alkyloxy; R³ is optionally substituted naphthyl or phenyl; R⁴ and R⁵ each independently are hydrogen or C₁₋₄alkyl; or R⁴ and R⁵ together and including the N to which they are attached form a piperidinyl; R⁶ is hydrogen, halo, C₁₋₄alkyl or C₁₋₄alkyloxy; r is equal to 1; R⁷ is hydrogen; Alk is methylene or ethylene.

20. Use according to any one of the preceding claims wherein the bacterial infection is an infection with a gram-positive bacterium.

21. Use according to any one of the preceding claims wherein the compound is selected from 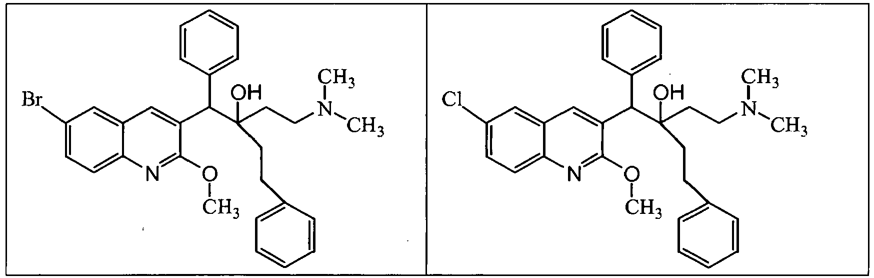 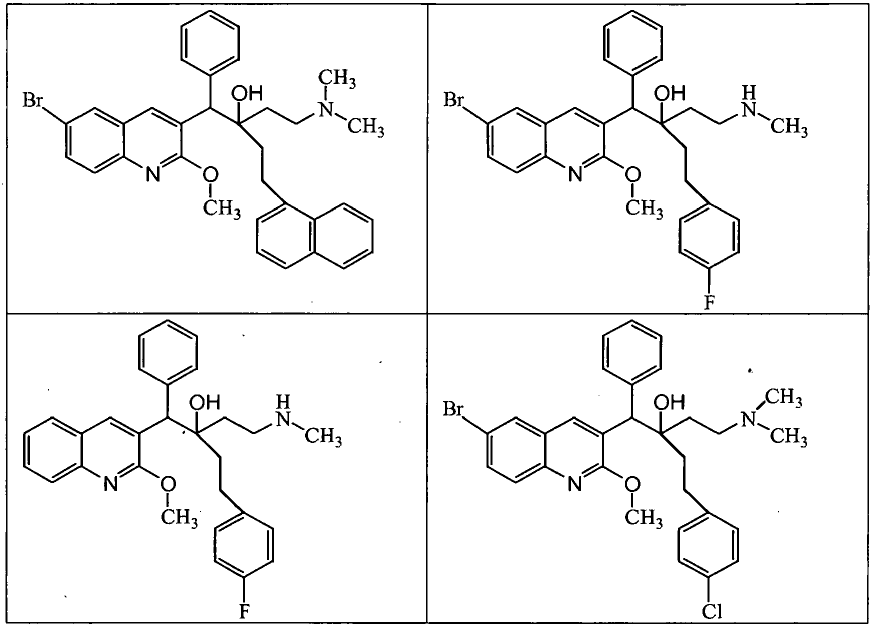 a pharmaceutically acceptable acid or base addition salt thereof, a stereochemically isomeric form thereof, a tautomeric form thereof or a *N*-oxide form thereof.

22. A compound selected from 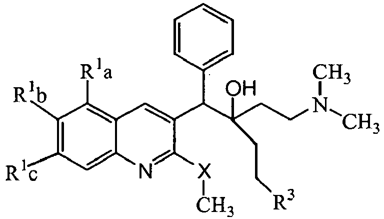
| R¹ₐ | R¹_{b} | R¹_{c} | R³ | X |
|---|---|---|---|---|
| H | H | H | phenyl | O |
| H | CH₃ | H | phenyl | O |
| H | OCH₃ | H | phenyl | O |
| H | Br | H | phenyl | S |
| H | Br | H | 1-naphthyl | O |
| H | Br | CH₃ | phenyl | O |
| H | Cl | H | phenyl | O |
| Br | H | H | phenyl | O |
a pharmaceutically acceptable acid or base addition salt thereof, a stereochemically isomeric form thereof, a tautomeric form thereof or a *N*-oxide form thereof.

23. A compound selected from 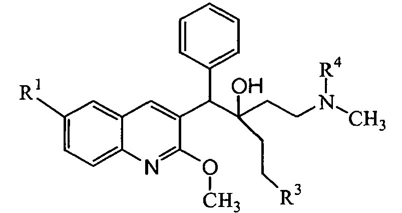
| R¹ | R³ | R⁴ |
|---|---|---|
| Br | 4-fluorophenyl | H |
| H | 4-fluorophenyl | H |
| Br | 4-chlorophenyl | CH₃ |
a pharmaceutically acceptable acid or base addition salt thereof, a stereochemically isomeric form thereof, a tautomeric form thereof or a *N*-oxide form thereof.

24. A compound selected from 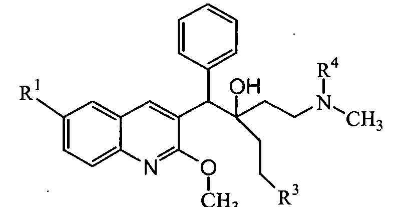
| R¹ | R³ | R⁴ | stereochemistry |
|---|---|---|---|
| Br | 4-fluorophenyl | H | (A) |
| Br | 4-fluorophenyl | H | (B) |
| H | 4-fluorophenyl | H | (A) |
| H | 4-fluorophenyl | H | (B) |
| Br | 4-chlorophenyl | CH₃ | (B) |
a pharmaceutically acceptable acid or base addition salt thereof, a stereochemically isomeric form thereof, a tautomeric form thereof or a *N*-oxide form thereof, wherein the stereochemically isomeric form which was first isolated is designated as "A" and the second as "B".

25. A combination of (a) a compound of formula (Ia) or (Ib) as defined in any one of the preceding claims, and (b) one or more other antibacterial agents provided that the one or more other antibacterial agents are other than antimycobacterial agents, wherein the combination is for use in the treatment of a bacterial infection caused by *Staphylococci, Enterococci* or *Streptococci.*

26. A pharmaceutical composition comprising a pharmaceutically acceptable carrier and, as active ingredient, a therapeutically effective amount of (a) a compound of formula (Ia) or (Ib) as defined in any one of claims 1 to 24, and (b) one or more other antibacterial agents provided that the one or more other antibacterial agents are other than antimycobacterial agents, wherein the composition is for use in the treatment of a bacterial infection caused by *Staphylococci, Enterococci* or *Streptococci.*

27. A compound as defined in any one of claims 1 to 24, for use in the treatment of a bacterial infection caused by *Staphylococci, Enterococci* or *Streptococci.*

28. A product containing (a) a compound of formula (Ia) or (Ib) as defined in any one of claims 1 to 21, and (b) one or more other antibacterial agents provided that the one or more other antibacterial agents are other than antimycobacterial agents, as a combined preparation for simultaneous, separate or sequential use in the treatment of a bacterial infection caused by *Staphylococci, Enterococci* or *Streptococci.*

29. Use as claimed in any of claims 1 to 24, combination as claimed in claim 25, composition as claimed in claim 26, compound as claimed in claim 27 or product as claimed in claim 28, wherein the bacterial infection is an infection with methicillin resistant *Staphylococcus aureus* (MRSA), methicillin resistant coagulase negative staphylococci (MRCNS), penicillin resistant *Streptococcus pneumoniae* or multiple resistant *Enterococcus faecium.*

30. Use, combination, composition, compound or product as claimed in claim 29, wherein the bacterial infection is an infection with *Staphylococcus aureus* or *Streptococcus pneumoniae.*

31. Use, combination, composition, compound or product as claimed in claim 30, wherein the bacterial infection is an infection with *Staphylococcus aureus* (MRSA).

## Patentansprüche

1. Verwendung einer Verbindung für die Herstellung eines Arzneimittels für die Behandlung einer durch *Staphylococci*, *Enterococci* oder *Streptococci* verursachten bakteriellen Infektion, wobei es sich bei dieser Verbindung um eine Verbindung der Formel (Ia) oder (Ib) 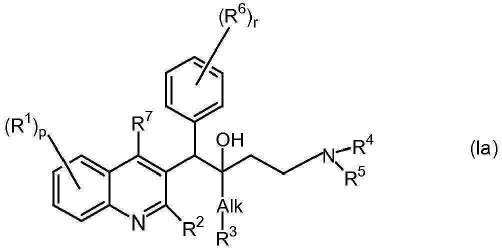 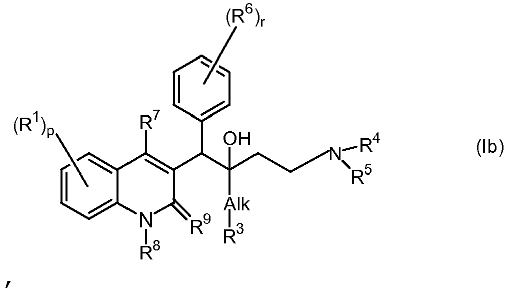 ein pharmazeutisch annehmbares Säure- oder Basenadditionssalz davon, eine stereochemisch isomere Form davon, eine tautomere Form davon oder eine N-Oxidform davon handelt, wobei
R¹ Wasserstoff, Halogen, Halogenalkyl, Cyano, Hydroxy, Ar, Het, Alkyl, Alkyloxy, Alkylthio, Alkyloxyalkyl, Alkylthioalkyl, Ar-Alkyl oder Di(Ar)alkyl bedeutet;
p eine ganze Zahl gleich 1, 2, 3 oder 4 ist;
R² Wasserstoff, Hydroxy, Mercapto, Alkyloxy, Alkyloxyalkyloxy, Alkylthio, Mono oder Di(alkyl)amino oder einen Rest der Formel 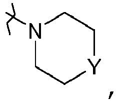 worin Y CH₂, 0, S, NH oder *N*-Alkyl ist, bedeutet;
R³ Ar oder Het bedeutet;
R⁴ und R⁵ jeweils unabhängig Wasserstoff, Alkyl oder Benzyl bedeuten; oder
R⁴ und R⁵ gemeinsam und einschließlich des N, an den sie gebunden sind, einen Rest, ausgewählt aus der Gruppe Pyrrolidinyl, 2-Pyrrolinyl, 3-Pyrrolinyl, Pyrrolyl, Imidazolidinyl, Pyrazolidinyl, 2-Imidazolinyl, 2-Pyrazolinyl, Imidazolyl, Pyrazolyl, Triazolyl, Piperidinyl, Pyridinyl, Piperazinyl, Pyridazinyl, Pyrimidinyl, Pyrazinyl, Triazinyl, Morpholinyl und Thiomorpholinyl, gegebenenfalls substituiert durch Alkyl, Halogen, Halogenalkyl, Hydroxy, Alkyloxy, Amino, Mono- oder Dialkylamino, Alkylthio, Alkyloxyalkyl, Alkylthioalkyl oder Pyrimidinyl bilden können;
R⁶ Wasserstoff, Halogen, Halogenalkyl, Hydroxy, Ar, Alkyl, Alkyloxy, Alkylthio, Alkyloxyalkyl, Alkylthioalkyl, Ar-Alkyl oder Di(Ar)alkyl bedeutet oder
zwei benachbarte R⁶-Reste gemeinsam einen zweiwertigen Rest der Formel
-CH=CH-CH=CH- bilden können;
r eine ganze Zahl gleich 1, 2, 3, 4 oder 5 ist;
R⁷ Wasserstoff, Alkyl, Ar oder Het bedeutet;
R⁸ Wasserstoff oder Alkyl bedeutet;
R⁹ Oxo bedeutet; oder
R⁸ und R⁹ gemeinsam den Rest -CH=CH-N= bilden;
Alkyl einen geradkettigen oder verzweigten gesättigten Kohlenwasserstoffrest mit 1 bis 6 Kohlenstoffatomen bedeutet; oder einen cyclischen gesättigten Kohlenwasserstoffrest mit 3 bis 6 Kohlenstoffatomen bedeutet; oder einen cyclischen gesättigten Kohlenwasserstoffrest mit 3 bis 6 Kohlenstoffatomen, der an einen geradkettigen oder verzweigten gesättigten Kohlenwasserstoffrest mit 1 bis 6 Kohlenstoffatomen gebunden ist, bedeutet; wobei jedes Kohlenstoffatom gegebenenfalls durch Hydroxy, Alkyloxy oder Oxo substituiert sein kann;
Alk einen geradkettigen oder verzweigten gesättigten Kohlenwasserstoffrest mit 1 bis 6 Kohlenstoffatomen bedeutet;
Ar einen Homocyclus, ausgewählt aus der Gruppe Phenyl, Naphthyl, Acenaphthyl und Tetrahydronaphthyl bedeutet, wobei jeder Homocyclus gegebenenfalls durch 1, 2 oder 3 Substituenten substituiert ist, wobei jeder Substituent unabhängig aus der Gruppe Hydroxy, Halogen, Cyano, Nitro, Amino, Mono- oder Dialkylamino, Alkyl, Halogenalkyl, Alkyloxy, Halogenalkyloxy, Carboxyl, Alkyloxycarbonyl, Aminocarbonyl, Morpholinyl und Mono- oder Dialkylaminocarbonyl ausgewählt ist;
Het einen monocyclischen Heterocyclus, ausgewählt aus der Gruppe N-Phenoxypiperidinyl, Piperidinyl, Pyrrolyl, Pyrazolyl, Imidazolyl, Furanyl, Thienyl, Oxazolyl, Isoxazolyl, Thiazolyl, Isothiazolyl, Pyridinyl, Pyrimidinyl, Pyrazinyl und Pyridazinyl bedeutet; oder einem bicyclischen Heterocyclus, ausgewählt aus der Gruppe Chinolinyl, Chinoxalinyl, Indolyl, Benzimidazolyl, Benzoxazolyl, Benzisoxazolyl, Benzothiazolyl, Benzisothiazolyl, Benzofuranyl, Benzothienyl, 2,3-Dihydrobenzo[1,4]dioxinyl und Benzo[1,3]dioxolyl bedeutet; wobei jeder monocyclische und bicyclische Heterocyclus gegebenenfalls durch 1, 2 oder 3 Substituenten substituiert sein kann, wobei jeder Substituent unabhängig aus der Gruppe Halogen, Hydroxy, Alkyl, Alkyloxy and Ar-Carbonyl ausgewählt ist;
Halogen einen Substituenten, ausgewählt aus der Gruppe Fluor, Chlor, Brom und Iod bedeutet; und
Halogenalkyl einen geradkettigen oder verzweigten gesättigten Kohlenwasserstoffrest mit 1 bis 6 Kohlenstoffatomen bedeutet; oder einen cyclischen gesättigten Kohlenwasserstoffrest mit 3 bis 6 Kohlenstoffatomen bedeutet; oder einen cyclischen gesättigten Kohlenwasserstoffrest mit 3 bis 6 Kohlenstoffatomen, der an einen geradkettigen oder verzweigten gesättigten Kohlenwasserstoffrest mit 1 bis 6 Kohlenstoffatomen gebunden ist, bedeutet; wobei ein oder mehrere Kohlenstoffatome durch ein oder mehrere Halogenatome substituiert sind.

2. Verwendung nach Anspruch 1, wobei R¹ Wasserstoff, Halogen, Alkyl, Alkyloxy, Ar oder Het bedeutet.

3. Verwendung nach Anspruch 2, wobei R¹ Wasserstoff, Halogen, Alkyl oder Alkyloxy bedeutet.

4. Verwendung nach Anspruch 3, wobei R¹ Wasserstoff oder Halogen bedeutet.

5. Verwendung nach Anspruch 4, wobei R¹ Halogen bedeutet.

6. Verwendung nach einem der vorhergehenden Ansprüche, wobei p gleich 1 ist.

7. Verwendung nach Anspruch 6, wobei der R¹-Substituent in Position 6 des Chinolinrings steht.

8. Verwendung nach einem der vorhergehenden Ansprüche, wobei R² Alkyloxy, Alkylthio, Mono- oder Di(alkyl)amino oder Alkyloxyalkyloxy bedeutet.

9. Verwendung nach einem der Ansprüche 1 bis 7, wobei R² Wasserstoff, Alkyloxy oder Alkylthio bedeutet.

10. Verwendung nach Anspruch 8 oder 9, wobei R² C₁₋₄Alkyloxy bedeutet.

11. Verwendung nach einem der vorhergehenden Ansprüche, wobei R³ Ar bedeutet.

12. Verwendung nach Anspruch 11, wobei R³ Naphthyl oder Phenyl, gegebenenfalls substituiert durch ein oder zwei Halogen, bedeutet.

13. Verwendung nach einem der vorhergehenden Ansprüche, wobei R⁴ und R⁵ jeweils unabhängig voneinander Wasserstoff oder C₁₋₄Alkyl bedeuten.

14. Verwendung nach einem der vorhergehenden Ansprüche, wobei R⁶ Wasserstoff bedeutet.

15. Verwendung nach einem der vorhergehenden Ansprüche, wobei R⁷ Wasserstoff bedeutet.

16. Verwendung nach einem der vorhergehenden Ansprüche, wobei Alk Methylen oder Ethylen bedeutet.

17. Verwendung nach Anspruch 16, wobei Alk Ethylen bedeutet.

18. Verwendung nach einem der vorhergehenden Ansprüche, wobei die Verbindung eine Verbindung gemäß Formel (Ia) ist.

19. Verwendung nach Anspruch 1, wobei die Verbindung eine Verbindung der Formel (Ia) ist, worin R¹ Wasserstoff, Halogen, C₁₋₄Alkyl, C₁₋₄Alkyloxy, Ar oder Het bedeutet; p = 1 oder 2; R² C₁₋₄Alkyloxy, C₁₋₄Alkylthio, Mono- oder Di(C₁₋₄alkyl)amino, C₁₋₄AlkyloxyC₁₋₄alkyloxy bedeutet; R³ gegebenenfalls substituiertes Naphthyl oder Phenyl bedeutet; R⁴ und R⁵ jeweils unabhängig Wasserstoff oder C₁₋₄Alkyl bedeuten; oder R⁴ und R⁵ gemeinsam und einschließlich des N, an das sie gebunden sind, ein Piperidinyl bilden; R⁶ Wasserstoff, Halogen, C₁₋₄Alkyl oder C₁₋₄Alkyloxy bedeuten; r gleich 1 ist; R⁷ Wasserstoff bedeutet; Alk Methylen oder Ethylen bedeutet.

20. Verwendung nach einem der vorhergehenden Ansprüche, wobei es sich bei der bakteriellen Infektion um eine Infektion mit einem grampositiven Bakterium handelt.

21. Verwendung nach einem der vorhergehenden Ansprüche, wobei die Verbindung aus 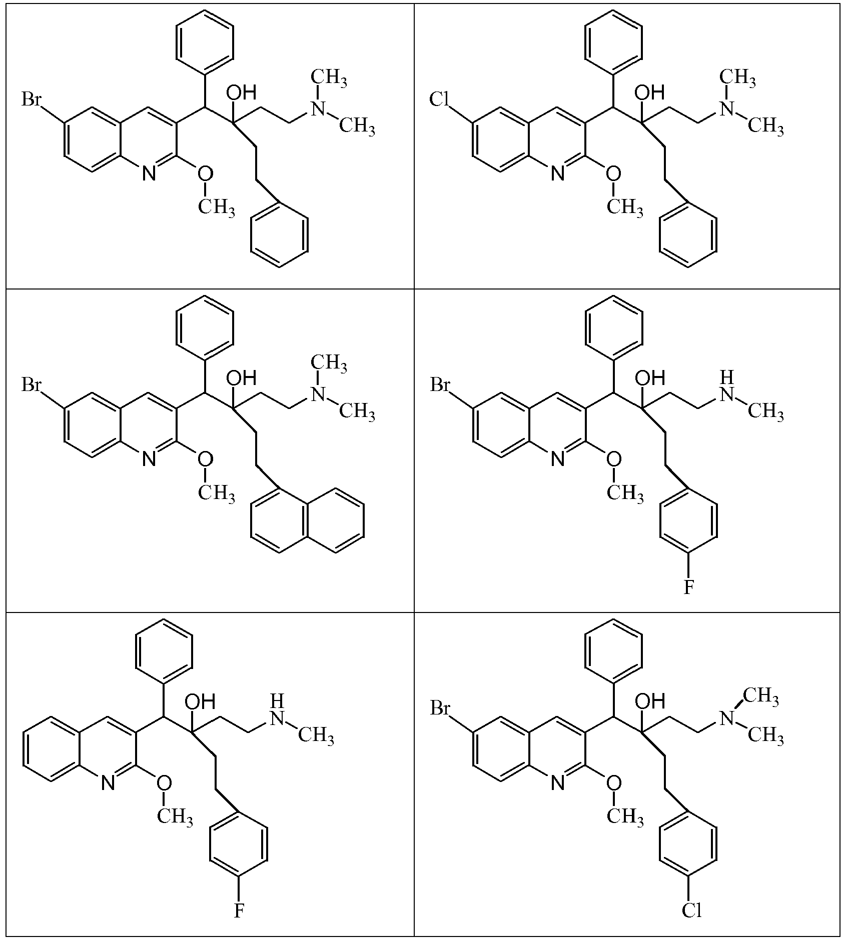 einem pharmazeutisch annehmbaren Säure- oder Basenadditionssalz davon, einer stereochemisch isomeren Form davon, einer tautomeren Form davon oder einer N-Oxidform davon ausgewählt ist.

22. Verbindung, die aus 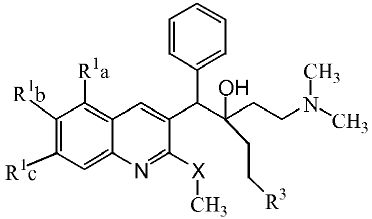
| R¹ₐ | R¹_{b} | R¹_{c} | R³ | X |
|---|---|---|---|---|
| H | H | H | Phenyl | O |
| H | CH₃ | H | Phenyl | O |
| H | OCH₃ | H | Phenyl | O |
| H | Br | H | Phenyl | S |
| H | Br | H | 1-Naphthyl | O |
| H | Br | CH₃ | Phenyl | O |
| H | Cl | H | Phenyl | O |
| Br | H | H | Phenyl | O |
einem pharmazeutisch annehmbaren Säure- oder Basenadditionssalz davon, einer stereochemisch isomeren Form davon, einer tautomeren Form davon oder einer N-Oxidform davon ausgewählt ist.

23. Verbindung, die aus 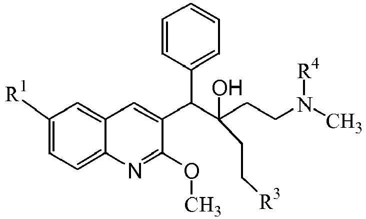
| R¹ | R³ | R⁴ |
|---|---|---|
| Br | 4-Fluorphenyl | H |
| H | 4-Fluorphenyl | H |
| Br | 4-Chlorphenyl | CH₃ |
einem pharmazeutisch annehmbaren Säure- oder Basenadditionssalz davon, einer stereochemisch isomeren Form davon, einer tautomeren Form davon oder einer N-Oxidform davon ausgewählt ist.

24. Verbindung, die aus 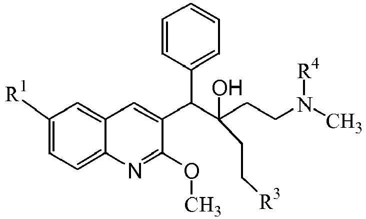
| R¹ | R³ | R⁴ | Stereochemie |
|---|---|---|---|
| Br | 4-Fluorphenyl | H | (A) |
| Br | 4-Fluorphenyl | H | (B) |
| H | 4-Fluorphenyl | H | (A) |
| H | 4-Fluorphenyl | H | (B) |
| Br | 4-Chlorphenyl | CH₃ | (B) |
einem pharmazeutisch annehmbaren Säure- oder Basenadditionssalz davon, einer stereochemisch isomeren Form davon, einer tautomeren Form davon oder einer N-Oxidform davon ausgewählt ist,
wobei die stereochemisch isomere Form, die zuerst isoliert wurde, mit "A" und die zweite mit "B" bezeichnet wird.

25. Kombination von (a) einer Verbindung der Formel (Ia) oder (Ib) wie in einem der vorhergehenden Ansprüche definiert und (b) einem oder mehreren anderen antibakteriellen Agenzien, mit der Maßgabe, dass es sich bei dem einen oder den mehreren anderen antibakteriellen Agenzien nicht um antimykobakterielle Agenzien handelt, wobei die Kombination für die Verwendung in der Behandlung einer durch *Staphylococci*, *Enterococci* oder *Streptococci* verursachten bakteriellen Infektion bestimmt ist.

26. Pharmazeutische Zusammensetzung, die einen pharmazeutisch annehmbaren Träger sowie als Wirkstoff eine therapeutisch wirksame Menge an (a) einer Verbindung der Formel (Ia) oder (Ib) wie in einem der Ansprüche 1 bis 24 definiert und (b) einem oder mehreren anderen antibakteriellen Agenzien, umfasst, mit der Maßgabe, dass es sich bei dem einen oder den mehreren anderen antibakteriellen Agenzien nicht um antimykobakterielle Agenzien handelt, wobei die Zusammensetzung für die Verwendung in der Behandlung einer durch *Staphylococci*, *Enterococci* oder *Streptococci* verursachten bakteriellen Infektion bestimmt ist.

27. Verbindung wie in einem der Ansprüche 1 bis 24 definiert zur Verwendung in der Behandlung einer durch *Staphylococci*, *Enterococci* oder *Streptococci* verursachten bakteriellen Infektion.

28. Produkt, das (a) eine Verbindung der Formel (Ia) oder (Ib) wie in einem der Ansprüche 1 bis 21 definiert und (b) ein oder mehrere andere antibakterielle Agenzien enthält, mit der Maßgabe, dass das eine oder die mehreren anderen antibakteriellen Agenzien nicht antimykobakterielle Agenzien sind, als Kombinationspräparat für die gleichzeitige, separate oder aufeinanderfolgende Verwendung in der Behandlung einer durch *Staphylococci*, *Enterococci* oder *Streptococci* verursachten bakteriellen Infektion.

29. Verwendung nach einem der Ansprüche 1 bis 24, Kombination nach Anspruch 25, Zusammensetzung nach Anspruch 26, Verbindung nach Anspruch 27 oder Produkt nach Anspruch 28, wobei es sich bei der bakteriellen Infektion um eine Infektion mit methicillinresistentem *Staphylococcus aureus* (MRSA), methicillinresistenten coagulasenegativen Staphylokokken (MRCNS), penicillinresistentem *Streptococcus pneumoniae* oder multipel resistentem *Enterococcus faecium* handelt.

30. Verwendung, Kombination, Zusammensetzung, Verbindung oder Produkt nach Anspruch 29, wobei es sich bei der bakteriellen Infektion um eine Infektion mit *Staphylococcus aureus* oder *Streptococcus pneumoniae* handelt.

31. Verwendung, Kombination, Zusammensetzung, Verbindung oder Produkt nach Anspruch 30, wobei es sich bei der bakteriellen Infektion um eine Infektion mit *Staphylococcus aureus* (MRSA) handelt.

## Revendications

1. Utilisation d'un composé dans la fabrication d'un médicament destiné au traitement d'une infection bactérienne provoquée par *Staphylococci*, *Enterococci* ou *Streptococci,* ledit composé étant un composé de formule (Ia) ou (Ib) 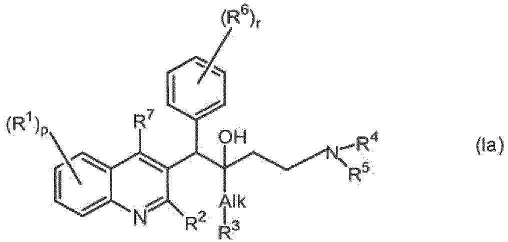 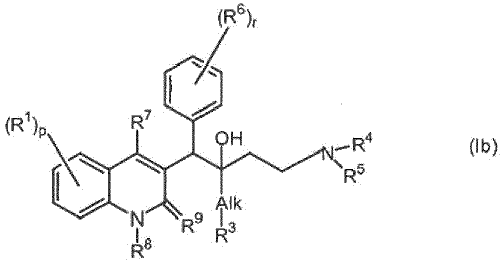
l'un de ses sels d'addition acide ou basique pharmaceutiquement acceptables, l'une de ses formes stéréoisomères, l'une de ses formes tautomères ou l'une de ses formes *N*-oxyde, où
R¹ représente un atome d'hydrogène ou d'halogène ou un groupement halogénoalkyle, cyano, hydroxy, Ar, Het, alkyle, alkoxy, alkylthio, alkoxyalkyle, alkylthioalkyle, Ar-alkyle ou di(Ar)alkyle ;
p représente un entier égal à 1, 2, 3 ou 4 ;
R² représente un atome d'hydrogène ou un groupement hydroxy, mercapto, alkoxy, alkoxyalkyloxy, alkylthio, mono ou di(alkyl)amino, ou un radical de formule 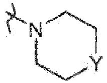 où Y représente CH₂, 0, S, NH ou *N*-alkyle ;
R³ représente un groupement Ar ou Het ;
chacun des radicaux R⁴ et R⁵ représente indépendamment un atome d'hydrogène ou un groupement alkyle ou benzyle ; ou
R⁴ et R⁵ peuvent former ensemble et avec l'atome N auquel ils sont liés un radical choisi dans le groupe constitué par les groupements pyrrolidinyle, 2-pyrrolinyle, 3-pyrrolinyle, pyrrolyle, imidazolidinyle, pyrazolidinyle, 2-imidazolinyle, 2-pyrazolinyle, imidazolyle, pyrazolyle, triazolyle, pipéridinyle, pyridinyle, pipérazinyle, pyridazinyle, pyrimidinyle, pyrazinyle, triazinyle, morpholinyle et thiomorpholinyle, éventuellement substitué par alkyle, halogéne, halogénoalkyle, hydroxy, alkyloxy, amino, mono- ou dialkylamino, alkylthio, alkyloxyalkyle, alkylthioalkyle ou pyrimidinyle ;
R⁶ représente un atome d'hydrogène ou d'halogène ou un groupement halogénoalkyle, hydroxy, Ar, alkyle, alkoxy, alkylthio, alkoxyalkyle, alkylthioalkyle, Ar-alkyle ou di(Ar)alkyle ; ou
deux radicaux R⁶ vicinaux peuvent former ensemble un radical divalent de formule -CH=CH-CH=CH- ;
r représente un entier égal à 1, 2, 3, 4 ou 5 ;
R⁷ représente un atome d'hydrogène ou un groupement alkyle, Ar ou Het ;
R⁸ représente un atome d'hydrogène ou un groupement alkyle ;
R⁹ représente un groupement oxo ; ou
les radicaux R⁸ et R⁹ forment ensemble le radical-CH=CH-N= ;
alkyle désigne un radical hydrocarboné saturé linéaire ou ramifié comportant entre 1 et 6 atomes de carbone ;
ou désigne un radical hydrocarboné saturé cyclique comportant entre 3 et 6 atomes de carbone ; ou désigne un radical hydrocarboné saturé cyclique comportant entre 3 et 6 atomes de carbone lié à un radical hydrocarboné saturé linéaire ou ramifié comportant entre 1 et 6 atomes de carbone ; où chacun des atomes de carbone est éventuellement substitué par un groupement hydroxy, alkoxy ou oxo ;
Alk désigne un radical hydrocarboné saturé linéaire ou ramifié comportant entre 1 et 6 atomes de carbone ;
Ar désigne un homocycle choisi dans le groupe constitué par les groupements phényle, naphtyle, acénaphtyle et tétrahydronaphtyle, chaque homocycle étant éventuellement substitué par 1, 2 ou 3 substituants, chaque substituant étant indépendamment choisi dans le groupe constitué par les groupements hydroxy, halogéno, cyano, nitro, amino, mono- ou dialkylamino, alkyle, halogénoalkyle, alkoxy, halogénoalkoxy, carboxyle, alkoxycarbonyle, aminocarbonyle, morpholinyle et mono- ou dialkylaminocarbonyle ;
Het désigne un hétérocycle monocyclique choisi dans le groupe constitué par les groupements N-phénoxypipéridinyle, pipéridinyle, pyrrolyle, pyrazolyle, imidazolyle, furanyle, thiényle, oxazolyle, isoxazolyle, thiazolyle, isothiazolyle, pyridinyle, pyrimidinyle, pyrazinyle et pyridazinyle ; ou un hétérocycle bicyclique choisi dans le groupe constitué par les groupements quinoléinyle, quinoxalinyle, indolyle, benzimidazolyle, benzoxazolyle, benzisoxazolyle, benzothiazolyle, benzisothiazolyle, benzofuranyle, benzothiényle, 2,3-dihydrobenzo[1,4]dioxinyle et benzo[1,3]dioxolyle ; chacun des hétérocycles monocycliques et bicycliques étant éventuellement substitué par 1, 2 ou 3 substituants, chacun des substituants étant indépendamment choisi dans le groupe constitué par les groupements halogéno, hydroxy, alkyle, alkoxy et Ar-carbonyle ;
halogéno désigne un substituant choisi dans le groupe constitué par les groupements fluoro, chloro, bromo et iodo ; et
halogénoalkyle désigne un radical hydrocarboné saturé linéaire ou ramifié comportant entre 1 et 6 atomes de carbone ou un radical hydrocarboné saturé cyclique comportant entre 3 et 6 atomes de carbone, ou un radical hydrocarboné saturé cyclique comportant entre 3 et 6 atomes de carbone lié à un radical hydrocarboné saturé linéaire ou ramifié comportant entre 1 et 6 atomes de carbone ; où un ou plusieurs des atomes de carbone sont substitués par un ou plusieurs atomes d'halogène.

2. Utilisation selon la revendication 1, où R¹ représente un atome d'hydrogène ou d'halogène ou un groupement alkyle, alkoxy, Ar ou Het.

3. Utilisation selon la revendication 2, où R¹ représente un atome d'hydrogène ou d'halogène ou un groupement alkyle ou alkoxy.

4. Utilisation selon la revendication 3, où R¹ représente un atome d'hydrogène ou d'halogène.

5. Utilisation selon la revendication 4, où R¹ représente un atome d'halogène.

6. Utilisation selon l'une quelconque des revendications précédentes, où p est égal à 1.

7. Utilisation selon la revendication 6, où le substituant R¹ est placé en position 6 du cycle quinoléine.

8. Utilisation selon l'une quelconque des revendications précédentes, où R² représente un groupement alkoxy, alkylthio, mono- ou di(alkyl)amino ou alkoxyalkoxy.

9. Utilisation selon l'une quelconque des revendications 1 à 7, où R² représente un atome d'hydrogène ou un groupement alkoxy ou alkylthio.

10. Utilisation selon la revendication 8 ou 9, où R² représente un groupement alkoxy en C₁₋₄.

11. Utilisation selon l'une quelconque des revendications précédentes, où R³ représente un groupement Ar.

12. Utilisation selon la revendication 11, où R³ représente un groupement naphtyle ou phényle, éventuellement substitué par 1 ou 2 atomes d'halogène.

13. Utilisation selon l'une quelconque des revendications précédentes, où chacun des radicaux R⁴ et R⁵ représente indépendamment un atome d'hydrogène ou un groupement alkyle en C₁₋₄.

14. Utilisation selon l'une quelconque des revendications précédentes, où R⁶ représente un atome d'hydrogène.

15. Utilisation selon l'une quelconque des revendications précédentes, où R⁷ représente un atome d'hydrogène.

16. Utilisation selon l'une quelconque des revendications précédentes, où Alk représente un groupement méthylène ou éthylène.

17. Utilisation selon la revendication 16, où Alk représente un groupement éthylène.

18. Utilisation selon l'une quelconque des revendications précédentes, où le composé est un composé répondant à la formule (Ia).

19. Utilisation selon la revendication 1, où le composé est un composé de formule (Ia), où R¹ représente un atome d'hydrogène ou d'halogène ou un groupement alkyle en C₁₋₄, alkoxy en C₁₋₄, Ar ou Het ; p = 1 ou 2 ; R² représente un groupement alkoxy en C₁₋₄, (alkyle en C₁₋₄)-thio, mono- ou di- (alkyle en C₁₋₄)-amino, (alkoxy en C₁₋₄)-(alkoxy en C₁₋₄) ; R³ représente un groupement naphtyle ou phényle éventuellement substitué ; chacun des radicaux R⁴ et R⁵ représente indépendamment un atome d'hydrogène ou un groupement alkyle en C₁₋₄ ; ou R⁴ et R⁵ forment ensemble et avec l'atome N auquel ils sont liés un groupement pipéridinyle ; R⁶ représente un atome d'hydrogène ou d'halogène ou un groupement alkyle en C₁₋₄ ou alkoxy en C₁₋₄ ; r est égal à 1 ; R⁷ représente un atome d'hydrogène ; Alk représente un groupement méthylène ou éthylène.

20. Utilisation selon l'une quelconque des revendications précédentes, où l'infection bactérienne est une infection par une bactérie à Gram positif.

21. Utilisation selon l'une quelconque des revendications précédentes, où le composé est choisi parmi 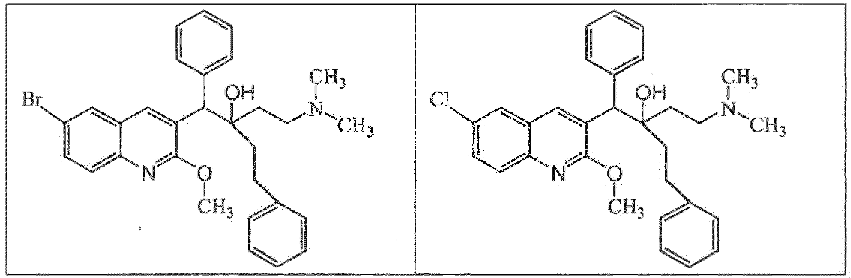 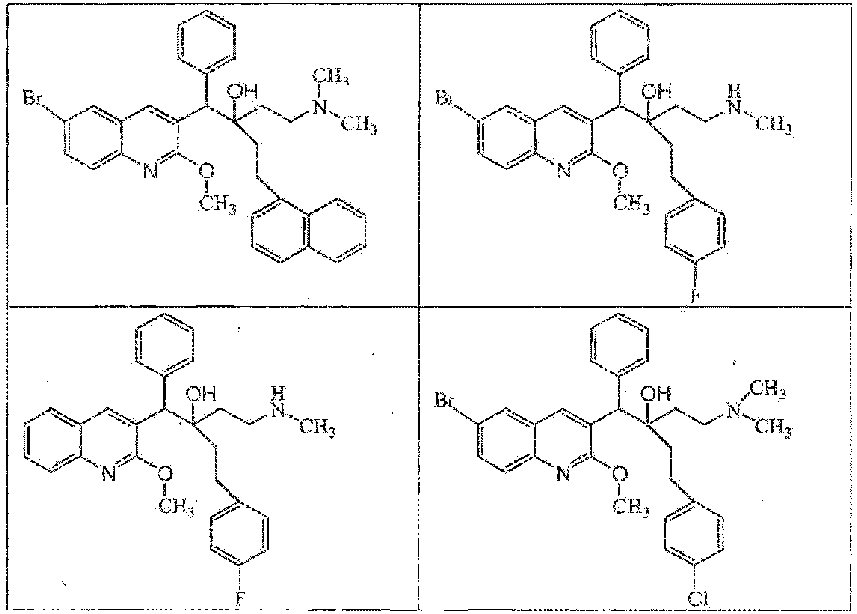 l'un de leurs sels d'addition acide ou basique pharmaceutiquement acceptables, l'une de leurs formes stéréoisomères, l'une de leurs formes tautomères ou l'une de leurs formes N-oxyde.

22. Composé choisi parmi les suivants : 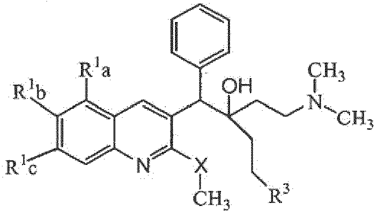
| R¹ₐ | R¹_{b} | R¹_{c} | R³ | X |
|---|---|---|---|---|
| H | H | H | phényle | O |
| H | CH₃ | H | phényle | O |
| H | OCH₃ | H | phényle | O |
| H | Br | H | phényle | S |
| H | Br | H | 1-naphtyle | O |
| H | Br | CH₃ | phényle | O |
| H | Cl | H | phényle | O |
| Br | H | H | phényle | O |
l'un de leurs sels d'addition acide ou basique pharmaceutiquement acceptables, l'une de leurs formes stéréoisomères, l'une de leurs formes tautomères ou l'une de leurs formes N-oxyde.

23. Composé choisi parmi les suivants : 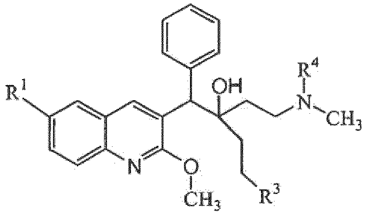
| R¹ | R³ | R⁴ |
|---|---|---|
| Br | 4-fluorophény le | H |
| H | 4-fluorophény le | H |
| Br | 4-chlorophény le | CH₃ |
l'un de leurs sels d'addition acide ou basique pharmaceutiquement acceptables, l'une de leurs formes stéréoisomères, l'une de leurs formes tautomères ou l'une de leurs formes N-oxyde.

24. Composé choisi parmi les suivants : 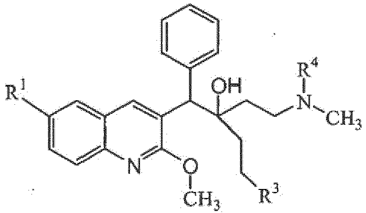
| R¹ | R³ | R⁴ | stéréochimie |
|---|---|---|---|
| Br | 4-fluorophény le | H | (A) |
| Br | 4-fluorophény le | H | (B) |
| H | 4-fluorophény le | H | (A) |
| H | 4-fluorophény le | H | (B) |
| Br | 4-chlorophény le | CH₃ | (B) |
l'un de leurs sels d'addition acide ou basique pharmaceutiquement acceptables, l'une de leurs formes stéréoisomères, l'une de leurs formes tautomères ou
l'une de leurs formes N-oxyde, où la forme stéréoisomère qui est la première à avoir été isolée est désignée par « A », et la deuxième par « B ».

25. Combinaison de (a) un composé de formule (Ia) ou (Ib) selon l'une quelconque des revendications précédentes, et de (b) un ou plusieurs autres agents antibactériens, à la condition que le ou lesdits autres agents antibactériens soient différents d'agents antimycobactériens, où la combinaison est destinée à une utilisation dans le traitement d'une infection bactérienne provoquée par *Staphylococci*, *Enterococci* ou *Streptococci.*

26. Composition pharmaceutique comprenant un vecteur de qualité pharmaceutique et, au titre de principe actif, une quantité thérapeutiquement active de (a) un composé de formule (Ia) ou (Ib) selon l'une quelconque des revendications 1 à 24, et de (b) un ou plusieurs autres agents antibactériens, à la condition que le ou lesdits autres agents antibactériens soient différents d'agents antimycobactériens, où la composition est destinée à une utilisation dans le traitement d'une infection bactérienne provoquée par *Staphylococci*, *Enterococci* ou *Streptococci.*

27. Composé selon l'une quelconque des revendications 1 à 24, pour utilisation dans le traitement d'une infection bactérienne provoquée par *Staphylococci*, *Enterococci* ou *Streptococci.*

28. Produit contenant (a) un composé de formule (Ia) ou (Ib) selon l'une quelconque des revendications 1 à 21, et (b) un ou plusieurs autres agents antibactériens, à la condition que le ou lesdits autres agents antibactériens soient différents d'agents antimycobactériens, sous forme d'une préparation combinée pour utilisation simultanée, séparée ou séquentielle dans le traitement d'une infection bactérienne provoquée par *Staphylococci*, *Enterococci* ou *Streptococci.*

29. Utilisation selon l'une quelconque des revendications 1 à 24, combinaison selon la revendication 25, composition selon la revendication 26, composé selon la revendication 27 ou produit selon la revendication 28, où l'infection bactérienne est une infection par *Staphylococcus aureus* résistant à la méthicilline (MRSA), un staphylocoque négatif à la coagulase résistant à la méthicilline (MRCNS), *Streptococcus pneumoniae* résistant à la pénicilline ou *Enterococcus faecium* multi-résistant.

30. Utilisation, combinaison, composition, composé ou produit selon la revendication 29, où l'infection bactérienne est une infection par *Staphylococcus aureus* ou *Streptococcus pneumoniae.*

31. Utilisation, combinaison, composition, composé ou produit selon la revendication 30, où l'infection bactérienne est une infection par *Staphylococcus aureus* (MRSA).
